(19) Europäisches Patentamt
European Patent Office
Office européen des brevets

(11) EP 0 555 537 B1

(12) **EUROPEAN PATENT SPECIFICATION**

(45) Date of publication and mention of the grant of the patent:
**02.11.2000 Bulletin 2000/44**

(51) Int. Cl.$^7$: **C07K 5/06**, C07K 5/08, C07K 5/023, A61K 38/04

(21) Application number: **92120225.5**

(22) Date of filing: **26.11.1992**

(54) **Endothelin antagonists**
Endothelin Antagonisten
Antagonistes d'endothéline

(84) Designated Contracting States:
**AT BE CH DE DK ES FR GB GR IE IT LI LU MC NL PT SE**

(30) Priority: **04.12.1991 JP 34767091**
**18.12.1991 JP 35373891**
**10.08.1992 JP 23420792**

(43) Date of publication of application:
**18.08.1993 Bulletin 1993/33**

(73) Proprietor:
**BANYU PHARMACEUTICAL CO., LTD.**
**Chuo-ku, Tokyo (JP)**

(72) Inventors:
- **Ishikawa, Kiyofumi, c/o Banyu Pharmaceutical Co., Tsukuba-shi, Ibaraki-ken 300-33 (JP)**
- **Fukami, Takehiro, c/o Banyu Pharmaceutical Co., Tsukuba-shi, Ibaraki-ken 300-33 (JP)**
- **Nagase, Toshio, c/o Banyu Pharmaceutical Co., Tsukuba-shi, Ibaraki-ken 300-33 (JP)**
- **Mase, Toshiaki, c/o Banyu Pharmaceutical Co., Tsukuba-shi, Ibaraki-ken 300-33 (JP)**
- **Ihara, Masaki, c/o Banyu Pharmaceutical Co., Tsukuba-shi, Ibaraki-ken 300-33 (JP)**
- **Yano, Mitsuo, c/o Banyu Pharmaceutical Co., Tsukuba-shi, Ibaraki-ken 300-33 (JP)**
- **Nishikibe, Masaru, c/o Banyu Pharmaceutical Co., Tsukuba-shi, Ibaraki-ken 300-33 (JP)**

(74) Representative:
**Wächtershäuser, Günter, Prof. Dr.**
**Patentanwalt,**
**Tal 29**
**80331 München (DE)**

(56) References cited:
**EP-A- 0 457 195** **EP-A- 0 460 679**
**WO-A-93/10144**

Remarks:
The file contains technical information submitted after the application was filed and not included in this specification

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 555 537 B1

## Description

The present invention relates to novel compounds having antagonism against three endothelin isopeptides (endothelin-1, endothelin-2 and endothelin-3), which are physiologically highly active endogenous peptides, processes for their preparation and their use as a drug.

EP-A 0 457 195 discloses peptides having endothelin antagonist activity.

Two endothelin receptor subtypes $ET_A$ and $ET_B$ are known so far. The compounds of the present invention possess high affinity to at least the $ET_B$ receptors, thereby inhibiting vasoconstriction and bronchoconstriction induced by the endothelins. The compounds of the present invention provide a new therapeutic potential, particularly for the treatment of hypertension, pulmonary hypertension, Raynaud's disease, acute renal failure, myocardial infarction, angina pectoris, cerebral infarction, cerebral vasospasm, arteriosclerosis, asthma, gastric ulcer, diabetes, endotoxin shock, endotoxin-induced multiple organ failure or disseminated intravascular coagulation, and/or cyclosporin-induced renal failure or hypertension.

Endothelin is a polypeptide composed of 21 amino acids, and it is produced by vascular endothelial cells of human or pig. Endothelin has a potent vasoconstrictor effect and a sustained and potent pressor action (Nature, 332, 411-415 (1988)).

Three endothelin isopeptides (endothelin-1, endothelin-2 and endothelin-3), which resemble one another in structure, exist in the bodies of animals including human, and these peptides have vasoconstriction and pressor effects (Proc. Natl. Acad, Sci, USA, 86, 2863-2867 (1989)).

As reported, the endothelin levels are clearly elevated in the blood of patients with essential hypertension, acute myocardial infarction, pulmonary hypertension, Raynaud's disease, diabetes or atherosclerosis, or in the washing fluids of the respiratory tract or the blood of patients with asthmaticus as compared with normal levels (Japan, J. Hypertension, 12, 79, (1989), J. Vascular Medicine Biology, 2, 207 (1990), Diabetologia, 33, 306-310 (1990), J. Am. Med. Association, 264, 2868 (1990), and The Lancet, ii, 747-748 (1989) and ii, 1144-1147 (1990)).

Further, an increased sensitivity of the cerebral blood vessel to endothelin in an experimental model of cerebral vasospasm (Japan. Soc. Cereb. Blood Flow & Metabol., 1, 73 (1989)), an improved renal function by the endothelin antibody in an acute renal failure model (J. Clin, Invest., 83, 1762-1767 (1989), and inhibition of gastric ulcer development with an endothelin antibody in a gastric ulcer model (Extract of Japanese Society of Experimental Gastric Ulcer, 50 (1991)) have been reported. Therefore, endothelin is assumed to be one of mediators causing acute renal failure or cerebral vasospasm following subarachnoid hemorrhage.

Further, endothelin is secreted not only by endothelial cells but also by tracheal epithelial cells or from kidney cells (FEBS Letters, 255, 129-132 (1989), and FEBS Letters, 249, 42-46 (1989)).

Endothelin was also found to control the release of physiologically active endogenous substances such as renin, atrial natriuretic peptide, endothelium-derived relaxing factor (EDRF), thromboxane $A_2$, prostacyclin, noradrenaline, angiotensin II and substance P (Biochem. Biophys, Res. Commun., 157, 1164-1168 (1988); Biochem. Biophys, Res. Commun., 155, 167-172 (1989); Proc. Natl. Acad. Sci. USA, 85, 9797-9800 (1989); J. Cardiovasc. Pharmacol., 13, S89-S92 (1989); Japan. J. Hypertension, 12, 76 (1989) and Neuroscience Letters, 102, 179-184 (1989)). Further, endothelin causes contraction of the smooth muscle of gastrointestinal tract and the uterine smooth muscle (FEBS Letters, 247, 337-340 (1989); Eur. J. Pharmacol., 154, 227-228 (1988); and Biochem. Biophys. Res. Commun., 159, 317-323 (1989)). Further, endothelin was found to promote proliferation of rat vascular smooth muscle cells, suggesting a possible relevance to the arterial hypertrophy (Atherosclerosis, 78, 225-228 (1989)). Furthermore, since the endothelin receptors are present in a high density not only in the peripheral tissues but also in the central nervous system, and the cerebral administration of endothelin induces a behavioral change in animals, endothelin is likely to play an important role for controlling nervous functions (Neuroscience Letters, 97, 276-279 (1989)). Particularly, endothelin is suggested to be one of mediators for pain (Life Sciences, 49, PL61-PL65 (1991)).

On the other hand, endotoxin is one of potential candidates to promote the release of endothelin. Remarkable elevation of the endothelin levels in the blood or in the culture supernatant of endothelial cells was observed when endotoxin was exogenously administered to animals or added to the culture endothelial cells, respectively. These findings suggest that endothelin is one of important mediators for endotoxin-induced diseases (Biochem. Biophys. Res. Commun., 161, 1220-1227 (1989); and Acta Physiol. Scand., 137, 317-318 (1989)).

Further, it was reported that cyclosporin remarkably increased endothelin secretion in the renal cell culture (LLC-PK1 cells) (Eur. J. Pharmacol., 180, 191-192 (1990)). Further, dosing of cyclosporin to rats reduced the glomerular filtration rate and increased the blood pressure in association with a remarkable increase in the circulating endothelin level. This cyclosporin-induced renal failure can be suppressed by the administration of endothelin antibody (Kidney Int., 37, 1487-1491 (1990)). Thus, it is assumed that endothelin is significantly involved in the pathogenesis of the cyclosporin-induced diseases.

Such various effects of endothelin are caused by the binding of endothelin to endothelin receptors widely distributed in many tissues (Am. J. Physiol., 256, R856-R866 (1989)).

It is known that vasoconstriction by the endothelins is caused via at least two subtypes of endothelin receptors (J. Cardiovasc. Pharmacol., 17(Suppl.7), S119-S121 (1991)). One of endothelin receptors is $ET_A$ receptor selective to ET-1 rather than ET-3, and the other is $ET_B$ receptor equally active to ET-1 and ET-3. These receptor proteins are reported to be different from each other (Nature, 348, 730-735 (1990)).

These two subtypes of endothelin receptors are differently distributed in tissues. It is known that the $ET_A$ receptor is present mainly in cardiovascular tissues, whereas the $ET_B$ receptor is widely distributed in various tissues such as brain, kidney, lung, heart and vascular tissues.

Substances which specifically inhibit the binding of endothelin to the endothelin receptors are believed to antagonize various pharmacological activities of endothelin and to be useful as a drug in a wide field. The present inventors already disclosed potent endothelin $ET_A$ receptor antagonists in EP 0460679A2. Since, the action of the endothelins is caused via not only the $ET_A$ receptor but also the $ET_B$ receptor, novel substances with $ET_B$ receptor antagonistic activity are desired to block activities of the endothelins in various diseases.

Endothelin is an endogenous substance which directly or indirectly (by controlling liberation of various endogenous substances) induces sustained contraction of vascular or non-vascular smooth muscles, and its excess production or excess secretion is believed to be one of pathogeneses for hypertension, pulmonary hypertension, Raynaud's disease, bronchial asthma, gastric ulcer, diabetes, arteriosclerosis, acute renal failure, myocardial infarction, angina pectoris, cerebral vasospasm and cerebral infarction. Further, it is suggested that endothelin serves as an important mediator involved in disease such as endotoxin shock, endotoxin-induced multiple organ failure or disseminated intravascular coagulation, and/or cyclosporin-induced renal failure or hypertension. Two endothelin receptors $ET_A$ and $ET_B$ are known so far. An antagonistic agent against the $ET_B$ receptor as well as the $ET_A$ receptor is useful as a drug. Accordingly, it is an object of the present invention to provide a novel therapeutics for the treatment of the above-mentioned various diseases by an invention of a potent $ET_B$ receptor antagonist.

In order to accomplish the above object, the present inventors have synthesized various peptide derivatives and have investigated their endothelin antagonistic activities, and as a result have found that novel peptide derivatives represented by the following formula (I) and their pharmaceutically acceptable salts have strong potent $ET_B$ receptor antagonistic activities. The present invention has been accomplished on the basis of this discovery.

Thus, the present invention provides a peptide derivative of the formula:

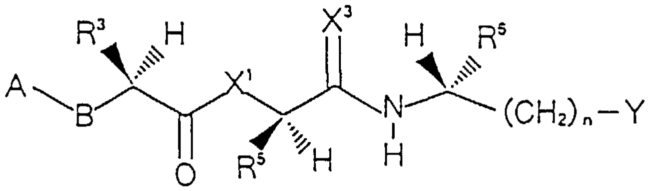

(I)

wherein A is a group of the formula $R^{12}R^{13}N-C(=O)-$ (wherein $R^{12}$ is a $C_{1-6}$ alkyl group, a cyclopropyl group, a cyclobutyl group, a cyclopentyl group, a cyclohexyl group, a cycloheptyl group, a cyclooctyl group, a cyclononyl group, a 1-adamantyl group, a phenyl group wherein one or two optional hydrogen atoms on the benzene ring may independently be replaced by a halogen atom, a trifluoromethyl group, a nitro group, an amino group or a formylamino group, or a thienyl group, $R^{13}$ is a hydrogen atom, a $C_{1-6}$ alkyl group or a cyclopropyl group, a cyclobutyl group, a cyclopentyl group, a cyclohexyl group, a cycloheptyl group, a cyclooctyl group, a cyclononyl group, or $R^{12}$ and $R^{13}$ form, together with the adjacent nitrogen atom, a 5- to 9- membered nitrogen-containing saturated heterocyclic ring having 4 to 8 carbon atoms, wherein among methylene groups forming the ring, one optional methylene group not adjacent to the above nitrogen atom may be replaced by a thio group, and one to four optional hydrogen atoms on the carbon atoms of the heterocyclic ring may independently be replaced by a $C_{1-6}$ alkyl group, and further two adjacent carbon atoms in the heterocyclic ring may form a benzo-fused ring); B is an oxygen atom or a group of the formula $-NR^2-$ (wherein $R^2$ is a hydrogen atom or a $C_{1-6}$ alkyl group); $R^3$ is a $C_{1-6}$ alkyl group, a cyclopropyl group, a cyclobutyl group, a cyclopentyl group, a cyclohexyl group, a cycloheptyl group, a cyclooctyl group, a cyclononyl group, a phenyl group, a 1-naphthyl group, a 2-naphthyl group, a thienyl group, a furyl group, a thiazolyl group, an imidazolyl group, a pyridyl group, an indolyl group, a benzothienyl group, a $C_{1-6}$ alkyl group having a substituent selected from the group consisting of a cyclopropyl group, a cyclobutyl group, a cyclopentyl group, a cyclohexyl group, a cycloheptyl group, a cyclooctyl group, a cyclononyl group, a phenyl group, a 1-naphthyl group, a 2-naphthyl group, a thienyl group, a furyl group, a thiazolyl group, an imidazolyl group, a pyridyl group, an indolyl group and a benzothienyl group; $X^1$ is an oxygen atom or a group of the formula $-NR^4-$ (wherein $R^4$ is a hydrogen atom or a $C_{1-6}$ alkyl group); $R^5$ is a 3-indolylmethyl group wherein the indole ring is substituted at the 1-position and/or 2-position by one or two substituents selected from the group consisting of a $C_{1-6}$ alkoxy group, a $C_{1-6}$ alkylthio group, a $C_{2-6}$ alkanoyloxy group, a $C_{1-6}$ alkoxy-carbonyl group and a halogen

atom; $X^3$ is an oxygen atom or a sulfur atom; $R^6$ is a $C_{1-6}$ alkyl or $C_{2-6}$ alkenyl group which may have a substituent selected from the group consisting of a $C_{1-6}$ alkoxy group and a $C_{1-6}$ alkylthio group;
n is 0 or 1; Y is a hydroxymethyl group, a group of the formula $CO_2R^{71}$ (wherein $R^{71}$ is a hydrogen atom or a $C_{1-6}$ alkyl group), a group of the formula $CONHR^{72}$ (wherein $R^{72}$ is a hydrogen atom or a $C_{1-6}$ alkyl group which may have a substituent selected from the group consisting of a hydroxyl group, a carboxyl group and a sulfo group), a 1H-tetrazol-5-yl group, a sulfo group and a phosphono group; or a pharmaceutically acceptable salt thereof.

In a preferred embodiment the invention provides a compound of the formula:

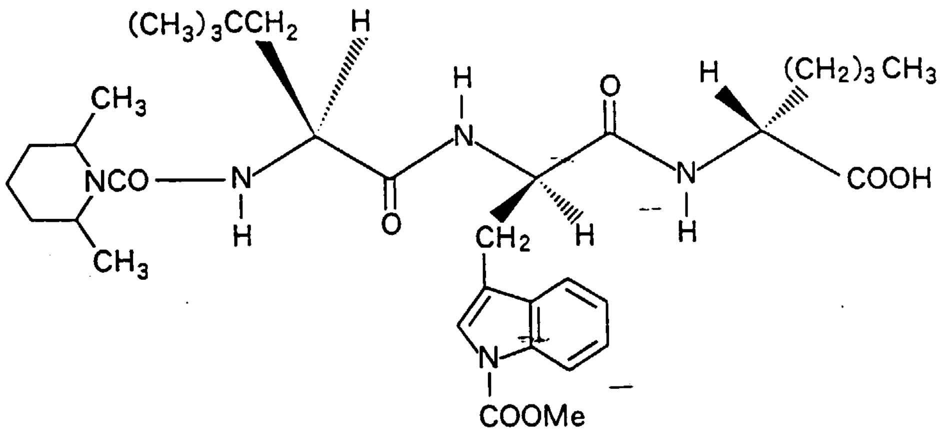

The invention also provides an endothelin antagonistic agent comprising a compound as defined in Formula I or a pharmaceutically acceptable salt thereof.

Furthermore the invention provides the use of a peptide derivative of the formula (I) or a pharmaceutically acceptable salt thereof for preparing a medicine having $ET_B$ receptor antagonistic activity.

In the accompanying drawings:

Figure 1 shows the activities of 10 μM of Compound 1 (○) against endothelin-3-induced contraction of isolated guinea pig bronchial tube as compared with the case in which no drug is present (●).
Figure 2 shows the activities of 10 μM of Compound 20 (○) against endothelin-3-induced contraction of isolated guinea pig bronchial tube as compared with the case in which no drug is present (●).
Figure 3 shows the activities of 10 μM of Compound 21 (○) against endothelin-3-induced contraction of isolated guinea pig bronchial tube as compared with the case in which no drug is present (●).

Now, the present invention will be described in further detail with reference to the preferred embodiments.

Now, the meanings of various abbreviations used in this specification will be given.

| Abbreviation | Meaning of Abbreviation |
|---|---|
| DβAbu | D-3-aminobutyric acid |
| DBal | D-3-(3-benzothienyl)alanine |
| Ile | L-isoleucine |
| DIle | D-isoleucine |
| Leu | L-leucine |
| γMeLeu | γ-methyl-L-leucine |
| DMet | D-methionine |
| DNal | D-3-(1-naphthyl)alanine |
| Nle | L-norleucine |
| DNle | D-norleucine |
| Nva | L-norvaline |
| DNva | D-norvaline |
| DPhe(m-$NO_2$) | D-3-(3-nitrophenyl)alanine |
| DPhe(m-$NH_2$) | D-3-(3-aminophenyl)alanine |
| DLPhe(3-COOEt) | DL-3-(3-ethoxycarbonylphenyl)alanine |
| DLPhe(3-COOMe) | DL-3-(3-methoxycarbonylphenyl)alanine |
| DSer | D-serine |
| DSer(Me) | O-methyl-D-serine |
| D-mTyr | D-methatyrosine |
| DL-mTyr | DL-methatyrosine |
| DTrp | D-tryptophan |

| | |
|---|---|
| DTrp(7-OBzl) | D-(7-benzyloxy)tryptophan |
| DTrp(7-OH) | D-(7-hydroxy)tryptophan |
| DTrp(COOEt) | D-($N^{in}$-ethoxycarbonyl)tryptophan |
| DTrp(COOMe) | D-($N^{in}$-methoxycarbonyl)tryptophan |
| DTrp(OH) | D-($N^{in}$-hydroxy)tryptophan |
| DTrp(OMe) | D-($N^{in}$-methoxy)tryptophan |
| DLTrp(Me) | DL-($N^{in}$-methyl)tryptophan |
| DTrp(2-SMe) | D-(2-methylthio)tryptophan |
| DTrp(2-Br) | D-(2-bromo)tryptophan |
| DTrp(2-Cl) | D-(2-chloro)tryptophan |
| DTrp(1-Me,2-Cl) | D-(2-chloro-1-methyl)tryptophan |
| Ac | acetyl |
| Boc | tert-butoxycarbonyl |
| Et | ethyl |
| Me | methyl |
| $^{n}Pr$ | n-propyl |
| $^{i}Pr$ | isopropyl |
| $^{n}Bu$ | n-butyl |
| $^{t}Bu$ | tert-butyl |
| Ph | phenyl |
| Bzl | benzyl |
| c-Pent | cyclopentyl |
| Cpeg | L-cyclopentylglycine |
| Cprg | L-cyclopropylglycine |
| CDI | 1,1'-carbonyldiimidazole |
| DCC | N,N'-dicyclohexylcarbodiimide |
| DMAP | 4-(dimethylamino)pyridine |

| | |
|---|---|
| DMF | N,N-dimethylformamide |
| DMSO | dimethylsulfoxide |
| NMP | N-methylpyrrolidone |
| NMM | N-methylmorpholine |
| EDCI·HCl | 1-ethyl-3-(3-dimethylaminopropyl)-carbodiimide·hydrochloride |
| HOBT·$H_2O$ | 1-hydroxy-1H-benzotriazole·monohydrate |
| HOSu | N-hydroxysuccinimide |
| TBAHS | tetra-n-butylammonium hydrogensulfate |
| TEA | triethylamine |
| TFA | trifluoro acetic acid |
| THF | tetrahydrofuran |
| TsOH | p-toluene sulfonic acid |
| Ts | p-toluenesulfonyl |
| Z | benzyloxycarbonyl |
| MOPS | 3-morpholinopropane sulfonic acid |
| HEPES | 2-[4-(2-hydroxyethyl)-1-piperazinyl]-ethane sulfonic acid |
| Tris | tris(hydroxymethyl)aminomethane |
| PMSF | phenylmethanesulfonyl=fluoride |

Now, the definitions of the various terms mentioned in this specification will be explained.

In this specification, the $C_{1-6}$ alkyl group means a linear or branched alkyl group having 1 to 6 carbon atoms such as a methyl, ethyl, propyl, isopropyl, butyl, isobutyl, sec-butyl, tert-butyl, pentyl, isopentyl, neopentyl, tert-pentyl, 1-methylbutyl, 2-methylbutyl, 1,2-dimethylpropyl, 1-ethylpropyl, hexyl, isohexyl, 1-methylpentyl, 2-methylpentyl, 3-methylpentyl, 1,1-dimethylbutyl, 1,2-dimethylbutyl, 2,2-dimethylbutyl, 1-ethylbutyl, 1,1,2-trimethylpropyl, 1,2,2-trimethylpropyl, 1-ethyl-2-methylpropyl or 1-ethyl-1-methylpropyl group.

The cycloalkyl group may be a cyclopropyl, cyclobutyl, cyclopentyl, cyclohexyl, cycloheptyl, cyclooctyl or cyclononyl group.

The aryl group may be a phenyl, 1-naphthyl or 2-naphthyl group.

The heterocyclic group means a heterocyclic or fused heterocyclic group containing at least one hetero atom such as an oxygen, nitrogen or sulfur atom, for example, a thienyl, furyl, thiazolyl, imidazolyl, pyridyl, indolyl or benzothienyl group.

The $C_{2-6}$ alkenyl group means a linear or branched alkenyl group having 2 to 6 carbon atoms such as a vinyl, allyl, 2-propenyl, isopropenyl, 3-butenyl, 2-butenyl, 1-butenyl, 1-methyl-2-propenyl, 1-methyl-1-propenyl, 1-ethyl-1-ethenyl, 2-methyl-2-propenyl, 2-methyl-1-propenyl, 4-pentenyl or 5-pentenyl group.

The $C_{1-6}$ alkoxy group means a linear or branched alkoxy group having 1 to 6 carbon atoms such as a methoxy, ethoxy, propoxy or isopropoxy group.

The $C_{2-6}$ alkanoyloxy group means a linear or branched alkanoyloxy group having 2 to 6 carbon atoms such as an acetoxy or propionyloxy group.

The halogen atom means a fluorine, chlorine, bromine or iodine atom.

The $C_{1-6}$ alkylthio group means a linear or branched alkylthio group having 1 to 6 carbon atoms such as a methylthio, ethylthio, propylthio or isopropyl group. The $C_{1-6}$ alkylsulfinyl group means a linear or branched alkylsulfinyl group having 1 to 6 carbon atoms such as a methylsulfinyl, ethylsulfinyl, propylsulfinyl or isopropylsulfinyl group. The $C_{1-6}$ alkylsulfonyl group means a linear or branched alkylsulfonyl group having 1 to 6 carbon atoms such as a methylsulfonyl, ethylsulfonyl, propylsulfonyl or isopropylsulfonyl group.

Now, this invention will be described in more detail with reference to specific examples for the various symbols used in the formula (I).

In A, $R^{12}$ means a $C_{1-6}$ alkyl group, a cycloalkyl group, a 1-adamantyl group, a phenyl group wherein 1 or 2 optional hydrogen atoms on the benzene ring may be replaced by an optional group selected from the group consisting of a halogen atom, a trifluoromethyl group, a nitro group, an amino group and a formylamino group or a thienyl group. Examples of the $C_{1-6}$ alkyl group are methyl, ethyl, propyl, isopropyl, butyl, isobutyl, sec-butyl, tert-butyl, pentyl, isopentyl, neopentyl, tert-pentyl, 1,1-dimethylbutyl, 1-ethyl-1-methylpropyl and 1,1,2-trimethylpropyl groups. Examples of the cycloalkyl group are cyclopropyl, cyclobutyl, cyclopentyl, cyclohexyl and cycloheptyl groups. Examples of the phenyl group wherein 1 or 2 optional hydrogen atoms on the benzene ring may be replaced by an optional group selected from the group consisting of a halogen atom, a trifluoromethyl group, a nitro group, an amino group and a formylamino group, are phenyl, 2-fluorophenyl, 2-chlorophenyl, 2-bromophenyl, 3-fluorophenyl, 3-chlorophenyl, 3-bromophenyl, 4-fluorophenyl, 4-chlorophenyl, 4-bromophenyl, 2,6-difluorophenyl, 2,6-dichlorophenyl, 2,6-dibromophenyl, 2-aminophenyl, 2-formylaminophenyl, 2-trifluoromethylphenyl, 2-nitrophenyl, 3-aminophenyl and 3-formylaminophenyl groups. Examples of the thienyl group are 2-thienyl and 3-thienyl groups.

In A, $R^{13}$ means a hydrogen atom, a $C_{1-6}$ alkyl group or a cycloalkyl group. Examples of the $C_{1-6}$ alkyl group are methyl, ethyl, propyl, isopropyl, butyl, isobutyl, sec-butyl, tert-butyl, pentyl, isopentyl, neopentyl and tert-pentyl groups. Examples of the cycloalkyl group are cyclopropyl, cyclobutyl, cyclopentyl, cyclohexyl and cycloheptyl groups.

In A, $R^{12}$ and $R^{13}$ may also form, together with the adjacent nitrogen atom, a 5- to 9-membered nitrogen-containing saturated heterocyclic group having 4 to 8 carbon atoms. Among methylene groups forming the heterocycle, one optional methylene group not adjacent to the above nitrogen atom may be replaced by a thio group, and one to four optional hydrogen atoms on the carbon atoms of the heterocycle may independently be replaced by a $C_{1-6}$ alkyl group, and further two adjacent carbon atoms in the heterocycle may form a fused-benzene ring. Examples of the heterocyclic group are pyrrolidino, piperidino, perhydroazepin-1-yl, perhydroazocin-1-yl, perhydroazonin-1-yl, 1,3-thiazolidin-1-yl, indolin-1-yl, isoindolin-2-yl, 3-pyrolin-1-yl, 1,5-dihydro-2H-pyrrol-1-yl, perhydro-1,4-thiadin-4-yl, 1,2,3,4-tetrahydroquinolin-1-yl, 1,2,3,4-tetrahydroisoquinolin-2-yl, 1,2,3,4-tetrahydropyridin-1-yl, 1,2,3,6-tetrahydropyridin-1-yl, perhydro-1,4-thiazepin-4-yl, 2,3,4,5-tetrahydro-1-benzazepin-1-yl, 2,3,4,5-tetrahydro-2-benzazepin-2-yl, 1,2,4,5-tetrahydro-3-benzazepin-3-yl, 2,3,4,5-tetrahydro-1H-azepin-1-yl, 2,3,6,7-tetrahydro-1H-azepin-1-yl, 1,3,4,7-tetrahydro-2H-azepin-1-yl, perhydro-1,4-thiazocin-4-yl, 1,2,3,4,5,6-hexahydro-1-benzazocin-1-yl, 1,2,3,4,5,6-hexahydro-2-benzazocin-2-yl, 1,2,3,4,5,6-hexahydro-3-benzazocin-3-yl, 1,2,3,4,5,6-hexahydroazocin-1-yl, 1,2,3,4,7,8-hexahydroazocin-1-yl and 1,2,3,4,5,8-hexahydroazocin-1-yl groups, or the above-mentioned heterocyclic groups wherein one to four optional hydrogen atoms on the carbon atoms of the heterocycle may independently be replaced by a $C_{1-6}$ alkyl group. Examples of the alkyl group are methyl, ethyl, propyl, isopropyl, butyl, isobutyl, sec-butyl and tert-butyl groups.

In B, $R^2$ means a hydrogen atom or a $C_{1-6}$ alkyl group. Examples of the $C_{1-6}$ alkyl group are methyl and ethyl groups.

$R^3$ means a $C_{1-6}$ alkyl group, a cycloalkyl group as defined by claim 1, an aryl group as defined by claim 1, a heterocyclic group as defined by claim 1, a cycloalkyl $C_{1-6}$ alkyl group as defined by claim 1, an aryl $C_{1-6}$ alkyl group or a heterocyclic $C_{1-6}$ alkyl group as defined by claim 1. Examples of the $C_{1-6}$ alkyl group are propyl, isopropyl, butyl, isobutyl, sec-butyl, tert-butyl, pentyl, isopentyl, neopentyl and tert-pentyl groups. Examples of the cycloalkyl group are cyclopropyl, cyclobutyl, cyclopentyl and cyclohexyl groups. An example of the aryl group is a phenyl group. Examples of the heterocyclic group are 2-thienyl, 3-thienyl, 2-thiazolyl, 4-thiazolyl, 5-thiazolyl, 2-furyl and 3-furyl groups. Examples of the cycloalkyl $C_{1-6}$ alkyl group are cyclopropylmethyl, 1-cyclopropylethyl, 2-cyclopropylethyl, 1-cyclopropyl-1-methylethyl, cyclobutylmethyl, 1-cyclobutylethyl, 2-cyclobutylethyl, 1-cyclobutyl-1-methylethyl, cyclopentylmethyl, 1-cyclopentylethyl, 2-cyclopentylethyl, 1-cyclopentyl-1-methylethyl, 1-cyclohexylmethyl, 1-cyclohexylethyl, 1-cyclohexyl-1-methylethyl, 1-cycloheptylmethyl, 1-cycloheptylethyl, 1-cyclooctylmethyl and 1-cyclooctylethyl groups. Examples of the aryl $C_{1-6}$ alkyl group are benzyl and phenylethyl groups. Examples of the heterocyclic $C_{1-6}$ alkyl group are 2-thienylmethyl, 3-thienylmethyl, 2-thienylethyl, 2-thiazolylmethyl, 2-furylmethyl, 3-furylmethyl, 2-pyridylmethyl, 3-pyridylmethyl, 4-pyridylmethyl, 2-indolylmethyl, 3-indolylmethyl, 2-benzothienylmethyl and 3-benzothienylmethyl groups.

In $X^1$, $R^4$ means a hydrogen atom or a $C_{1-6}$ alkyl group. Examples of the $C_{1-6}$ alkyl group are methyl and ethyl groups.

$R^5$ is a 3-indolylmethyl group wherein the 1- and/or 2-position of the indole ring may be substituted by 1 to 2 substituents selected from the group consisting of a halogen atom, a $C_{1-6}$ alkoxy group, a $C_{2-6}$ alkanoyloxy group, a $C_{1-6}$ alkylthio group, and a $C_{1-6}$ alkoxycarbonyl group, such as, 3-indolylmethyl, (1-methoxycarbonyl-3-indolyl)methyl, (1-ethoxycarbonyl-3-indolyl)methyl, (1-propoxycarbonyl-3-indolyl)methyl, (1-isopropoxycarbonyl-3-indolyl)methyl, (1-butoxycarbonyl-3-indolyl)methyl, (1-isobutoxycarbonyl-3-indolyl)methyl, (1-sec-butoxycarbonyl-3-indolyl)methyl, 1-tert-butoxycarbonyl-3-indolyl)methyl, (1-acetoxy-3-indolyl)methyl, (2-acetoxy-3-indolyl)methyl, (1-methoxy-3-indolyl)methyl, (2-bromo-3-indolyl)methyl, (2-chloro-3-indolyl)methyl, (2-fluoro-3-indolyl)methyl, (2-chloro-1-methyl-3-indolyl)methyl and (2-methylthio-3-indolyl)methyl groups.

$R^6$ means a $C_{1-6}$ alkyl or $C_{2-6}$ alkenyl group which may have a substituent selected from the group consisting of $C_{1-6}$ lower alkoxy, and $C_{1-6}$ alkylthio groups, such as, methyl, ethyl, propyl, isopropyl, butyl, isobutyl, neopentyl, tert-pentyl, hexyl, methoxymethyl, 1-methoxyethyl, methylthiomethyl, ethylthiomethyl, propylthiomethyl, butylthiomethyl, 2-methylthioethyl, 2-ethylthioethyl, vinyl, allyl, 2-propenyl, isopropenyl, 3-butenyl, 2-butenyl, 1-butenyl, 1-methyl-2-propenyl, 1-methyl-1-propenyl, 1-ethyl-1-ethenyl, 2-methyl-2-propenyl, 2-methyl-1-propenyl, 4-pentenyl, 1-methoxy-2-propenyl, 1-methylthio-2-propenyl, and 2-ethylthio-3-butenyl groups.

$R^{71}$ means a hydrogen atom or a $C_{1-6}$ alkyl group. Examples of the $C_{1-6}$ alkyl group are methyl, ethyl, propyl, isopropyl, butyl, isobutyl, sec-butyl, tert-butyl, pentyl, isopentyl, neopentyl and hexyl groups.

$R^{72}$ means a hydrogen atom or a $C_{1-6}$ alkyl group which may be substituted by a substituent selected from the group consisting of hydroxyl, carboxyl, and sulfo groups. Examples of the $C_{1-6}$ alkyl group which may be substituted by a substituent selected from the group consisting of hydroxyl, carboxyl, and sulfo groups, are 2-hydroxyethyl, carboxymethyl, 1-carboxyethyl, 2-carboxyethyl, sulfomethyl and 2-sulfoethyl groups.

Now, the process for producing the novel peptide derivatives of the present invention will be described.

A peptide derivative of the present invention can be prepared by a method wherein amino acids composing the target peptide derivative are condensed one by one, and then, if necessary, removing a C-terminal protective group (Method 1). Peptide derivatives prepared by Method 1 can be converted into other peptide derivatives of the present invention, as the case requires, by a combination of the following reactions: (1) alkoxycarbonylation, aryloxycarbonylation or carbamoylation of the N-terminal $\alpha$-amino group after removal of an N-terminal amino-protecting group (Methods 2 and 3), (2) alkoxycarboylation (Method 4) or hydroxylation or alkoxylation (Method 5) at the 1-position of the indole ring of tryptophan, and (3) halogenation (Method 6), or lower alkylthiolation, lower alkylsulfinylation or lower alkylsulfonation (Method 7) at the 2-position of the indole ring of tryptophan. Furthermore, these peptide derivatives can, if necessary, be converted to pharmaceutically acceptable salts.

Each method will be detailed as follows.

[Method 1]

Method 1 is a conventional synthetic method for peptides, that is, a method wherein amino acids or amino acid derivatives are condensed one by one to prepare a desired peptide derivative. Furthermore, after condensation, a C-terminal protective group can be removed by alkaline hydrolysis, catalytic hydrogenation or acid decomposition. Condensation can be conducted according to known methods such as a DCC method, an azide method, an active ester method and a mixed acid anhydride method (described, for example, by M. Bodansky and M. A. Ondetti in Peptide Synthesis, Interscience, New York, 1966; by F. M. Finn and K. Hofmann in The Proteins, Vol. 2, ed. by H. Nenrath and R. L. Hill, Academic Press Inc., New York, 1976; by Nobuo Izumiya et al. in Peptide Synthesis, Maruzen, 1975).

For example, in the case wherein condensation is carried out by a DCC method, an $\alpha$-amino acid derivative or an $\alpha$-hydroxycarboxylic acid derivative of the formula

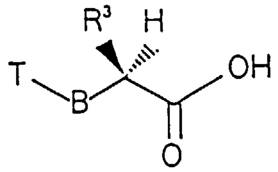

(II)

wherein T is A or an α-amino-protecting group, and A, B and $R^3$ are as defined before, is treated with a condensing reagent such as DCC(or EDCI • HCl)-EOBT • $H_2O$ in a suitable solvent such as DMSO, NMP, DMF, THF, 1,4-dioxane, acetonitrile, dichloromethane or chloroform at around -40°C to room temperature, then condensed with an α-amino acid derivative or an α-hydroxycarboxylic acid derivative having an α-carboxyl-protecting group, of the formula:

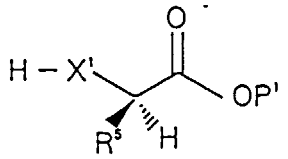

(III)

wherein $P^1$ is an α-carboxyl-protecting group, and $X^1$ and $R^5$ are as defined before, to afford a dipeptide derivative of the formula:

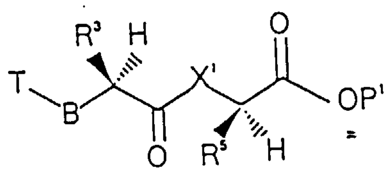

(IV)

wherein T, B, $R^3$, $R^5$, $X^1$ and $P^1$ are as defined before. An N-terminal α-amino-protecting group is usually selected from the groups well-known to those skilled in the art, for example, from urethane type protective groups such as a Z group, a Boc group, a p-methoxybenzyloxycarbonyl group and a p-nitrobenzyloxycarbonyl group, while the C-terminal α-carboxyl group is usually protected as, for example, a methyl ester, an ethyl ester, a benzyl ester or a tert-butyl ester. Each protective group should be selected so that it can be selectively deprotected after condensation. For example, in the case that a Boc group is selected as an N-terminal protective group, it is preferable to protect the C-terminus as a methyl group, an ethyl group or a benzyl group. A Boc group will be readily removed by use of a mild acid such as TFA, while the carboxyl-protecting groups described above will be usually intact under these conditions. On the other hand, a methyl, ethyl or benzyl ester will be easily deprotected by alkaline hydrolysis and a benzyl ester will be also deprotected by catalytic hydrogenation, while a Boc group will be intact under these conditions.

In the case that T is an α-amino-protecting group, the group T will be formally converted to A by removal of T from the dipeptide derivative (IV) followed by N-alkoxycarbonylation, N-aryloxycarbonylation or N-carbamoylation which will be carried out under the reaction conditions described later in Method 2.

A C-terminal protective group of the dipeptide derivative (IV) prepared in the above-mentioned manner is now removed, and the resulting deprotected dipeptide is treated with a condensation reagent (for example, EDCI • HCl-HOBT • $H_2O$) in the same manner described above and then with an amino acid whose C-terminal carboxyl group is protected, to afford a desired peptide derivative.

In the case that each of B and $X^1$ is $-NR^2-$ or $-NR^4-$, and $P^1$ is a lower alkyl group or a benzyl group, the dipeptide derivative (IV) may be treated with an excess amount of hydrazine in a solvent such as methanol or DMF at room temperature to afford the corresponding hydrazide, which can be converted to a desired peptide derivative by an azide method. Namely, the hydrazide is first converted to the corresponding azide on treatment with a reagent such as a lower alkyl ester of nitrous acid (for example, tert-butyl nitrite or isoamyl nitrite) or an alkaline metal salt of nitrous acid (for example, sodium nitrite or potassium nitrite) in the presence of a strong acid such as hydrochloric acid or sulfuric acid (this reaction can be performed in a solvent such as water, and/or DMF, THF or 1,4-dioxane at around -60°C to -15°C). Then, the azide is mixed with a tertiary amine such as TEA, and a C-terminal ester derivative of an amino acid

at -70°C to -60°C, and then allowed to react at -20°C to room temperature to afford a desired peptide derivative. A tetrabutylammonium-, triethylammonium-, sodium- or potassium-salt of an amino acid can also be used instead of the C-terminal ester derivative.

In the process so far described, a C-terminal amino acid or a C-terminal dipeptide is lastly condensed to give a target peptide derivative. The alternative process wherein an N-terminal amino acid is lastly condensed to give a target product is also available. Namely, a compound of the formula:

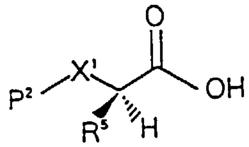

(V)

wherein $P^2$ is a hydrogen atom or an α-amino-protecting group, and $R^5$ and $X^1$ are as defined before, is condensed with a compound of the formula:

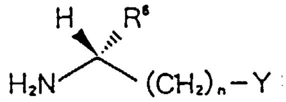

(VI)

wherein n, $R^6$ and Y are as defined before, by a DCC method or an azide method to afford an N-terminal protected peptide derivative or α-hydroxycarboxylic acid derivative. A suitable α-amino-protecting group can be selected from the urethane type protective groups described before, and a C-terminal carboxyl group can be protected as an ester. In the case that a C-terminal carboxyl group is protected as, for example, a tert-butyl ester, a Z group or a Boc group is preferable for a N-terminal amino-protecting group. A Z group will be readily removed by catalytic hydrogenation and a Boc group will be readily removed by treatment with a mild acid such as formic acid or TFA under ice-cooling, while under these conditions these C-terminal carboxyl-protecting groups will be intact.

An α-hydroxycarboxylic acid derivative or a dipeptide derivative (in the case of a dipeptide derivative, an N-terminal amino protecting group is removed prior to following treatments) prepared in the above-mentioned manner is treated with a condensation reagent (for example, EDCI • HCl-HOBT • $H_2O$) in the same manner described above and then with an $N^{\alpha}$-substituted amino acid or an $O^{\alpha}$- substituted hydroxycarboxylic acid, to afford a desired peptide derivative. A peptide derivative of the formula (I) wherein $X^3$ is a sulfur atom, can be prepared by condensation of a compound of the formula (V) with a compound of the formula (VI) whose C-terminal carboxyl group is protected, followed by conversion of the resulting amide bond to the thioamide bond on treatment with, for example, the Lawesson's reagent, then condensed with a compound of the formula (II) by, for example, a DCC method or an azide method to afford a target peptide derivative elongated toward the N-terminus.

A C-terminal protective group of a peptide derivative prepared by the method so far described, can be deprotected by a suitable method, if necessary. For example, in the case that a carboxyl group is protected as a tert-butyl ester, the protective group can be readily removed by acid decomposition, that is, by treatment with a mild acid such as formic acid or TFA. In the case that a carboxylic acid is protected as a benzyl ester, the protective group can be readily removed by catalytic hydrogenation, that is, by hydrogenation under 1 to 4 atmospheric pressures of hydrogen in the presence of a catalyst such as Pd-C or palladium black in a solvent such as methanol, ethanol, DMF, THF, 1,4-dioxane or acetic acid.

[Method 2]

Method 2 is a process for producing a peptide derivative which possesses an alkoxycarbonyl, aryloxycarbonyl or carbamoyl group at the N-terminus, wherein an N-terminal amino protective group of an amino acid derivative or a peptide derivative prepared by Method 1, is removed, and the resulting deprotected amino group is reacted with an acid chloride such as a chloroformate ($R^{11}OCOCl$) or a carbamoyl chloride ($R^{12}R^{13}NCOCl$) in the presence of a base, or reacted with an isocyanate ($R^{12}NCO$) (wherein $R^{11}$, $R^{12}$ and $R^{13}$ are as defined before), furthermore, a C-terminal protective group is optionally removed by alkaline hydrolysis, catalytic hydrogenation or acid decomposition.

An N-terminal protective group of the precursor can be readily removed by a conventional method such as catalytic hydrogenation (a Z group) or by treatment with a mild acid such as TFA (a Boc group). The reaction of the

resulting deprotected peptide derivative from which the N-terminal amino protective group is removed by the method described above, with an acid chloride such as a chloroformate ($R^{11}OCOCl$) or a carbamoyl chloride ($R^{12}R^{13}NCOCl$) can be performed in a suitable solvent such as chloroform, dichloromethane, THF, 1,4-dioxane, toluene or pyridine in the presence of a base such as TEA, DMAP, N-methylmorpholine or pyridine at 0°C to the boiling point of the solvent. The reaction with an isocyanate ($R^{12}NCO$) can be performed in a solvent such as chloroform, dichloromethane, THF, 1,4-dioxane or toluene at 0°C to the boiling point of the solvent.

A C-terminal protective group of peptide derivatives prepared by the above-mentioned method can be removed by alkaline hydrolysis, catalytic hydrogenation or acid decomposition in the same manner described in Method 1, if necessary.

[Method 3]

Method 3 is a process for producing an amino acid or a peptide derivative which possesses a carbamoyl group at the N-terminus, by treatment of an amino acid derivative or a peptide derivative (prepared by Method 1 or 2) having an aryloxycarbonyl group at the N-terminus, with a primary or secondary amine $R^{12}NHR^{13}$ wherein $R^{12}$ and $R^{13}$ are as defined before, furthermore optionally removing a C-terminal protective group by alkaline hydrolysis, catalytic hydrogenation or acid decomposition.

That is, a peptide derivative possessing a carbamoyl group at the N-terminus can be prepared by dissolving a peptide derivative possessing an aryloxycarbonyl group at the N-terminus in a solvent such as chloroform, dichloromethane, THF, 1,4-dioxane, toluene or pyridine, followed by addition of the primary or secondary amine described above, optional addition of a tertiary amine such as TEA or DMAP, and allowing them to react at room temperature to the boiling point of the solvent. A C-terminal protective group of the product can be removed, if necessary, by alkaline hydrolysis, catalytic hydrogenation or acid decomposition in the same manner described in Method 1.

[Method 4]

Method 4 is a process for alkoxycarbonylation at the 1-position of the indole ring of a tryptophanyl residue when the peptide derivative prepared in the present invention contains the tryptophanyl residue.

The alkoxycarbonylation of the indole ring at the 1-position can be performed by treatment of a tryptophan-containing dipeptide or tripeptide derivative prepared by optionally combining Methods 1, 2 and 3, or an optionally protected tryptophan derivative as the starting material thereof, with a base (such as sodium hydroxide) and alkyl chloroformate in a solvent such as dichloromethane in the presence of TBAHS.

[Method 5]

Method 5 is a process for hydroxylation or lower alkoxylation at the 1-position of the indole ring when the peptide derivative prepared in the present invention contains a tryptophanyl residue.

The hydroxylation of the indole ring at the 1-position can be performed by treatment of a tryptophan-containing dipeptide or tripeptide derivative prepared by optionally combining Methods 1, 2, and 3, or an optionally protected tryptophan derivative as the starting material thereof, with sodium cyanoborohydride in a solvent such as acetic acid, and treatment of the resulting indoline derivative with hydrogen peroxide in methanol, water or a mixed solvent thereof. The lower alkoxylation of the indole ring at the 1-position can be performed by treatment of the hydroxylated derivative with, for example, diazomethane or diazoethane.

[Method 6]

Method 6 is a process for halogenation at the 2-position of the indole ring of a tryptophanyl residue when the peptide derivative prepared in the present invention contains the tryptophanyl residue.

The halogenation of the indole ring at the 2-position can be performed by treatment of a tryptophan-containing dipeptide or tripeptide derivative prepared by optionally combining methods 1, 2 and 3, or an optionally protected tryptophan derivative as the starting material thereof, with an N-halosuccinimide such as N-bromosuccinimide or N-chlorosuccinimide in a solvent such as acetic acid or carbon tetrachloride, optionally in the presence of 2,2'-azobis(isobutyronitrile) or the like.

[Method 7]

Method 7 is a process for $C_{1-6}$ alkylthiolation at the 2-position of the indole ring of a tryptophanyl residue when the peptide derivative prepared in the present invention contains the tryptophanyl residue.

The $C_{1-6}$ alkylthiolation of the indole ring at the 2-position can be performed by treatment of a tryptophan-containing dipeptide or tripeptide derivative prepared by optionally combining methods 1, 2 and 3, or an optionally protected tryptophan derivative as the starting material thereof, with a $C_{1-6}$ alkylsulfenyl chloride in a solvent such as acetic acid.

All reaction intermediates and products so far described can be purified by well-known methods such as recrystallization, reprecipitation, partition procedures, normal- or reverse-phase chromatography and ion-exchange chromatography.

Starting materials used in the methods so far described are commercially available except for the following materials, which are prepared by the known method in the literature.

DL-3-(3-ethoxycarbonylphenyl)alanine and DL-3-(4-methoxycarbonylphenyl)alanine: Synthesis, 53 (1984).
D-3-(3-benzothienyl)alanine: Chem. Pharm. Bull., 24, 3149 (1976).
D-S-methylcysteine, D-S-ethylcysteine, D-S-n-propylcysteine: by Nobuo Izumiya et al in Peptide Synthesis, Maruzen, 1975.
D-norleucinol: synthesized in the same manner described in J. Am. Chem. Soc., 107, 7974 (1985).
D-3-cyclopropylalanine: J. Am. Chem. Soc., 111, 6354 (1989).
D-cyclopropylglycine: J. Am. Chem. Soc., 111, 6354 (1989).
D-cyclopentylglycine: J. Org. Chem., 30, 1320 (1965).
D-3-(3-nitrophenyl)alanine: Pept. Res.), 3, 176 (1990).
DL-1-aminopentanesulfonic acid: European Laid-open Patent Publication No. 33504.
D-1-aminopentylphosphonic acid diethyl ester: synthesized in the same manner described in Liebigs Ann. Chem., 45 (1987).
D-(7-benzyloxy)tryptophan: synthesized in the same manner described in Tetrahedron Letters, 30, 4073 (1989).
(R)-5-(1-aminopentyl)-1H-tetrazole hydrochloride: synthesized in the same manner described in J. Org. Chem., 56, 2395 (1991).
α-N-trifluoroacetyl-2-chloro-D-tryptophan methyl ester and
α-N-trifluoroacetyl-1-methyl-2-chloro-D-tryptophan: synthesized in the same manner described in J. Am. Chem. Soc., 108, 2023 (1986).

The chemical structures, Example Nos. and compound Nos. of the prepared peptide derivatives in the present invention are shown in Tables 1 to 6.

Table 1

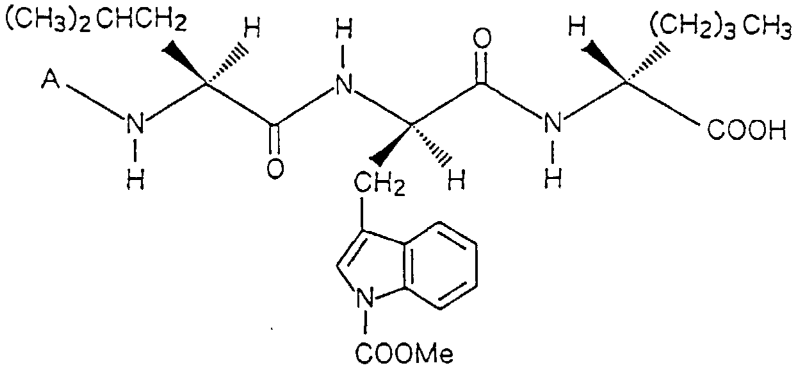

| Exp. No. | Compd No. | A | Exp. No. | Compd No. | A |
|---|---|---|---|---|---|
| | | | 12 | 12 | 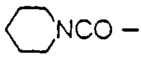 |
| 1 | 1 | 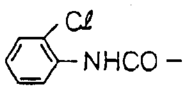 | 13 | 13 | 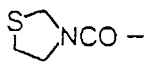 |
| 3 | 3 | 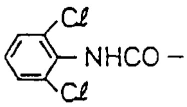 | 14 | 14 | 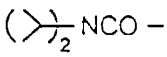 |
| 4 | 4 | 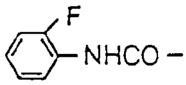 | 15 | 15 | 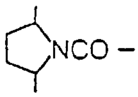 |
| 5 | 5 | 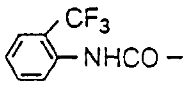 | 16 | 16 | 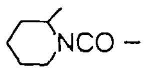 |
| 6 | 6 | 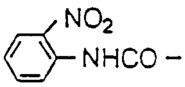 | 17 | 17 | 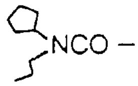 |
| 7 | 7 | 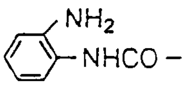 | 18 | 18 | 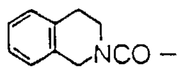 |
| 8 | 8 | 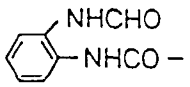 | 19 | 19 | 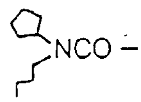 |
| | | | 20 | 20 | 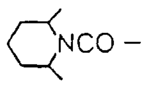 |
| 9 | 9 | 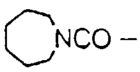 | 25 | 25 | 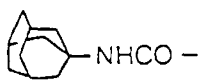 |
| 10 | 10 | 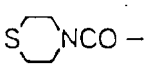 | 27 | 27 | 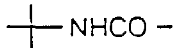 |
| 11 | 11 | 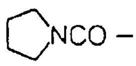 | | | |

Table 2

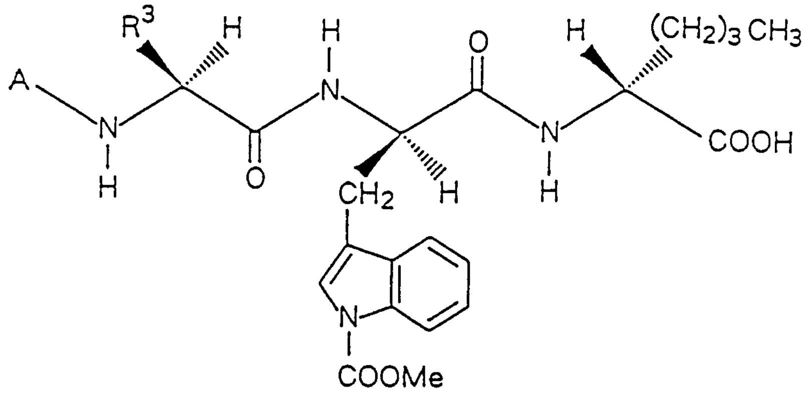

| Exp. No. | Compd No. | A | $R^3$ |
|---|---|---|---|
| 21 | 21 | 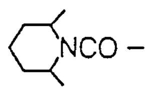 | $-CH_2C(CH_3)_3$ |
| 22 | 22 | 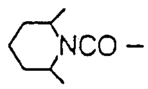 | $-CH_2Ph$ |
| 23 | 23 | 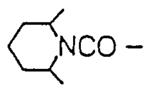 | 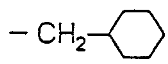 |
| 24 | 24 | 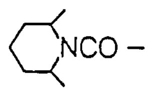 | $-CH(CH_3)_2$ |
| 39 | 41 | 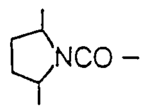 | $-CH(CH_3)_2$ |
| 40 | 42 | 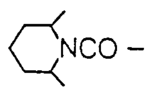 | 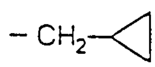 |
| 41 | 43 | 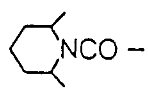 | 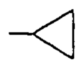 |
| 42 | 44 | 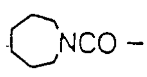 | 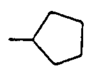 |

Table 3

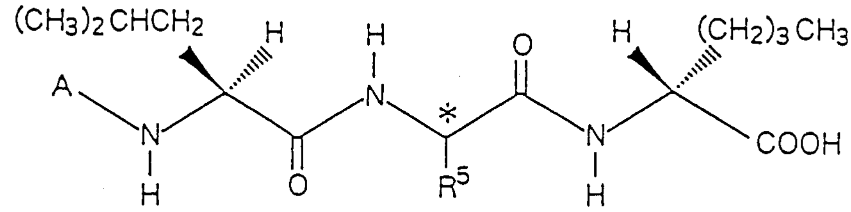

| Exp. No. | Compd No. | A | * | $R^5$ |
| --- | --- | --- | --- | --- |
| 2 | 2 | 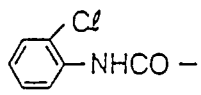 | (R) | 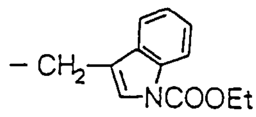 |
| 62 | 67 | 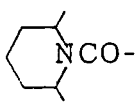 | (R) | 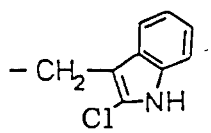 |
| 63 | 68 | 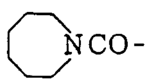 | (R) | 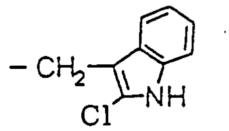 |

Table 4

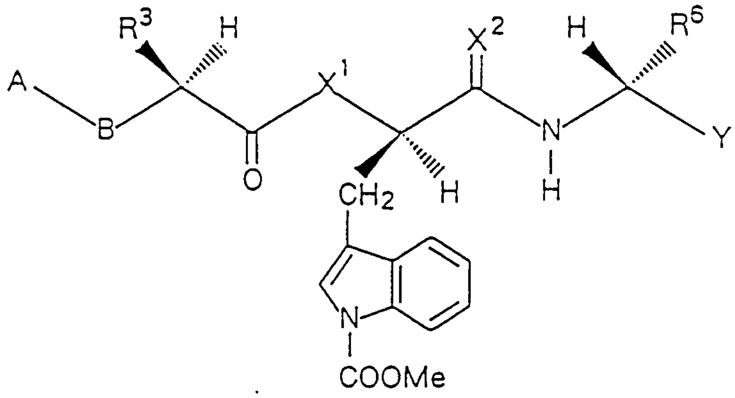

| Exp. No. | Comp. No. | A | B | $R^3$ | $X^1$ | $X^2$ | $R^6$ | Y |
|---|---|---|---|---|---|---|---|---|
| 28 | 28 | 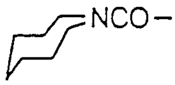 | -NH- | $-CH_2CH(CH_3)_2$ | NH | O | $-(CH_2)_3CH_3$ | $COO^tBu$ |
| 28 | 29 | 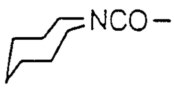 | -NH- | $-CH_2CH(CH_3)_2$ | NH | O | $-(CH_2)_3CH_3$ | COOH |
| 29 | 30 | 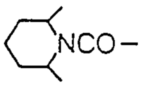 | -NH- | $-CH_2CH(CH_3)_2$ | NH | O | $-(CH_2)_2CH_3$ | COOH |
| 30 | 31 | 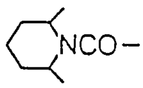 | -NH- | $-CH_2C(CH_3)_3$ | NH | O | $-(CH_2)_2CH_3$ | COOH |
| 31 | 32 | 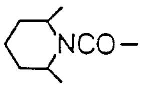 | -NH- | (S) $-\overset{*}{C}HCH_2CH_3$<br>$CH_3$ | NH | O | $-(CH_2)_3CH_3$ | COOH |
| 32 | 33 | 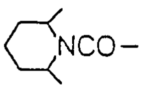 | -NH- | (R) $-\overset{*}{C}HCH_2CH_3$<br>$CH_3$ | NH | O | $-(CH_2)_3CH_3$ | COOH |
| 33 | 34 | 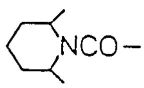 | -NH- | $-C(CH_3)_3$ | NH | O | $-(CH_2)_3CH_3$ | COOH |
| 34 | 35 | 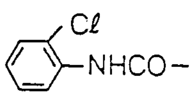 | -O- | $-CH_2CH(CH_3)_2$ | NH | O | $-(CH_2)_3CH_3$ | COOH |

Table 4 (continued)

| Exp. No. | Comp No. | A | B | $R^3$ | $X^1$ | $X^2$ | $R^6$ | Y |
|---|---|---|---|---|---|---|---|---|
| 35 | 36 | 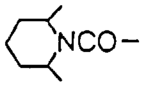 | $-NH-$ | $-CH_2C(CH_3)_3$ | NH | S | $-(CH_2)_3CH_3$ | $COO^tBu$ |
| 35 | 37 | 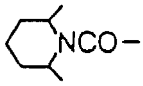 | $-NH-$ | $-CH_2C(CH_3)_3$ | NH | S | $-(CH_2)_3CH_3$ | COOH |
| 36 | 38 | 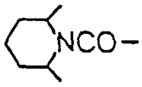 | $-NH-$ | $-CH_2C(CH_3)_3$ | O | O | $-(CH_2)_3CH_3$ | COOH |
| 37 | 39 | 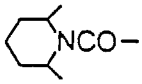 | $-NH-$ | $-CH_2C(CH_3)_3$ | NH | O | $-(CH_2)_3CH_3$ | $COO^tBu$ |
| 38 | 40 | 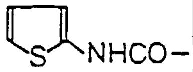 | $-NH-$ | $-CH_2C(CH_3)_3$ | NH | O | $-(CH_2)_3CH_3$ | COOH |

Table 5

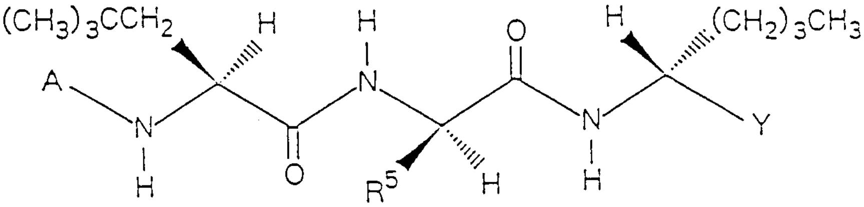

| Exp. No. | Compd No. | A | $R^5$ | Y |
|---|---|---|---|---|
| 43 | 46 | 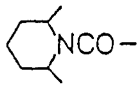 | 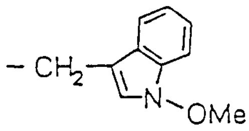 | COOH |
| 47 | 50 | 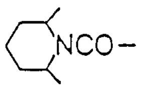 | 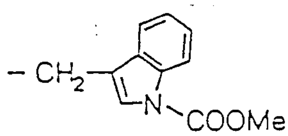 | $CH_2OH$ |
| 47 | 51 | 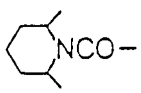 | 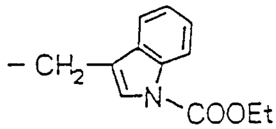 | $CH_2OH$ |

Table 5 (continued)

| Exp. No. | Compd No. | A | $R^5$ | Y |
|---|---|---|---|---|
| 60 | 65 | 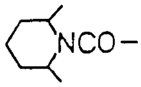 | 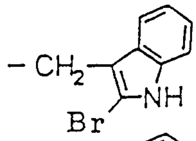 | COOH |
| 61 | 66 | 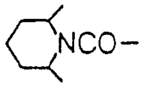 | 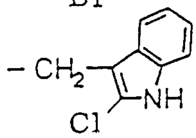 | COOH |
| 64 | 69 | 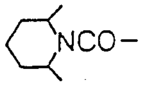 | 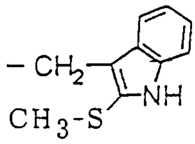 | COOH |
| 65 | 70 | 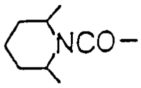 | 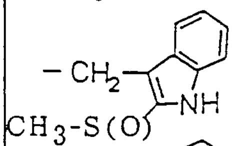 | COOH |
| 66 | 71 | 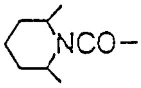 | 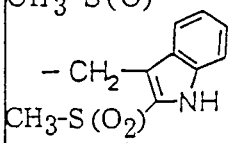 | COOH |

Table 6

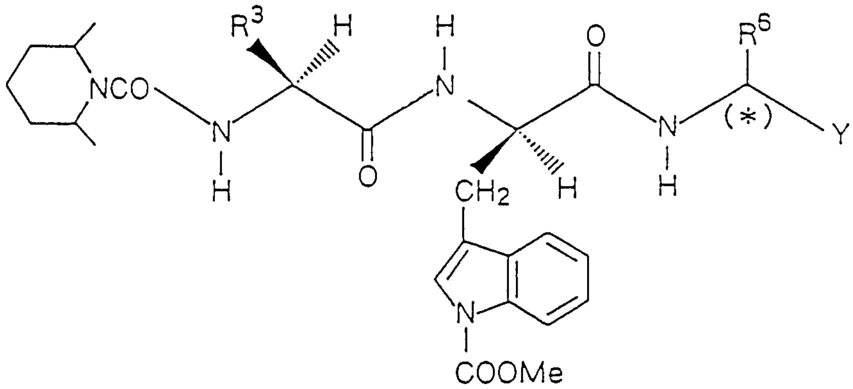

| Exp. No. | Compd No. | $R^3$ | $R^6$ | * | Y |
|---|---|---|---|---|---|
| 44 | 47 | $-CH_2C(CH_3)_3$ | $-(CH_2)_3CH_3$ | (R) | COOMe |
| 45 | 48 | $-CH_2C(CH_3)_3$ | $-(CH_2)_3CH_3$ | (R) | COOEt |
| 46 | 49 | $-CH_2C(CH_3)_3$ | $-(CH_2)_3CH_3$ | (R) | $COO^iPr$ |
| 48 | 52 | 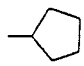 | $-(CH_2)_3CH_3$ | (R) | $COO^tBu$ |
| 48 | 53 | 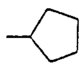 | $-(CH_2)_3CH_3$ | (R) | COOH |
| 49 | 54 | 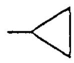 | $-CH_3$ | (R) | $CH_2COOH$ |

Table 7 (continued)

| Exp. No. | Compd No. | $R^3$ | $R^6$ | * | Y |
|---|---|---|---|---|---|
| 50 | 55 | $-CH_2C(CH_3)_3$ | $-(CH_2)_3CH_3$ | (R) | $CH_2COOH$ |
| 51 | 56 | $-CH_2C(CH_3)_3$ | $-(CH_2)_3CH_3$ | (R) | $CH_2SO_3H$ |
| 52 | 57 | $-CH_2C(CH_3)_3$ | $-(CH_2)_3CH_3$ | (RS) | $SO_3H$ |
| 53 | 58 | $-CH_2C(CH_3)_3$ | $-(CH_2)_3CH_3$ | (S) | $PO_3H_2$ |
| 54 | 59 | $-CH_2C(CH_3)_3$ | $-(CH_2)_3CH_3$ | (R) | $CONHCH_2CH_2OH$ |
| 55 | 60 | $-CH_2C(CH_3)_3$ | $-(CH_2)_3CH_3$ | (R) | 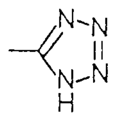 |
| 56 | 61 | $-CH(CH_3)_2$ | $-(CH_2)_3CH_3$ | (RS) | $SO_3H$ |
| 57 | 62 | $-CH(CH_3)_2$ | $-(CH_2)_3CH_3$ | (R) | $CH_2SO_3H$ |
| 58 | 63 | $-CH_2CH(CH_3)_2$ | $-(CH_2)_3CH_3$ | (R) | $CH_2SO_3H$ |
| 59 | 64 | $-CH_2CH(CH_3)_2$ | $-(CH_2)_3CH_3$ | (RS) | $SO_3H$ |

Now, the endothelin antagonistic properties of the peptide derivatives of the present invention will be described.

Endothelin binding inhibition test

The cerebellum of porcine was homogenized in a buffer solution of 10 mM MOPS, pH 7.4, at 4°C by a polytron. To the homogenate, sucrose was added to a concentration of 20%, and the mixture was centrifuged at 1,000 x g for 15 minutes, and the supernatant was further centrifuged at 10,000 x g for 15 minutes. The supernatant thereof was further centrifuged at 90,000 x g for 40 minutes. The membrane precipitate thereby obtained was suspended in a buffer solution of 5 mM HEPES/Tris, pH 7.4, at a concentration of 3.3 mg/ml.

Then, 16 μl of this membrane suspension was added to 340 μl of 50 mM tris/HCl buffer, pH 7.4, containing 10 μl calcium chloride, 10 μM magnesium chloride, 0.1 mM PMSF, 1 μM pepstatin A, 2 μM leupeptin, 1 mM 1,10-phenanthroline and 0.1% bovine serum albumin. To this suspension, 4 μl of (A) endothelin-1 (for nonspecific binding; 0.2 μM as the final concentration), (B) buffer solution A (for total control binding), or (C) a test compound (1.1 μM as the final concentration), was added. Further, to each suspension, 40 μl of $^{125}$I-endothelin-1 (12000-18000 cpm) was added. These mixtures were incubated at 25°C for 4 hours, then subjected to filtration on a glass filter GF/C and then washed with 5 mM HEPES/Tris, pH 7.4, containing 0.3% bovine serum albumin. Then, the radioactivity trapped by the glass filter was measured, and the $^{125}$I-endothelin-1 binding inhibition D (%) at 1.1 μM of the test compound was determined by the following equation.

$$D(\%) = 100 - \frac{(C) - (A)}{(B) - (A)} \times 100$$

Each test was performed in triplicate.

Table 7

| $^{125}$I-endothelin-1 binding inhibition by 1.1 μM of the test compound | | | | | | | |
|---|---|---|---|---|---|---|---|
| Compd No. | Inhibition(%) | Compd No. | Inhibition(%) | Compd No. | Inhibition(%) | Compd No. | Inhibition(%) |
| 1 | 99 | 18 | 90 | 35 | 100 | 52 | 96 |
| 2 | 97 | 19 | 84 | 36 | 89 | 53 | 100 |
| 3 | 94 | 20 | 98 | 37 | 98 | 54 | 94 |
| 4 | 93 | 21 | 99 | 38 | 95 | 55 | 100 |
| 5 | 100 | 22 | 67 | 39 | 95 | 56 | 98 |
| 6 | 97 | 23 | 97 | 40 | 91 | 57 | 99 |
| 7 | 88 | 24 | 99 | 41 | 100 | 58 | 96 |
| 8 | 85 | 25 | 97 | 42 | 100 | 59 | 98 |
| 9 | 99 | 26 | 71 | 43 | 100 | 60 | 100 |
| 10 | 89 | 27 | 91 | 44 | 100 | 61 | 99 |
| 11 | 73 | 28 | 92 | 45 | 84 | 62 | 100 |
| 12 | 96 | 29 | 99 | 46 | 96 | 63 | 99 |
| 13 | 72 | 30 | 99 | 47 | 98 | 64 | 99 |
| 14 | 99 | 31 | 99 | 48 | 98 | 65 | 98 |
| 15 | 99 | 32 | 100 | 49 | 94 | 66 | 99 |
| 16 | 97 | 33 | 100 | 50 | 99 | 67 | 97 |
| 17 | 95 | 34 | 97 | 51 | 96 | 68 | 93 |
| | | | | | | 69 | 88 |

As shown in Table 7, the compounds of the present invention were found to be very potent inhibitor of endothelin binding to $ET_B$ receptor. The test compounds are indicated by compound Nos.

**[0077]** On the other hand, each of the representative compounds (Reference Compounds 1, 2 and 3) of the

endothelin antagonistic peptides described in EP 0460679A2 showed, at a concentration of 1.1 μM, 69, 77 or 86% of the $^{125}$I-ET-1 binding inhibition to porcine aorta membrane suspension containing a large amount of $ET_A$ receptors, and, on the contrary, only 6.4, 3.8 or 10.1% of the $^{125}$I-ET-1 binding inhibition to $ET_B$ receptors of porcine cerebellum membrane suspension used in the present experiments.

Reference Compound 1

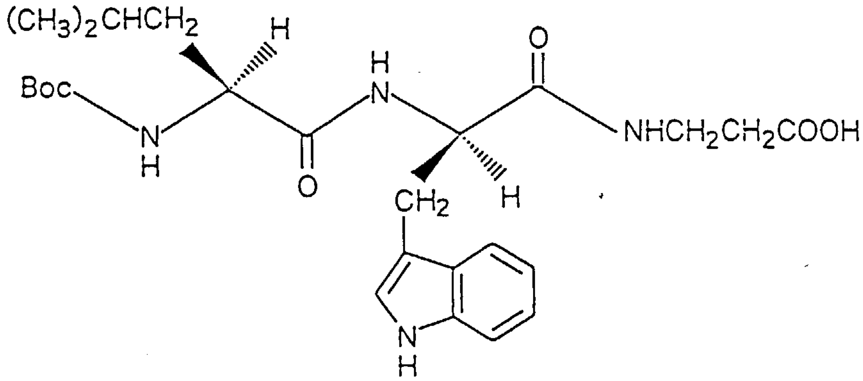

Reference Compound 2

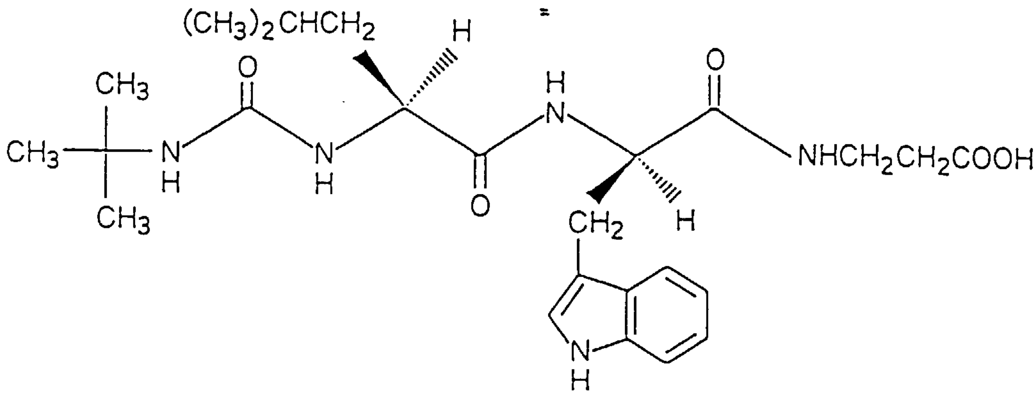

Reference Compound 3

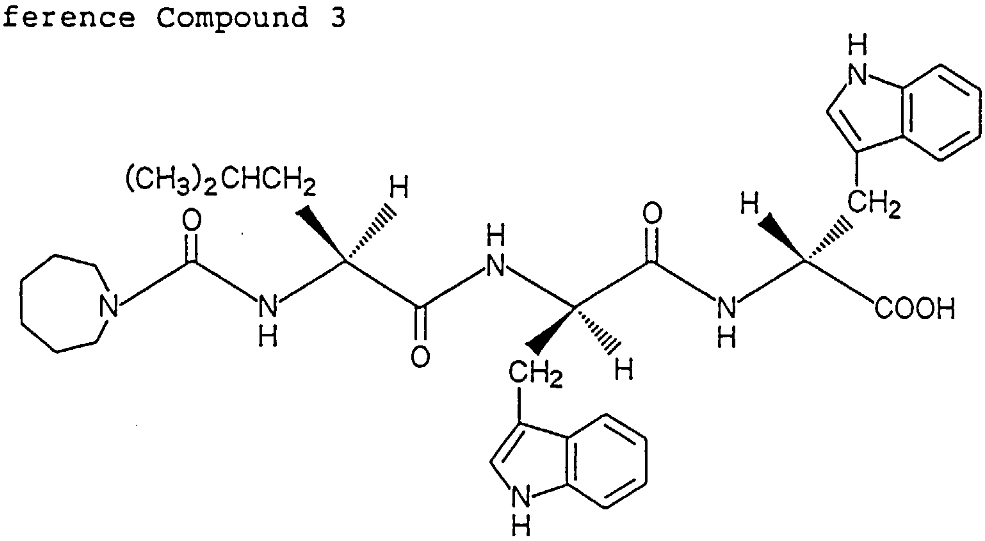

Activities against endothelin-induced contraction of isolated rabbit pulmonary arteries

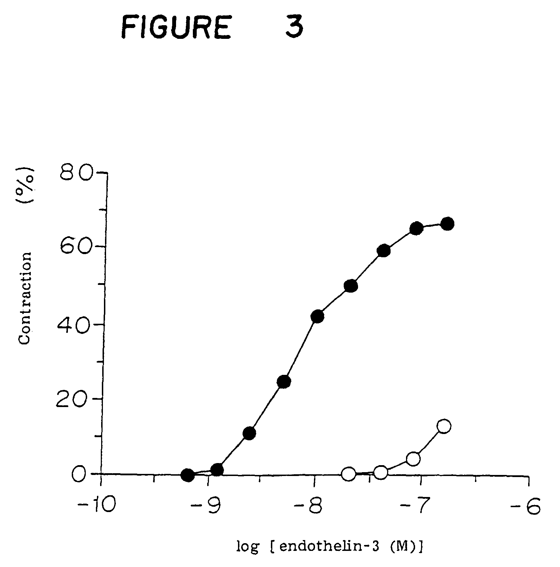

The pulmonary artery of rabbit was isolated, and a spiral preparation having a width of 1 mm and a length of 10 mm was prepared therefrom. The preparation having the endothelial cells denuded, was placed in a 5 ml organ bath filled with a Krebs • Henseleit solution saturated with a gas mixture of 95% $O_2$ and 5% $CO_2$, and a change in the tension was isometrically measured and recorded.
Endothelin-3 was added into the organ bath in a cumulatively increasing manner, whereby the influence of a compound of the present invention to the concentration-response curve for endothelin-3 was examined. The compound was added into the organ bath 20 minutes prior to the addition of endothelin-3.
As shown in Figures 1 to 3, Compound 1, Compound 20 and Compound 21 (0.1 to 10 μM) remarkably shifted the concentration-response curves of endothelin-3 to the right. Table 8 shows $pA_2$ values (-log of the concentration of the compound necessary for shifting the concentration-response curve for endothelin-3 twice to the right. Further, the compounds showed no effects to the isolated pulmonary artery when applied alone. As is evident from the above, the compounds showed remarkable antagonistic activities against endothelin-induced concentration of isolated rabbit pulmonary artery.

Table 8

| Antagonistic activities of the compound of the present invention against endothelin-3-induced contraction of isolated rabbit pulmonary artery | |
|---|---|
| Compound No. | $PA_2$ value |
| 1 | 6.7 |
| 20 | 6.7 |
| 21 | 7.1 |

Activities against endothelin-induced contraction of isolated guinea pig bronchus

The bronchus of a guinea pig was isolated, and the bronchus was cut into rings having an outer diameter of 2 mm and a width of 4 mm to afford a preparation. The preparation was placed in a 5 ml organ bath filled with a Krebs • Henseleit solution saturated with a gas mixture of 95% $O_2$ and 5% $CO_2$, and a change in the tension was isometrically measured and recorded.
Endothelin-3 was added into the organ bath in a cumulatively increasing manner, and the influence of a compound of the present invention to the concentration-response curve for endothelin was examined. The compound was added into the organ bath 20 minutes prior to the addition of endothelin-3.
As shown in Figures 1 to 3, Compound 1, Compound 20 and Compound 21 (10 μM) remarkably shifted to concentration-response curves for endothelin-3 to the right in isolated bronchus. Further, the compounds showed no effects to the isolated bronchus when applied alone. As is evident from the foregoing, the compounds showed remarkable antagonistic activities against endothelin-induced contraction of isolated guinea pig bronchus.
Thus, the compounds of the present invention have excellent endothelin antagonistic activities and are useful as vasodilators for bronchodilators in the field of medicines, and they can be drugs for treating hypertension, pulmonary hypertension, Raynaud's disease, acute renal failure, myocardial infarction, angina pectoris, cerebral infarction, cerebral vasospasm, arteriosclerosis, asthma, gastric ulcer, diabetes, endotoxin shock endotoxin-induced multiple organ failure or disseminated intravascular coagulation, and/or cyclosporin-induced renal failure or hypertension. When used as drugs for treating such diseases, the compounds of the present invention can be used alone or in combination with other drugs for treatment.
The compounds of the present invention may be used in the form of drug formulations suitable for parenteral administration, oral administration or external administration by mixing them with solid or liquid excipient carriers known in this field. The drug formulations include a liquid formulation such as an injection formulation, an inhalant formulation, a syrup formulation or an emulsion, a solid formulation such as tablets, capsules or granules, and an external drug such as an ointment or a suppository. Further, these drug formulations may contain additives which are commonly employed, such as an adjuvant, a stabilizer, a wetting agent, an emulsifier, an absorption-promoting agent or a surfactant, as the case requires. As the additives, distilled water for injection, physiological saline, Ringer's solution, glucose, sugar syrup, gelatin, vegetable oil, cacao butter, ethylene glycol, hydroxypropyl cellulose, lactose, sucrose, corn starch, magnesium stearate and talc may be mentioned.

The dose of a compound of the present invention as an endothelin antagonist varies depending upon the manner of administration, the age and body weight of the patient and the condition of the patient to be treated. However, a typical administration method for an adult is oral administration or parenteral administration. The daily dose in the case of oral administration to an adult patient is from 0.1 to 100 mg/kg body weight, and the daily dose in the case of parenteral administration is from 0.01 to 10 mg/kg body weight.

The following Examples, Preparative Examples and Referential Examples illustrate the present invention more specifically. It should be understood that the present invention is not limited to these examples alone. Preparative Example 1

(1) Preparation of Boc-Leu-DTrp(COOMe)-OH

To a mixture of Boc-Leu-OH • $H_2O$ (2.5 g), H-DTrp-OBzl (2.9 g) and HOBT • $H_2O$ (1.6 g) in dichloromethane (50 ml) was added EDCI • HCl (2.0 g) under ice cooling. After being stirred at room temperature for 16 h, the mixture was washed with water, 10% aq. citric acid, sat. aq. $NaHCO_3$ and brine successively, dried over $MgSO_4$ and evaporated in vacuo. The residue was triturated with hexane to give Boc-Leu-DTrp-OBzl (4.79 g). To a solution of the dipeptide (753 mg) in dichloromethane (7 ml) were added methyl chloroformate (1 ml), pulverized NaOH (89 mg) and TBAHS (20 mg) under ice cooling. After being stirred of the mixture at room temperature for 30 min, pulverized NaOH (89 mg) was added. This procedure was repeated twice more. The reaction mixture was diluted with dichloromethane, washed with sat. aq. $NaHCO_3$, 10% aq. citric acid, water and brine successively, dried over $MgSO_4$ and evaporated in vacuo. The residue was recrystallized from dichloromethane and petroleum ether to give Boc-Leu-DTrp(COOMe)-OBzl (721 mg) as colorless crystals. The dipeptide (708 mg) was dissolved in methanol (7 ml) - THF (5 ml) and 10% Pd/C (70 mg) was added. The mixture was vigorously stirred at room temperature under an atmospheric pressure of hydrogen for 1 h. The reaction mixture was filtered and the filtrate was evaporated in vacuo to give Boc-Leu-DTrp(COOMe)-OH (580 mg).

FAB-MS(m/e,$(C_{24}H_{33}N_3O_7+H)^+$):476

(2) Preparation of Boc-Leu-DTrp(COOMe)-DNle-OBzl

To a mixture of Boc-Leu-DTrp(COOMe)-OH (50 mg), H-DNle-OBzl • TsOH (46 mg), NMM (13 μl) and HOBT • $H_2O$ (16 mg) in dichloromethane (1 ml) was added EDCI • HCl (22 mg) under ice cooling. After being stirred at room temperature for 2 h, the mixture was diluted with dichloromethane, washed with water, 10% aq. citric acid, sat. aq. $NaHCO_3$ and brine successively, dried over $MgSO_4$ and evaporated in vacuo. The residue was purified by preparative TLC (Merck, Kieselgel 60 $F_{254}$) with chloroform/methanol/acetic acid = 100/10/1 for development to give Boc-Leu-DTrp(COOMe)-DNle-OBzl (67 mg).

FAB-MS(m/e,$(C_{37}H_{50}N_4O_8+H)^+$):679

(3)

A solution of Boc-Leu-DTrp(COOMe)-DNle-OBzl (25 mg) in methanol (1 ml) was hydrogenated over 10% Pd/C at an atmospheric pressure of hydrogen for 1 h. After removal of the catalyst by filtration, the filtrate was concentrated in vacuo. The residue was purified by preparative TLC (3M, Empore Silica Sheet) with chloroform/methanol/acetic acid = 100/10/1 for development and the product was reprecipitated from methanol and water to give the Compound (11 mg).

m.p.: 154-163 °C
IR(KBr,$cm^{-1}$):
3328,2962,2872,1728,1695,1650,1536,1461,1386,1371, 1341,1311,1260,1167,1092,762,747

| High Resolution FAB-MS(m/e,$(C_{30}H_{44}N_4O_8+H)^+$): | |
|---|---|
| Calcd: | 589,3237 |
| Found: | 589,3260 |

$^{1}H$-NMR(300MHz,DMSO-$d_6$, δppm):
0.65(6H,d,J=6.2Hz),0.85(3H,t,J=7.0Hz),0.93-1.10 (2H,m),1.10-1.55(5H,m),1.31(9H,s),1.55-1.82(2H,m), 2.86(1H,dd,J=10.6Hz,15.2Hz),3.00-3.30(1H,m),3.80-3.90(1H,m),3.95(3H,s),4.05-4.20(1H,m),4.59-4.70 (1H,m),6.74(1H,d,J=7.1Hz),7.23(1H,t,J=7.5Hz),7.31 (1H,t,J=7.5Hz),7.49(1H,s),7.70(1H,d,J=7.5Hz),8.04 (2H,d,J=7.5Hz),8.20(1H,d,J=8.3Hz)

Example 1

Synthesis of Compound 1

A mixture of Boc-Leu-DTrp(COOMe)-DNle-OBzl (20 mg, prepared in Preparative Example 1-(2)) in TFA (0.5 ml) was stirred under ice cooling for 15 min and evaporated in vacuo. To a solution of the residue in chloroform (0.5 ml) were added TEA (6 µl) and 2-chlorophenylisocyanate (4 µl) under ice cooling. After being stirred at the same temperature for 1 h, the mixture was diluted with chloroform, washed with 1N-hydrochloric acid, 5% aq. $NaHCO_3$, and brine successively, dried over $MgSO_4$ and evaporated in vacuo. The residue was purified by preparative TLC (Merck, Kieselgel 60 $F_{254}$) with chloroform/methanol = 50/1 for development, and the product was hydrogenated over 10% Pd/C at an atmospheric pressure of hydrogen for 1 h. The reaction mixture was filtered and the filtrate was evaporated in vacuo. The residue was purified by reverse phase MPLC (Merck, LiChroprep RP-18) with methanol/water = 5/1 for elution to give Compound 4 (5 mg).

m.p.: 193-201 °C
IR(KBr,$cm^{-1}$):
3328,2962,2872,1740,1647,1593,1548,1461,1446,1386, 1341,1311,1263,1230,1092,747

| High Resolution FAB-MS(m/e,$(C_{32}H_{40}ClN_5O_7+H)^+$): | |
|---|---|
| Calcd: | 642.2695 |
| Found: | 642.2661 |

$^{1}H$-NMR(300MHz,DMSO-$d_6$, δ ppm):
0.65(3H,d,J=5.8Hz),0.67(3H,d,J=5.8Hz),0.82(3H,t, J=7.1Hz),1.00-1.40(7H,m),1.57-1.80(2H,m),2.89 (1H,dd,J=3.2Hz,11.5Hz),3.10-3.20(1H,m),3.95(3H,s), 4.08-4.22(2H,m),4.62-4.72(1H,m),6.91(1H,dt,J=1.4Hz, 7.8Hz),7.14-7.32(4H,m),7.35(1H,dd,J=1.4Hz,7.8Hz), 7.54(1H,s),7.76(1H,d,J=7.8Hz),8.05(1H,d,J=7.8Hz), 8.08(1H,dd,J=1.4Hz,7.8Hz),8.11(1H,s),8.13-8.20 (1H,m),8.50(1H,d,J=8.8Hz)

Example 2

Synthesis of Compound 2

Compound 2 was prepared using Boc-Leu-DTrp(COOEt)-DNle-OBzl as a starting material in the same manner described in Example 1.

m.p.: 161-168 °C
IR(KBr,$cm^{-1}$):
3328,2962,2872,1740,1644,1593,1551,1461,1446,1206, 1092,747

| High Resolution FAB-MS(m/e,$(C_{33}H_{42}ClN_5O_7+H)^+$): | |
|---|---|
| Calcd: | 656.2852 |

(continued)

| High Resolution FAB-MS(m/e,($C_{33}H_{42}ClN_5O_7$+H)$^+$): | |
|---|---|
| Found: | 656.2851 |

$^1$H-NMR(300MHz,DMSO-$d_6$, δ ppm):
0.65(3H,d,J=6.4Hz),0.66(3H,d,J=6.4Hz),0.82(3H,t, J=7.2Hz),0.88-1.80(9H,m),1.38(3H,t,J=7.0Hz),2.90 (1H,dd,J=11.8Hz,14.4Hz),3.15(1H,dd,J=2.7Hz,14.4Hz), 4.10-4.25(2H,m),4.41(2H,q,J=7.0Hz),4.64-4.75(1H,m), 6.86(1H,s),6.91(1H,dt,J=1.6Hz,7.6Hz),7.12-7.40 (5H,m),7.56(1H,s),7.75(1H,d,J=7.1Hz),8.05(1H,d, J=7.1Hz),8.09(1H,d,J=10.1Hz),8.17(1H,d,J=7.9Hz), 8.47(1H,d,J=8.8Hz)

Preparative Example 2

(1) Preparation of H-DBal-DNle-O$^t$Bu

To a solution of Boc-DBal-OH (80 mg) in dichloromethane (10 ml) were added H-DNle-O$^t$Bu (67 mg), HOBT • $H_2O$ (41 mg) and EDCI • HCl (58 mg) successively at room temperature. After being stirred at room temperature for 6 h, the mixture was diluted with dichloromethane, washed with sat. aq $NaHCO_3$, 10% aq. citric acid, water and brine successively, dried over $MgSO_4$ and evaporated in vacuo. The residue was purified by silica gel flash chromatography (Merck, Kieselgel 60) with chloroform for elution to give an oily product (123 mg), which was dissolved in formic acid. The mixture was stirred at room temperature for 2 h. The mixture was diluted with ethyl acetate, washed with sat. aq. $NaHCO_3$ and brine, dried over $MgSO_4$ and evaporated in vacuo to give H-DBal-DNle-O$^t$Bu (97 mg). FAB-MS(m/e, ($C_{21}H_{30}N_2O_3S$+H)$^+$):391

(2) Preparation of (2-chlorophenyl)carbamoyl-Leu-OH

(2-Chlorophenyl)carbamoyl-Leu-OH was prepared using H-Leu-OBzl • TsOH as a starting material in the same manner described in Example 1. FAB-MS(m/e, ($C_{13}H_{17}ClN_2O_3$+H)$^+$):285

(3)

To a solution of H-DBal-DNle-O$^t$Bu (98 mg, prepared in (1)) in DMF (10 ml) were added (2-chlorophenyl)carbamoyl-Leu-OH (85 mg, prepared in (2)), HOBT • $H_2O$ (46 mg) and EDCI • HCl (62 mg) successively under ice cooling. After being stirred at room temperature for 11 h, the reaction mixture was concentrated under reduced pressure. The residue was dissolved in chloroform and the solution was washed with sat. aq. $NaHCO_3$, 10% aq.citric acid and brine successively, dried over $MgSO_4$ and evaporated to give a corresponding tripeptide derivative (148 mg). The tripeptide (102 mg) was dissolved in TFA (2 ml). The solution was stirred at room temperature for 2 h and evaporated in vacuo. The residue was purified by reprecipitation from methanol and water to give the Compound (81 mg).

m.p.: 182-183 °C
IR(KBr,$cm^{-1}$):
3304,3076,2962,1716,1644,1590,1551,1443,750,729

| High Resolution FAB-MS(m/e,($C_{30}H_{37}ClN_4O_5S$+H)$^+$): | |
|---|---|
| Calcd: | 601.2252 |
| Found: | 601.2255 |

$^1$H-NMR(300MHz,$CDCl_3$, δ ppm):
0.77-0.82(9H,m),0.88-1.65(9H,m),3.32(1H,dd,J=7.8Hz, 14.5Hz),3.55(1H,dd,J=5.5Hz,14.5Hz),4.06-4.15(2H,m), 4.72-4.80(1H,m),6.80(1H,brs),6.88-6.97(2H,m),7.10-7.42(6H,m),7.56(1H,brs),7.78-7.88(2H,m),7.94-8.00 (1H,m)

Example 3

Synthesis of Compound 3

(1) Preparation of H-DTrp(COOMe)-DNle-$O^tBu$ • HCl

To a suspension of H-DNle-$O^tBu$ • HCl (603 mg) in dichloromethane (10 ml) were added NMM (297 µl), Boc-DTrp-OH (903 mg), HOBT • $H_2O$ (413 mg) and EDCI • HCl (569 mg) successively under ice cooling. The mixture was stirred at the same temperature for 2 h and at room temperature for 1 h. The reaction mixture was diluted with ethyl acetate, washed with 10% aq. citric acid, sat. aq. $NaHCO_3$, water and brine successively, dried over $MgSO_4$ and evaporated in vacuo to give Boc-DTrp-DNle-$O^tBu$ (1.22 g). To a solution of the dipeptide derivative in dichloromethane (12 ml) were added methyl chloroformate (1 ml), pulverized NaOH (103 mg) and TBAHS (20 mg). After being stirred of the mixture at room temperature for 30 min, pulverized NaOH (103 mg) was added. This procedure was repeated twice more. The reaction mixture was diluted with dichloromethane, washed with sat. aq. $NaHCO_3$, 10% aq.citric acid, water and brine successively, dried over $MgSO_4$ and evaporated in vacuo. The residue was recrystallized from ethyl acetate and hexane to give Boc-DTrp(COOMe)-DNle-$O^tBu$ (1.15 g). The dipeptide derivative (1.03 g) was dissolved in TFA (10 ml) and the solution was stirred under ice cooling for 10 min. After evaporation of the reaction mixture, the residue was dissolved in ethyl acetate. The solution was washed with sat. aq $NaHCO_3$, water and brine successively, dried over $MgSO_4$ and concentrated under reduced pressure to give H-DTrp(COOMe)-DNle-$O^tBu$ as a free base. To a solution of the free base (750 mg) in methanol was added 3.78N - hydrogen chloride / 1,4-dioxane (0.47 ml) under ice cooling. The solution was evaporated in vacuo to give H-DTrp(COOMe)-DNle-$O^tBu$ • HCl (810 mg).

FAB-MS(m/e, $(C_{23}H_{33}N_3O_5+H)^+$):432

(2) Preparation of H-Leu-DTrp(COOMe)-DNle-$O^tBu$

To a solution of H-DTrp(COOMe)-DNle-$O^tBu$ • HCl (60 mg, prepared in (1)) in DMF (2 ml) were added Z-Leu-OH (45 mg), HOBT • $H_2O$ (33 mg) and EDCI • HCl (41 mg) under ice cooling. After being stirred at the same temperature for 1 h, the mixture was diluted with ethyl acetate, washed with 10% aq. citric acid, sat. aq $NaHCO_3$ and brine successively, dried over $MgSO_4$ and evaporated in vacuo. The residue was purified by silica gel flash chromatography (Merck, Kieselgel 60) with ethyl acetate/hexane = 1/2 for elution to give an oily product (72 mg), which was dissolved in methanol (3 ml). The solution was hydrogenated over 10% Pd/C at an atmospheric pressure of hydrogen for 1 h. After removal of the catalyst by filtration, the filtrate was evaporated in vacuo to give H-Leu-DTrp(COOMe)-DNle-$O^tBu$ (48 mg).

(3) Preparation of Compound 3

To a solution of H-Leu-DTrp(COOMe)-DNle-$O^tBu$ (15 mg, prepared in (2)) in chloroform (1 ml) was added 2,6-dichlorophenylisocyanate (5.6 mg) at 0-5 °C under argon atmosphere. The mixture was stirred at the same temperature for 1.5 h, and diluted with chloroform. The solution was washed with 1N-hydrochloric acid, sat. aq. $NaHCO_3$ and brine successively, dried over $MgSO_4$ and evaporated in vacuo. The residue was purified by preparative TLC (Merck, Kieselgel 60 $F_{254}$) with chloroform/methanol = 30/1 for development to give an oily product (6.7 mg), which was dissolved in TFA (1 ml). After being stirred at room temperature for 1 h, the mixture was concentrated under reduced pressure. The residue was triturated with water to give Compound 3 (4.3 mg).

m.p.: 211-212 °C
IR(KBr,$cm^{-1}$):
3310,2962,1740,1647,1545,1461,1386,1260,1092,765

| High Resolution FAB-MS(m/e,$(C_{32}H_{39}Cl_2N_5O_7+H)^+$): | |
|---|---|
| Calcd: | 676.2305 |

(continued)

| High Resolution FAB-MS(m/e,($C_{32}H_{39}Cl_2N_5O_7$+H)$^+$): | |
|---|---|
| Found: | 676.2297 |

$^1$H-NMR(300MHz,Acetone-$d_6$, δ ppm):
0.78(3H,d,J=6.1Hz),0.79(3H,d,J=6.1Hz),0.80(3H,t, J=8.4Hz),1.10-1.76(9H,m),3.08(1H,dd,J=9.6Hz, 15.1Hz),3.37(1H,dd,J=4.7Hz,15.1Hz),4.00(3H,s),4.14-4.31(2H,m),4.77-4.91(1H,m),6.32(1H,d,J=7.1Hz),7.23 (1H,t,J=7.8Hz),7.28(1H,t,J=7.7Hz),7.33(1H,t, J=7.7Hz),7.40(2H,d,J=7.8Hz),7.58(1H,s),7.60(1H,s), 7.66(1H,d,J=7.6Hz),7.70(1H,d,J=7.3Hz),7.70(1H,d, J=7.7Hz),8.14(1H,d,J=7.7Hz)

Each Compound 4-6 in the following Examples 4-6 was prepared using corresponding isocyanate in the same manner described in Example 3-(3). Example 4

<u>Compound 4</u>

m.p.: 208-209 °C
IR(KBr,cm$^{-1}$):
3352,2962,2872,1737,1647,1551,1461,1389,1260,1095, 750

| High Resolution FAB-MS(m/e,($C_{32}H_{40}FN_5O_7$+H)$^+$): | |
|---|---|
| Calcd: | 626.2990 |
| Found: | 626.2984 |

$^1$H-NMR(300MHz,Acetone-$d_6$, δ ppm):
0.78(3H,d,J=6.3Hz),0.80(3H,d,J=6.3Hz),0.86(3H,t, J=6.9Hz),1.20-1.50(7H,m),1.65-1.90(2H,m),3.12(1H, dd,J=9.6Hz,15.0Hz),3.38(1H,dd,J=4.7Hz,15.0Hz),4.01 (3H,s),4.19-4.29(1H,m),4.34-4.46(1H,m),4.79-4.90 (1H,m),6.46(1H,d,J=7.1Hz),6.88-6.99(1H,m),7.00-7.14 (2H,m),7.25(1H,t,J=7.8Hz),7.32(1H,t,J=7.8Hz),7.61 (1H,s),7.67(1H,d,J=8.0Hz),7.70(1H,d,J=7.8Hz),7.81 (1H,d,J=8.2Hz),7.91(1H,d,J=2.1Hz),8.14(1H,d, J=7.8Hz),8.17-8.27(1H,m)

Example 5

<u>Compound 5</u>

m.p.: 212-224 °C
IR(KBr,cm$^{-1}$):
3322,3088,2962,2872,1740,1647,1554,1461,1389,1326, 1260,1122,762

| High Resolution FAB-MS(m/e,($C_{33}H_{40}F_3N_5O_7$+H)$^+$): | |
|---|---|
| Calcd: | 676.2958 |

(continued)

| High Resolution FAB-MS(m/e,($C_{33}H_{40}F_3N_5O_7$+H)$^+$): | |
|---|---|
| Found: | 676.2957 |

$^1$H-NMR(300MHz,DMSO-$d_6$, δ ppm):

0.64(3H,d,J=6.1Hz),0.65(3H,d,J=6.1Hz),0.81(3H,t, J=7.1Hz),0.78-1.82(9H,m),2.88(1H,dd,J=11.2Hz, 14.7Hz),3.10-3.25(1H,m),3.95(3H,s),4.10-4.28(2H,m), 4.62-4.76(1H,m),7.10-7.24(2H,m),7.26(1H,t,J=7.6Hz), 7.33(1H,t,J=7.6Hz),7.54(1H,s),7.43-7.63(2H,m),7.76 (1H,d,J=7.6Hz),7.88(1H,d,J=7.9Hz),7.88(1H,s),8.05 (1H,d,J=7.6Hz),8.22(1H,d,J=7.9Hz),8.49(1H,d, J=8.4Hz)

Example 6

Compound 6

m.p.: 186-188 °C

IR(KBr,$cm^{-1}$):

3346,2962,1740,1653,1506,1458,1386,1344,1260,1092, 741

| High Resolution FAB-MS(m/e,($C_{32}H_{40}N_6O_9$+H)$^+$): | |
|---|---|
| Calcd: | 653.2935 |
| Found: | 653.2939 |

$^1$H-NMR(300MHz,Acetone-$d_6$, δ ppm):

0.79(3H,d,J=6.4Hz),0.81(3H,d,J=6.4Hz),0.83(3H,t, J=7.2Hz),1.14-1.50(7H,m),1.65-1.90(2H,m),3.11 (1H,dd,J=9.6Hz,14.8Hz),3.39(1H,dd,J=4.2Hz,14.8Hz), 4.01(3H,s),4.17-4.29(1H,m),4.37-4.49(1H,m),4.79-4.90(1H,m),7.12(1H,dt,J=1.4Hz,7.2Hz),7.25(1H,t, J=7.6Hz),7.31(1H,t,J=7.6Hz),7.20-7.37(1H,m),7.55-7.67(2H,m),7.60(1H,s),7.69(1H,d,J=7.6Hz),7.78(1H,d, J=9.1Hz),8.12(1H,d,J=7.6Hz),8.13(1H,d,J=8.2Hz),8.54 (1H,dd,J=1.4Hz,7.2Hz),9.56(1H,s)

Example 7

Synthesis of Compound 7

A solution of Compound 24 (30 mg, obtained in Example 6) in 95% ethanol (3 ml) was hydrogenated over 10% Pd/C (20 mg) at an atmospheric pressure of hydrogen for 1.5 h. After removal of the catalyst by filtration, the filtrate was evaporated in vacuo to give Compound 7 (26 mg).

m.p.: 188-190 °C

IR(KBr,$cm^{-1}$):

3370,2962,1740,1650,1551,1461,1386,1260,747

| High Resolution FAB-MS(m/e,($C_{32}H_{42}N_6O_7$+H)$^+$): | |
|---|---|
| Calcd: | 623.3193 |

(continued)

| High Resolution FAB-MS(m/e,($C_{32}H_{42}N_6O_7$+H)$^+$): | |
|---|---|
| Found: | 623.3157 |

$^1$H-NMR(300MHz,DMSO-$d_6$, δ ppm):

0.64(3H,d,J=6.5Hz),0.66(3H,d,J=6.5Hz),0.83(3H,t, J=7.0Hz),0.78-1.40(7H,m),1.56-1.78(2H,m),2.88 (1H,dd,J=11.8Hz,14.9Hz),3.15(1H,dd,J=2.8Hz,14.9Hz), 3.95(3H,s),4.09-4.21(2H,m),4.60-4.73(1H,m),6.28 (1H,d,J=8.1Hz),6.47(1H,dt,J=1.7Hz,7.7Hz),6.64 (1H,dd,J=1.7Hz,7.7Hz),6.74(1H,dt,J=1.7Hz,7.7Hz), 7.26(1H,t,J=7.6Hz),7.27(1H,dd,J=1.7Hz,7.7Hz),7.33 (1H,t,J=7.6Hz),7.54(1H,s),7.59(1H,s),7.76(1H,d, J=7.6Hz),8.05(1H,d,J=7.6Hz),8.23(1H,d,J=7.3Hz),8.46 (1H,d,J=8.6Hz)

Example 8

Synthesis of Compound 8

To a solution of Compound 25 (15 mg, obtained in Example 7) were added TEA (21 μl) and formic pivalic anhydride (15 μl) at 0-5 °C under argon atmosphere. The mixture was stirred at the same temperature for 5 h. Water was added to the reaction mixture. The mixture was acidified with 1N-hydrochloric acid and extracted with ethyl acetate. The ethyl acetate extract was dried over anhydrous sodium sulfate and evaporated in vacuo. The residue was purified by preparative TLC (3M, Empore Silica Sheet) with chloroform/methanol/acetic acid = 20/1/1 for development and the product was triturated with methanol and water to give Compound 8 (11 mg).

m.p.: 174-178 °C

IR(KBr,$cm^{-1}$):

3310,2962,1743,1665,1554,1461,1389,1260,1092,747

| High Resolution FAB-MS(m/e,($C_{33}H_{42}N_6O_8$+H)$^+$): | |
|---|---|
| Calcd: | 651.3143 |
| Found: | 651.3134 |

$^1$H-NMR(300MHz,DMSO-$d_6$, δ ppm):

0.67(3H,d,J=6.1Hz),0.74(3H,d,J=6.1Hz),0.70(3H,t, J=7.2Hz),1.00-2.00(9H,m),2.81-2.99(1H,m),3.07-3.20 (1H,m),3.87(3H,s),4.20-4.48(2H,m),4.60-4.90(1H,m), 6.87(1H,dt,J=1.4Hz,7.1Hz),7.00(1H,dt,J=1.4Hz, 7.1Hz),7.05-7.34(2H,m),7.16(1H,t,J=7.7Hz),7.28 (1H,t,J=7.7Hz),7.48(1H,s),7.45-7.59(2H,m),7.61 (1H,d,J=7.7Hz),7.78(1H,brs),8.03(1H,d,J=7.7Hz),8.47 (1H,brs),8.78(1H,d,J=8.3Hz),11.77(1H,brs)

Preparative Example 3

(1) Preparation of PhOCO-Leu-DTrp(COOMe)-DNle-O$^t$Bu

To a solution of H-Leu-DTrp(COOMe)-DNle-O$^t$Bu (320 mg, prepared in Example 3-(2)) in pyridine (2.3 ml) was added phenyl chloroformate at 0-5 °C under argon atmosphere. The mixture was stirred at the same temperature for 1.5 h and concentrated in vacuo. The residue was dissolved in ethyl acetate and the solution was washed with 10% aq. citric acid, sat. aq. $NaHCO_3$ and brine successively, dried over $MgSO_4$ and evaporated in vacuo. The residue was purified by silica gel flash chromatography (Merck, Kieselgel 60) with ethyl acetate/hexane = 2/1 for elution to give the product (337 mg).

FAB-MS(m/e, ($C_{36}H_{48}N_4O_8$+H)$^+$):665

Example 9

Synthesis of Compound 9

To a solution of PhOCO-Leu-DTrp(COOMe)-DNle-$O^tBu$ (33 mg, prepared in Preparative Example 3-(1)) in chloroform were added hexamethyleneimine (56 µl) and TEA (100 µl) at room temperature under argon atmosphere. The mixture was stirred at 55 °C for 2 h and cooled to room temperature. The mixture was diluted with ethyl acetate, washed with 1N-hydrochloric acid, sat. aq. $NaHCO_3$ and brine successively, dried over $MgSO_4$ and evaporated in vacuo. The residue was purified by preparative TLC (Merck, Kieselgel 60 $F_{254}$) with ethyl acetate/hexane = 1/1 for development to give an oily product (28 mg), which was dissolved in TFA (1 ml). After being stirred at room temperature for 1 h, the mixture was evaporated in vacuo. The residue was triturated with water to give Compound 9 (22 mg).

m.p.: 94-97 °C
IR(KBr,$cm^{-1}$):
3352,2938,1743,1635,1536,1461,1386,1260,1221,1092, 765,747

| High Resolution FAB-MS(m/e,$(C_{32}H_{47}N_5O_7+H)^+$): | |
|---|---|
| Calcd: | 614.3554 |
| Found: | 614.3532 |

$^1$H-NMR(300MHz,$CDCl_3$, δ ppm):
0.79(3H,d,J=6.1Hz),0.80(3H,d,J=6.1Hz),0.83(3H,t, J=6.8Hz),1.10-1.93(17H,m),3.10-3.45(6H,m),3.88-3.98 (1H,m),4.02(3H,s),4.35-4.45(1H,m),4.77-4.85(1H,m), 5.21(1H,brs),6.74(1H,d,J=8.0Hz),7.25(1H,t,J=7.6Hz), 7.34(1H,t,J=7.6Hz),7.49(1H,s),7.58(1H,d,J=7.6Hz), 7.66(1H,d,J=8.4Hz),8.16(1H,d,J=7.6Hz)

Each Compound 10-13 in the following Examples 10-13 was prepared using each corresponding amine as a starting material in the same manner described in Example 10.

Example 10

Compound 10

m.p.: 106-110 °C
IR(KBr,$cm^{-1}$):
3328,2962,1743,1635,1539,1461,1386,1257,1092,765, 747

| High Resolution FAB-MS(m/e,$(C_{30}H_{43}N_5O_7S+H)^+$): | |
|---|---|
| Calcd: | 618.2961 |
| Found: | 618.2987 |

$^1$H-NMR(300MHz,$CDCl_3$, δ ppm):
0.80(3H,d,J=5.5Hz),0.81(3H,d,J=5.5Hz),0.83(3H,t, J=7.1Hz),1.08-1.90(9H,m),2.49-2.64(4H,m),3.24(1H, dd,J=7.3Hz,15.1Hz),3.31(1H,dd,J=6.1Hz,15.1Hz),3.55-3.73(4H,m),3.88-3.98(1H,m),4.03(3H,s),4.27-4.37 (1H,m),4.70-4.80(1H,m),5.73(1H,brs),6.68(1H,d, J=8.1Hz),7.26(1H,t,J=7.8Hz),7.35(1H,t,J=7.8Hz), 7.49(1H,s),7.50(1H,d,J=6.6Hz),7.57(1H,d,J=7.8Hz), 8.16(1H,d,J=7.8Hz)

Example 11

Compound 11

m.p.: 104-109 °C
IR(KBr,$cm^{-1}$):
3322,2962,1743,1644,1536,1461,1386,1260,1092,765, 747

| High Resolution FAB-MS(m/e,$(C_{30}H_{43}N_5O_7+H)^+$): | |
|---|---|
| Calcd: | 586.3241 |
| Found: | 586.3264 |

$^1$H-NMR(300MHz,$CDCl_3$, δ ppm):
0.79(3H,d,J=6.0Hz),0.81(3H,d,J=6.0Hz),0.83(3H,t, J=7.1Hz),1.10-1.98(13H,m),3.13-3.40(6H,m),3.87-3.98 (1H,m),4.02(3H,s),4.39-4.48(1H,m),4.78-4.85(1H,m), 5.14(1H,d,J=5.9Hz),6.69(1H,d,J=8.0Hz),7.25(1H,t, J=7.4Hz),7.34(1H,t,J=7.4Hz),7.49(1H,s),7.59(1H,d, J=7.4Hz),7.74(1H,d,J=7.9Hz),8.16(1H,d,J=7.4Hz)

Example 12

Compound 12

m.p.: 96-101 °C
IR(KBr,$cm^{-1}$):
3322,2956,2866,1743,1638,1539,1461,1386,1260,1092, 765,747

| High Resolution FAB-MS(m/e,$(C_{31}H_{45}N_5O_7+H)^+$): | |
|---|---|
| Calcd: | 600.3397 |
| Found: | 600.3385 |

$^1$H-NMR(300MHz,$CDCl_3$, δ ppm):
0.77(3H,d,J=6.3Hz),0.79(3H,d,J=6.3Hz),0.83(3H,t, J=7.1Hz),1.10-1.92(15H,m),3.15-3.37(6H,m),3.86-3.98 (1H,m),4.02(3H,s),4.31-4.40(1H,m),4.73-4.80(1H,m), 5.52(1H,brs),6.80(1H,d,J=6.4Hz),7.25(1H,t,J=7.6Hz), 7.34(1H,t,J=7.6Hz),7.50(1H,s)7.58(1H,d,J=7.6Hz), 7.62(1H,d,J=7.2Hz),8.16(1H,d,J=7.6Hz)

Example 13

Compound 13

m.p.: 104-108 °C
IR(KBr,$cm^{-1}$):
3328,2962,1743,1647,1539,1461,1386,1260,1092,765, 747

| High Resolution FAB-MS(m/e,($C_{29}H_{41}N_5O_7S$+H)$^+$): | |
|---|---|
| Calcd: | 604.2805 |
| Found: | 604.2799 |

$^1$H-NMR(300MHz,$CDCl_3$, δ ppm):
0.75-0.86(9H,m),1.05-1.92(9H,m),2.90-3.00(2H,m), 3.28(2H,d,J=7.4Hz),3.39-3.48(1H,m),3.62-3.70(1H,m), 3.85-3.95(1H,m),4.03(3H,s),4.17(1H,d,J=9.1Hz),4.43 (1H,d,J=9.1Hz),4.48-4.56(1H,m),4.73-4.82(1H,m),5.93 (1H,brs),6.57(1H,d,J=8.5Hz),7.26(1H,t,J=7.8Hz),7.35 (1H,t,J=7.8Hz),7.49(1H,s),7.58(1H,d,J=7.8Hz),7.59 (1H,d,J=11.9Hz),8.16(1H,d,J=7.8Hz)

Example 14

Synthesis of Compound 14

(1) Preparation of PhOCO-Leu-OBzl

To a solution of H-Leu-OBzl • TsOH (1.01 g) in pyridine (9 ml) was added phenyl chloroformate (386 μl) at 0-5 °C under argon atmosphere. The mixture was stirred at the same temperature for 2 h and concentrated in vacuo. The residue was dissolved in ethyl acetate and the solution was washed with 1N-hydrochloric acid, water and brine successively, dried over $MgSO_4$ and evaporated in vacuo. The residue was purified by silica gel chromatography (Merck, Kieselgel 60) with ethyl acetate/hexane = 1/10 for elution to give the product (828 mg).

FAB-MS(m/e, ($C_{20}H_{23}NO_4$+H)$^+$):342

(2) Preparation of $^i$Pr$_2$NCO-Leu-OBzl

To a solution of PhOCO-Leu-OBzl (70 mg, prepared in (1)) in chloroform (2 ml) were added diisopropylamine (575 μl) and TEA (286 μl) at room temperature under argon atmosphere. The mixture was stirred at 55 °C for 17 h and cooled to room temperature. The mixture was diluted with ethyl acetate, washed with 1N-hydrochloric acid, sat. aq. $NaHCO_3$ and brine successively, dried over $MgSO_4$ and evaporated in vacuo. The residue was purified by preparative TLC (Merck, Kieselgel 60 $F_{254}$) with ethyl acetate/hexane = 1/4 for development to give the product (32 mg).

FAB-MS(m/e, ($C_{20}H_{32}N_2O_3$+H)$^+$):349

(3) Preparation of Compound 14

A mixture of $^i$Pr$_2$NCO-Leu-OBzl (32 mg, obtained in (2)) and 10% Pd/C (10 mg) in methanol (1 ml) was stirred at room temperature under an atmospheric pressure of hydrogen for 16 h. The catalyst was filtered off and the filtrate was concentrated under reduced pressure. The residue was dissolved in dichloromethane (1.5 ml). To the solution were added H-DTrp(COOMe)-DNle-O$^t$Bu • HCl (40 mg, prepared in Example 21-(1)), NMM (10 μl), HOBT • $H_2O$ (13 mg) and EDCI • HCl (18 mg) under ice cooling. After being stirred at room temperature for 1.5 h, the mixture was diluted with ethyl acetate, washed with sat. aq. $NaHCO_3$, 10% aq. citric acid, water and brine successively, dried over $MgSO_4$ and evaporated in vacuo. The residue was purified by preparative TLC (Merck, Kieselgel 60 $F_{254}$) with ethyl acetate/hexane = 1/1 for development to give the product (52 mg), which was dissolved in TFA (1 ml). After being stirred at room temperature for 1 h, the mixture was evaporated in vacuo. The residue was triturated with water to give Compound 14 (18 mg).

m.p.: 94-96 °C
IR(KBr,cm$^{-1}$):
3322,2962,2872,1743,1650,1524,1461,1386,1341,1311, 1260,1221,1149,1092,765,747

| High Resolution FAB-MS(m/e,($C_{32}H_{49}O_7N_5$+H)$^+$): | |
|---|---|
| Calcd: | 616,3718 |
| Found: | 616,3699 |

$^1$H-NMR(300MHz,DMSO-$d_6$, δ ppm):
0.66(3H,d,J=6.0Hz),0.67(3H,d,J=6.0Hz),0.85(3H,t, J=7.0Hz),1.08+1.10(12H,d×2,J=6.8Hz),1.10-1.40 (7H,m),1.15-1.30(2H,m),2.86(1H,dd,J=11.0Hz,14.8Hz), 3.18(1H,dd,J=3.5Hz,14.8Hz),3.70(2H,sept,J=6.8Hz), 3.95(3H,s),3.95-4.07(1H,m),4.10-4.20(1H,m),4.60-4.70(1H,m),5.56(1H,d,J=7.3Hz),7.25(1H,t,J=7.6Hz), 7.32(1H,t,J=7.6Hz),7.49(1H,s),7.70(1H,d,J=7.6Hz), 8.04(1H,d,J=7.6Hz),8.15(1H,d,J=8.1Hz),8.18(1H,d, J=9.3Hz)

Each Compound 15 and 16 in the following Examples 15 and 16 was prepared using corresponding amine in the same manner described in Example 14-(2) and (3).

Example 15

<u>Compound 15</u>

m.p.: 85-94 °C
IR(KBr,$cm^{-1}$):
3328,2962,2872,1743,1638,1533,1461,1383,1344,1311, 1260,1224,1200,1092,765,747

| High Resolution FAB-MS(m/e,($C_{32}H_{47}N_5O_7$+H)$^+$): | |
|---|---|
| Calcd: | 614.3554 |
| Found: | 614.3569 |

$^1$H-NMR(300MHz,DMSO-$d_6$, δ ppm):
0.66(3H,d,J=6.0Hz),0.68(3H,d,J=6.0Hz),0.86(3H,t, J=7.0Hz),1.06(3H,d,J=6.1Hz),1.09(3H,d,J=6.1Hz), 1.10-1.40(7H,m),1.40-1.60(2H,m),1.65-1.78(2H,m), 1.80-1.94(2H,m),2.86(1H,dd,J=4.0Hz,10.7Hz),3.12-3.30(1H,m),3.78(2H,sext,6.1Hz),3.96(3H,s),4.08-4.20(2H,m),4.50-4.68(1H,m),5.65(1H,d,J=6.8Hz), 7.27(1H,t,J=7.4Hz),7.32(1H,t,J=7.4Hz),7.49(1H,s), 7.68(1H,d,J=7.4Hz),8.05(1H,d,J=7.4Hz),8.20-8.31 (2H,m)

Example 16

<u>Compound 16</u>

m.p.: 100-110 °C
IR(KBr,$cm^{-1}$):
3376,2962,2866,1743,1632,1539,1461,1386,1341,1311, 1260,1188,1152,747

| High Resolution FAB-MS(m/e,$(C_{32}H_{47}N_5O_7+H)^+$): | |
|---|---|
| Calcd: | 614,3554 |
| Found: | 614,3540 |

$^1$H-NMR(300MHz,DMSO-$d_6$, δ ppm):
0.62-0.75(6H,m),0.86(3H,t,J=7.0Hz),0.95+0.97 (3H,d×2,J=6.8Hz),1.03-1.58(13H,m),1.65-1.78(2H,m), 2.77-2.91(1H,m),3.15-3.50(2H,m),3.63-3.77(1H,m), 3.86-4.00(1H,m),4.01(3H,s),4.04-4.30(2H,m),4.53-4.64(1H,m),6.15-6.25(1H,m),7.25(1H,t,J=7.3Hz), 7.32(1H,t,J=7.3Hz),7.47+7.48(1H,s×2),7.66+7.67 (1H,d×2,J=7.3Hz),8.05(1H,d,J=7.3Hz),8.15-8.28 (2H,m)

Example 17

Synthesis of Compound 17

(1) Preparation of (c-Pent)nPrNCO-Leu-OBzl

To a mixture of H-Leu-OBzl • TsOH (1.0 g) and CDI (469 mg) in chloroform (10 ml) was added TEA (0.43 ml) at room temperature and the mixture was stirred at the same temperature for 1 h. Cyclopentylpropylamine (0.47 ml) was added and the reaction mixture was stirred at room temperature for 6 h. The mixture was diluted with ethyl acetate, washed with 1N-hydrochloric acid and brine, dried over $MgSO_4$ and evaporated in vacuo. The residue was purified by silica gel chromatography (Merck, Kieselgel 60) with ethyl acetate/hexane = 3/7 for elution to give the product (850 mg). FAB-MS(m/e, $(C_{22}H_{34}NO_3+H)^+$):375

(2) Preparation of Compound 17

Compound 17 was prepared using (c-Pent)nPrNCO-Leu-OBzl (prepared in (1)) as a starting material in the same manner described in Example 14-(3).

m.p.: 68-73 °C
IR(KBr,$cm^{-1}$):
3382,2962,2872,1743,1632,1533,1461,1386,1344,1311, 1260,1227,1092,765,747
High Resolution FAB-MS(m/e,$(C_{34}H_{51}N_5O_7+H)^+$):642
$^1$H-NMR(300MHz,$CDCl_3$, δ ppm):
0.81(3H,d,J=5.8Hz),0.82(3H,d,J=6.7Hz),0.83(3H,t, J=6.7Hz),0.87(3H,t,J=7.3Hz),1.12-1.95(19H,m), 2.91(1H,dt,J=7.9Hz,15.8Hz),3.00(1H,dt,J=7.9Hz, 15.8Hz),3.27(1H,dd,J=4.5Hz,15.0Hz),3.33(1H,dd, J=6.0Hz,15.0Hz),3.84-3.98(1H,m),4.02(3H,s), 4.14(1H,quint,J=7.9Hz),4.34(1H,ddd,J=4.6Hz,6.4Hz, 8.2Hz),4.75(1H,d,J=6.4Hz),4.78-4.86(1H,m),6.60 (1H,d,J=7.9Hz),7.26(1H,t,J=7.6Hz),7.35(1H,t, J=7.6Hz),7.48(1H,s),7.59(1H,d,J=7.6Hz),7.61 (1H,d,J=8.2Hz),8.16(1H,d,J=7.6Hz)

Each Compound 18-20 in the following Examples 18-20 was prepared using each corresponding amine as a starting material in the same manner described in Example 17.

Example 18

Compound 18

m.p.: 98-107 °C
IR(KBr,$cm^{-1}$):
3388,2914,2860,1743,1653,1551,1461,1386,1260,1092, 765,747

| High Resolution FAB-MS(m/e,($C_{35}H_{45}N_5O_7$+H)$^+$): | |
|---|---|
| Calcd: | 648.3398 |
| Found: | 648.3346 |

$^1$H-NMR(300MHz,DMSO-$d_6$, δ ppm):

0.65(3H,d,J=5.6Hz),0.69(3H,d,J=5.6Hz),0.86(3H,t, J=7.0Hz),1.07-1.45(7H,m),1.65-1.83(2H,m),2.60-2.80 (2H,m),2.86(1H,dd,J=11.0Hz,14.4Hz,3.15-3.30(1H,m), 3.30-3.60(2H,m),3.94(3H,s),3.94-4.05(1H,m),4.06-4.20(1H,m),4.39(1H,d,J=16.4Hz),4.50(1H,d,J=16.4Hz), 4.52-4.66(1H,m),6.44(1H,d,J=7.1Hz),7.00-7.19(4H,m), 7.23(1H,t,J=7.5Hz),7.31(1H,t,J=7.5Hz),7.49(1H,s), 7.68(1H,d,J=7.5Hz),8.04(1H,d,J=7.5Hz),8.19(1H,d, J=7.6Hz),8.30(1H,d,J=8.7Hz)

Example 19

Compound 19

m.p.: 79-82 °C

IR(KBr,$cm^{-1}$):

3316,2692,2872,1743,1629,1533,1461,1386,1341,1311, 1260,1227,1092,765,747

| High Resolution FAB-MS(m/e,($C_{35}H_{53}N_5O_7$+H)$^+$): | |
|---|---|
| Calcd: | 656.4024 |
| Found: | 656.4029 |

$^1$H-NMR(300MHz,$CDCl_3$, δ ppm):

0.81(3H,d,J=6.3Hz),0.82(3H,d,J=6.3Hz),0.83(3H,t, J=6.3Hz),0.91(3H,t,J=7.4Hz),1.10-1.95(21H,m),2.93 (1H,dt,J=7.5Hz,15.0Hz),3.00(1H,dt,J=7.5Hz,15.0Hz), 3.27(1H,dd,J=6.6Hz,15.0Hz),3.31(1H,dd,J=6.0Hz, 15.0Hz),3.91(1H,q,J=6.7Hz),4.02(3H,s),4.14(1H, quint,J=8.2Hz),4.34(1H,ddd,J=5.4Hz,6.4Hz,8.7Hz), 4.72(1H,d,J=6.4Hz),4.82(1H,ddd,J=6.0Hz,6.6Hz, 7.6Hz),6.56(1H,d,J=7.6Hz),7.26(1H,t,J=7.6Hz), 7.35(1H,t,J=7.6Hz),7.48(1H,s),7.59(1H,d,J=6.7Hz), 7.64(1H,d,J=7.6Hz),8.16(1H,d,J=7.6Hz)

Example 20

Compound 20

m.p.: 95-110 °C

IR(KBr,$cm^{-1}$):

3334,2962,2872,1743,1662,1536,1461,1386,1344,1311, 1260,1149,1128,1092,765,747

| High Resolution FAB-MS(m/e,($C_{33}H_{49}N_5O_7$+H)$^+$): | |
|---|---|
| Calcd: | 628.3718 |
| Found: | 628.3699 |

$^1$H-NMR(300MHz,DMSO-$d_6$, δ ppm):
0.66(3H,d,J=6.0Hz),0.70(3H,d,J=6.0Hz),0.86(3H,t, J=7.1Hz),1.01(3H,d,J=7.2Hz),1.04(3H,d,J=7.2Hz), 1.05-1.55(11H,m),1.60-1.80(4H,m),2.84(1H,dd, J=11.5Hz,15.0Hz),3.15-3.25(1H,m),3.95(3H,s),4.01-4.18(4H,m),4.54-4.65(1H,m),6.04(1H,d,J=6.9Hz),7.25 (1H,t,J=7.5Hz),7.32(1H,t,J=7.5Hz),7.48(1H,s),7.67 (1H,d,J=7.5Hz),8.05(1H,d,J=7.5Hz),8.20(1H,d, J=7.6Hz),8.24(1H,d,J=8.8Hz)

Each Compound 21-24 in the following Examples 21-24 was prepared using each corresponding amino acid benzyl ester tosylate as a starting material in the same manner described in Example 20.

Example 21

Compound 21

m.p.: 103-107 °C
IR(KBr,cm$^{-1}$):
3376,2956,2872,1743,1656,1530,1461,1386,1341,1260, 1227,1137,1092,765,747
FAB-MS(m/e,($C_{34}H_{51}N_5O_7$+H)$^+$):642
$^1$H-NMR(300MHz,$CDCl_3$, δ ppm):
0.82(3H,t,J=7.3Hz),0.85(9H,s),1.14(6H,d,J=7.1Hz), 1.20-1.90(14H,m),3.26(1H,dd,J=5.5Hz,14.6Hz),3.35 (1H,dd,J=6.3Hz,14.6Hz),3.85-3.95(2H,m),4.02(3H,s), 4.09-4.21(1H,m),4.28(1H,ddd,J=5.3Hz,6.2Hz,8.3Hz), 4.76(1H,ddd,J=5.5Hz,6.3Hz,8.3Hz),4.93(1H,d, J=6.2Hz),6.48(1H,d,J=8.3Hz),7.27(1H,t,J=7.6Hz), 7.35(1H,t,J=7.6Hz),7.48(1H,s),7.58(1H,d,J=7.6Hz), 7.72(1H,d,J=7.3Hz),8.16(1H,d,J=7.6Hz)

Example 22

Compound 22

m.p.: 101-104.5 °C
IR(KBr,cm$^{-1}$):
3286,2944,2872,1743,1653,1560,1530,1461,1386,1341, 1311,1260,1143,1092,756

| High Resolution FAB-MS(m/e,($C_{36}H_{47}N_5O_7$+H)$^+$): | |
|---|---|
| Calcd: | 662.3554 |
| Found: | 662.3530 |

$^1$H-NMR(300MHz,$CDCl_3$,δ ppm):
0.70-1.92(12H,m),0.77(3H,t,J=7.4Hz),1.01(3H,d, J=7.1Hz),1.04(3H,d,J=7.1Hz),2.90(1H,dd,J=5.1Hz, 14.5Hz),2.94(1H,dd,6.5Hz,12.6Hz),3.06(1H,dd, J=8.1Hz,12.6Hz),3.32(1H,dd,J=5.1Hz,14.5Hz),3.74-3.93(1H,m),3.93-4.12(2H,m),4.03(3H,s),4.27(1H,ddd, J=4.6Hz,6.5Hz,8.1Hz),4.69(1H,dt,J=5.1Hz,7.6Hz),

5.05(1H,brs),6.32(1H,d,J=6.7Hz),7.06-7.45(9H,m), 7.60(1H,brs),8.14(1H,d,J=7.9Hz)

Example 23

Compound 23

m.p.: 102-110 °C

IR(KBr,$cm^{-1}$):

3406,2932,2860,1743,1665,1536,1461,1386,1341,1260, 1203,1149,1128,1092,765,747

| High Resolution FAB-MS(m/e,($C_{36}H_{53}N_5O_7$+H)$^+$): | |
|---|---|
| Calcd: | 668.4024 |
| Found: | 668.4004 |

$^1$H-NMR(300MHz,$CDCl_3$, δ ppm):

0.70-1.92(25H,m),0.81(3H,t,J=7.1Hz),1.14(3H,d, J=7.0Hz),1.15(3H,d,J=7.0Hz),3.23(1H,dd,J=5.3Hz, 15.1Hz),3.36(1H,dd,J=7.0Hz,15.1Hz),3.85-4.20(3H,m), 4.02(3H,s),4.22-4.38(1H,m),4.73-4.90(1H,m),5.02 (1H,brs),6.63(1H,d,J=6.2Hz),7.26(1H,t,J=7.3Hz), 7.35(1H,t,J=7.3Hz),7.49(1H,s),7.59(1H,d,J=7.3Hz), 7.65(1H,d,J=7.0Hz),8.16(1H,d,J=7.3Hz)

Example 24

Compound 24

m.p.: 96-104 °C

IR(KBr,$cm^{-1}$):

3328,2962,2872,1743,1656,1530,1461,1386,1341,1311, 1260,1227,1092,765,747

| High Resolution FAB-MS(m/e,($C_{32}H_{47}N_5O_7$+H)$^+$): | |
|---|---|
| Calcd: | 614.3554 |
| Found: | 614.3558 |

$^1$H-NMR(300MHz,$CDCl_3$, δ ppm):

0.79(3H,d,J=6.7Hz),0.82(3H,t,J=7.0Hz),0.90(3H,d, J=6.7Hz),1.00-1.40(6H,m),1.17(3H,d,J=7.0Hz),1.18 (3H,d,J=7.0Hz),1.42-2.10(7H,m),3.26(1H,dd,J=6.0Hz, 14.3Hz),3.31(1H,dd,J=6.0Hz,14.3Hz),3.73(1H,dd, J=7.2Hz,7.5Hz),3.94-4.05(1H,m),4.01(3H,s),4.06-4.22 (1H,m),4.34(1H,dt,J=5.8Hz,8.0Hz),4.86(1H,dt, J=6.0Hz,7.8Hz),5.09(1H,d,J=7.2Hz),6.74(1H,d, J=7.8Hz),7.26(1H,t,J=7.3Hz),7.34(1H,t,J=7.3Hz), 7.49(1H,s),7.50(1H,d,J=5.8Hz),7.61(1H,d,J=7.3Hz), 8.15(1H,d,J=7.3Hz)

Example 25

Synthesis of Compound 25

Compound 25 was prepared using 1-adamantanamine instead of diisopropylamine in the same manner

described in Example 14-(2).

m.p.: 129-137 °C
IR(KBr,$cm^{-1}$):
3316,2962,2872,1743,1635,1539,1461,1386,1260,1092, 747

| High Resolution FAB-MS(m/e,($C_{36}H_{51}N_5O_7$+H)$^+$): | |
|---|---|
| Calcd: | 666.3867 |
| Found: | 666.3879 |

$^1$H-NMR(300MHZ,DMSO-$d_6$, δ ppm):
0.61(3H,d,J=3.9Hz),0.63(3H,d,J=4.4Hz),0.87(3H,t, J=6.8Hz),0.90-2.00(24H,m),2.85(1H,dd,J=11.2Hz, 14.4Hz),3.11-3.25(1H,m),3.83-3.95(1H,m),3.96(3H,s), 4.10-4.20(1H,m),4.56-4.67(1H,m),5.63(1H,s),5.75 (1H,d,J=7.0Hz),7.25(1H,t,J=7.5Hz),7.32(1H,t, J=7.5Hz),7.49(1H,s),7.70(1H,d,J=7.5Hz),8.04 (1H,d,J=7.5Hz),8.18(1H,d,J=7.8Hz),8.31(1H,d, J=8.7Hz)

Example 26

Synthesis of Compound 26

Compound 26 was prepared using D-1-aminobutyric acid benzyl ester as a starting material in the same manner described in Preparative Example 1-(2) and (3).

m.p.: 216-218 °C
IR(KBr,$cm^{-1}$):
3448,2968,1743,1647,1599,1536,1461,1389,1341,1314, 1260,1170,1095,1050,765,747

| High Resolution FAB-MS(m/e,($C_{28}H_{40}N_4O_8$+H)$^+$): | |
|---|---|
| Calcd: | 561.2925 |
| Found: | 561.2911 |

$^1$H-NMR(300MHz,$CD_3OD$, δ ppm):
0.77(3H,d,J=6.4Hz),0.78(3H,d,J=6.4Hz),0.87(3H,t, J=7.4Hz),1.23-1.35(2H,m),1.37(9H,s),1.68-1.93 (3H,m),3.07(1H,dd,J=5.1Hz,10.7Hz),3.14-3.17(1H,m), 3.95-4.02(1H,m),4.00(3H,s),4.18(1H,dd,J=5.1Hz, 6.5Hz),4.65-4.75(1H,m),7.24(1H,t,J=7.7Hz),7.29 (1H,t,J=7.7Hz),7.51(1H,s),7.68(1H,d,J=7.7Hz), 8.10(1H,d,J=7.7Hz)

Example 27

Synthesis of Compound 27

Compound 27 was prepared using t-butylisocyanate in the same manner described in Example 3-(3).

m.p.: 116-121 °C
IR(KBr,$cm^{-1}$):
3400,2962,1743,1650,1557,1461,1389,1260,1092,765, 747

| High Resolution FAB-MS(m/e,($C_{30}H_{45}N_5O_7$+H)$^+$): | |
|---|---|
| Calcd: | 588.3397 |
| Found: | 588.3369 |

$^1$H-NMR(300MHz,DMSO-$d_6$, δ ppm):
0.61(6H,d,J=5.1Hz),0.87(3H,t,J=7.0Hz),1.15(9H,s), 0.82-1.89(9H,m),2.86(1H,dd,J=11.3Hz,14.4Hz),3.11-3.21(1H,m),3.96(3H,s),4.09-4.21(2H,m),4.58-4.70 (1H,m),5.73(1H,d,J=9.8Hz),5.73(1H,s),7.25(1H,t,J=7.5Hz),7.32(1H,t,J=7.5Hz),7.50(1H,s),7.72(1H,d, J=7.5Hz),8.04(1H,d,J=7.8Hz),8.21(1H,d,J=7.5Hz),8.31(1H,d,J=8.8Hz)

Example 28

<u>Synthesis of Compounds 28 and 29</u>

Compound 28 (ester form) and Compound 29 (deprotected form) were prepared using heptamethyleneimine instead of hexamethyleneimine in the same manner described in Example 9.

<u>Compound 28</u>

(colorless amorphous)

IR(KBr,$cm^{-1}$):
3304,2932,2866,1743,1632,1533,1461,1386,1257,1152, 1092,765,747

| High Resolution FAB-MS(m/e,($C_{37}H_{57}N_5O_7$+H)$^+$): | |
|---|---|
| Calcd: | 684.4337 |
| Found: | 684.4336 |

$^1$H-NMR(300MHz,$CDCl_3$, δ ppm):
0.82(3H,t,J=7.1Hz),0.84(3H,d,J=6.3Hz),0.85(3H,d, J=6.3Hz),1.06-1.80(19H,m),1.41(9H,s),3.19(1H,dd,J=5.9Hz,14.7Hz),3.33(1H,dd,J=6.1Hz,14.7Hz),3.13-3.50(4H,m),4.01(3H,s),3.96-4.10(1H,m),4.27-4.37 (1H,m),4.59(1H,d,J=6.8Hz),4.80-4.90(1H,m),6.53 (1H,d,J=8.8Hz),6.99(1H,d,J=7.7Hz),7.25(1H,t,J=7.3Hz),7.33(1H,t,J=7.3Hz),7.47(1H,s),7.62 (1H,d,J=7.3Hz),8.17(1H,d,J=7.3Hz)

<u>Compound 29</u>

m.p.: 99-104 °C
IR(KBr,$cm^{-1}$):
3316,2932,2872,1743,1632,1533,1461,1386,1260,1092, 765,747

| High Resolution FAB-MS(m/e,($C_{33}H_{49}N_5O_7$+H)$^+$): | |
|---|---|
| Calcd: | 628.3710 |
| Found: | 628.3693 |

$^1$H-NMR(300MHz,$CDCl_3$, δ ppm):
0.80(3H,d,J=5.9Hz),0.81(3H,d,J=5.9Hz),0.83(3H,t, J=6.0Hz),1.09-1.95(19H,m),3.05-3.46(6H,m),3.88-3.98 (1H,m),4.02(3H,s),4.31-4.41(1H,m),4.77-4.86(1H,m), 4.98(1H,d,J=7.0Hz),6.63(1H,d,J=7.4Hz),7.25(1H,t, J=7.5Hz),7.34(1H,t,J=7.5Hz),7.48(1H,s),7.58(1H,d, J=7.5Hz),7.68(1H,d,J=7.8Hz),8.16(1H,d,J=7.5Hz)

Example 29

Synthesis of Compound 30

Compound 30 was prepared using H-DNva-O$^t$Bu as a starting material in the same manner described in Example 20.

m.p.: 112-116 °C
IR(KBr,cm$^{-1}$):
3382,2962,2872,1743,1656,1620,1530,1461,1386,1260

| High Resolution FAB-MS(m/e,($C_{32}H_{47}N_5O_7$+H)$^+$): | |
|---|---|
| Calcd: | 614.3554 |
| Found: | 614.3539 |

$^1$H-NMR(300MHz,$CDCl_3$, δ ppm):
0.81(6H,d,J=5.4Hz),0.84(3H,t,J=7.3Hz),1.14(3H,d, J=6.5Hz),1.15(3H,d,J=6.6Hz),1.01-1.89(13H,m),3.29 (2H,d,J=5.7Hz),3.92-4.02(2H,m),4.01(3H,s),4.09-4.16 (1H,m),4.28-4.35(1H,m),4.83(1H,dt,J=6.3Hz,5.7Hz), 4.96(1H,d,J=4.8Hz),6.67-6.72(1H,brs),7.25(1H,t, J=7.8Hz),7.34(1H,t,J=7.8Hz),7.48(1H,s),7.58(1H,d, J=7.8Hz),7.65(1H,d,J=6.3Hz),8.16(1H,d,J=7.8Hz)

Example 30

Synthesis of Compound 31

Compound 31 was prepared using H-DNva-O$^t$Bu as a starting material in the same manner described in Example 21.

m.p.: 115-119 °C
IR(KBr,cm$^{-1}$):
3358,2962,2872,1746,1659,1620,1533,1461,1386,1260

| High Resolution FAB-MS(m/e,($C_{33}H_{49}N_5O_7$+H)$^+$): | |
|---|---|
| Calcd: | 628.3732 |

(continued)

| High Resolution FAB-MS(m/e,($C_{33}H_{49}N_5O_7$+H)$^+$): | |
|---|---|
| Found: | 628.3683 |

$^1$H-NMR(300MHz,$CDCl_3$, δ ppm):
0.84(9H,s),0.84(3H,t,J=7.2Hz),1.14(6H,d,J=7.1Hz), 1.01-1.89(12H,m),3.28(1H,dd,J=5.1Hz,14.1Hz),3.32 (1H,dd,J=6.0Hz,14.1Hz),3.87-4.01(2H,m),4.01(3H,s), 4.10-4.20(1H,m),4.32(1H,dt,J=8.1Hz,4.5Hz),4.76 (1H,ddd,J=5.1Hz,6.9Hz,8.1Hz),4.98(1H,d,J=5.4Hz), 6.57-6.60(1H,brs),7.24(1H,t,J=7.8Hz),7.34(1H,t, J=7.8Hz),7.48(1H,s),7.58(1H,d,J=7.8Hz),7.69(1H,d, J=8.1Hz),8.16(1H,d,J=7.8Hz)

Example 31

Synthesis of Compound 32

Compound 32 was prepared using H-Ile-OBzl • TsOH as a starting material in the same manner described in Example 20.

m.p.: 85-95 °C
IR(KBr,$cm^{-1}$):
3328,2968,2872,1743,1671,1539,1461,1386,1344,1311, 1260,1203,1149,1092,765,747

| High Resolution FAB-MS(m/e,($C_{33}H_{49}N_5O_7$+H)$^+$): | |
|---|---|
| Calcd: | 628.3710 |
| Found: | 628.3689 |

$^1$H-NMR(300MHz,DMSO-$d_6$, δ ppm):
0.42(3H,d,J=6.8Hz),0.64(3H,t,J=7.3Hz),0.81-0.98 (1H,m),0.86(3H,t,J=7.1Hz),1.04(3H,d,J=4.8Hz), 1.06(3H,d,J=4.8Hz),1.20-1.80(14H,m),2.84(1H,dd, J=11.4Hz,14.9Hz),3.24(1H,dd,J=2.6Hz,14.9Hz), 3.78(1H,t,J=7.8Hz),3.96(3H,s),4.07-4.20(3H,m), 4.58-4.68(1H,m),5.94(1H,d,J=7.8Hz),7.25(1H,t, J=7.6Hz),7.32(1H,t,J=7.6Hz),7.52(1H,s),7.66(1H,d, J=7.6Hz),8.04(1H,d,J=7.6Hz),8.27(1H,d,J=7.8Hz), 8.31(1H,d,J=8.7Hz)

Each Compound 33 and 34 in the following Example 32 and 33 was prepared using each corresponding amino acid benzyl ester tosylate as a starting material in the same manner described in Example 31.

Example 32

Compound 33

m.p.: 85-94 °C
IR(KBr,$cm^{-1}$):
3328,2968,2872,1743,1671,1530,1461,1386,1344,1311, 1260,1200,1149,1092,765,747

| High Resolution FAB-MS(m/e,($C_{33}H_{49}N_5O_7$+H)$^+$): | |
|---|---|
| Calcd: | 628.3710 |

(continued)

| High Resolution FAB-MS(m/e,($C_{33}H_{49}N_5O_7$+H)$^+$): | |
|---|---|
| Found: | 628.3698 |

$^1$H-NMR(300MHz,DMSO-$d_6$, δ ppm):
0.51(3H,d,J=6.5Hz),0.60(3H,t,J=7.1Hz),0.80-0.92 (1H,m),0.86(3H,t,J=7.0Hz),1.04(3H,d,J=6.2Hz), 1.06(3H,d,J=6.2Hz),1.20-1.80(14H,m),2.83(1H,dd, J=11.7Hz,14.7Hz),3.23(1H,dd,J=2.8Hz,14.7Hz), 3.88(1H,t,J=7.7Hz),3.96(3H,s),4.02-4.18(3H,m), 4.59-4.69(1H,m),5.78(1H,d,J=7.7Hz),7.25(1H,t, J=7.6Hz),7.32(1H,t,J=7.6Hz),7.53(1H,s),7.69(1H,d, J=7.6Hz),8.04(1H,d,J=7.6Hz),8.27(1H,d,J=7.52Hz), 8.32(1H,d,J=9.1Hz)

Example 33

Compound 34

m.p.: 108-111 °C
IR(KBr,$cm^{-1}$):
3340,2962,2872,1743,1650,1632,1518,1461,1386,1260, 1146,1092,765,747

| High Resolution FAB-MS(m/e,($C_{33}H_{49}N_5O_7$+H)$^+$): | |
|---|---|
| Calcd: | 628.3711 |
| Found: | 628.3705 |

$^1$H-NMR(400MHz,$CDCl_3$, δ ppm):
0.81(3H,t,J=6.8Hz),0.91(9H,s),1.19(6H,d,J=6.8Hz), 1.12-1.36(4H,m),1.43-1.87(8H,m),3.25(1H,dd,J=5.9Hz, 15.6Hz),3.34(1H,dd,J=6.8Hz,15.6Hz),3.87-4.00(1H,m), 4.01(3H,s),4.01-4.06(1H,m),4.13-4.18(1H,m),4.35-4.40(1H,m),4.81-4.87(1H,m),5.12(1H,d,J=7.8Hz),6.76-6.82(1H,brs),7.27(1H,t,J=7.8Hz),7.35(1H,t,J=7.8Hz), 7.39-7.42(1H,brs),7.50(1H,s),7.63(1H,d,J=7.8Hz), 8.17(1H,d,J=7.8Hz)

Example 34

Synthesis of Compound 35

Compound 35 was prepared using 2-(2-chlorophenyl)carbamoyloxy-4-methylpentanoic acid, which was prepared using 2-hydroxy-5-methylpentanoic acid instead of H-Leu-OBzl in the same manner described in Preparative Example 2-(2), as a starting material in the same manner described in Example 33-(3).

m.p.: 161-163 °C
IR(KBr,$cm^{-1}$):
3310,2962,1740,1659,1533,1461,1449,1386,1260,1212, 1089,747

| High Resolution FAB-MS(m/e,($C_{32}H_{39}ClN_4O_8$+H)$^+$): | |
|---|---|
| Calcd: | 643.2535 |

(continued)

| High Resolution FAB-MS(m/e,($C_{32}H_{39}ClN_4O_8$+H)$^+$): | |
|---|---|
| Found: | 643.2511 |

$^1$H-NMR(300MHz,$CDCl_3$, δ ppm):
0.82(3H,t,J=7.0Hz),0.91(6H,d,J=4.5Hz),1.13-1.31 (4H,m),1.56-1.84(5H,m),3.16(1H,dd,J=14.5Hz,7.2Hz), 3.29(1H,dd,J=14.5Hz,6.7Hz),3.96(3H,s),4.41-4.48 (1H,m),4.78-4.86(1H,m),5.04-5.09(1H,m),6.60(1H,d, J=7.6Hz),6.78(1H,d,J=7.7Hz),7.02(1H,t,J=7.7Hz), 7.18-7.30(4H,m),7.35(1H,d,J=7.7Hz),7.48(1H,s), 7.62(1H,d,J=6.3Hz),7.99(1H,d,J=8.0Hz),8.08(1H,d, J=7.7Hz)

Example 35

<u>Synthesis of Compound 36 and 37</u>

(1) Preparation of Boc-DTrp(COOMe)φ(CS-NH)-DNle-O$^t$Bu

Boc-DTrp(COOMe)-DNle-O$^t$Bu (300 mg, prepared in Example 3-(1)) and Lawesson's Reagent (140 mg) were suspended in toluene. The mixture was stirred at 100 °C under argon atmosphere for 5 h and concentrated under reduced pressure. The residue was purified by silica gel chromatography (Merck, Kieselgel 60) with chloroform/diethyl ether = 99/1 for elution to give the product (172 mg).

FAB-MS(m/e, ($C_{28}H_{41}N_3O_6S$+H)$^+$):547

(2) Preparation of Compound 36

A solution of Boc-DTrp(COOMe)φ(CS-NH)-DNle-O$^t$Bu (100 mg, obtained in (1)) in formic acid (3 ml) was stirred at room temperature for 2 h and concentrated under reduced pressure. The residue was dissolved in DMF (2 ml), and NMM (100 μl) was added. To the mixture were added 2,6-dimethylpiperidinocarbonyl-γMeLeu-OH (52 mg), HOBT • $H_2O$ (34 mg) and EDCI • HCl (42 mg) under ice cooling. The mixture was stirred at the same temperature for 30 mim and at room temperature for 14 h. The reaction mixture was diluted with ethyl acetate, washed with sat. aq $NaHCO_3$, 1N-hydrochloric acid and water successively, dried over $MgSO_4$ and evaporated in vacuo. The residue was purified by preparative TLC (Merck, Kieselgel 60 $F_{254}$) with hexane/ethyl acetate = 3/2 for development to give Compound 36 (28 mg).

m.p.: 75-78 °C
IR(KBr,$cm^{-1}$):
3256,2956,2872,1743,1626,1518,1461,1374,1341,1311, 1257,1227,1158,1089,1041,765,747

| High Resolution FAB-MS(m/e,($C_{38}H_{59}N_5O_6S$+H)$^+$): | |
|---|---|
| Calcd: | 714.4264 |
| Found: | 714.4252 |

$^1$H-NMR(300MHz,$CDCl_3$, δ ppm):
0.82(3H,t,J=7.2Hz),0.90(9H,s),0.95-1.92(14H,m), 1.19(3H,d,J=7.1Hz),1.20(3H,d,J=7.1Hz),1.37(9H,s), 3.29(1H,dd,J=7.2Hz,14.4Hz),3.47(1H,dd,J=5.5Hz, 14.4Hz),4.00(3H,s),4.05-4.13(1H,m),4.11-4.22(1H,m), 4.19-4.26(1H,m),4.65(1H,d,J=7.4Hz),4.72-4.79(1H,m), 4.97(1H,ddd,J=5.5Hz,7.2Hz,8.0Hz),7.06(1H,d, J=8.0Hz),7.26(1H,t,J=7.4Hz),7.33(1H,t,J=7.4Hz), 7.49(1H,s),7.72(1H,d,J=7.4Hz),8.15(2H,d,J=7.4Hz)

(3) Preparation of Compound 37

Compound 36 (13 mg, prepared in (2)) was dissolved in formic acid (1 ml) under ice cooling, and the mixture was stirred at the same temperature for 1.5 h and at room temperature for 6 h. The mixture was diluted with ethyl acetate, washed with brine and water, dried over $MgSO_4$ and evaporated in vacuo. The residue was purified by silica gel chromatography (Wakogel C-200) with chloroform/methanol = 97/3 for elution to give Compound 37 (7.3 mg).

m.p.: 74-82 °C
IR(KBr,$cm^{-1}$):
3310,2956,2866,1743,1620,1572,1524,1461,1386,1341, 1260,1230,1128,1092,765,747

| High Resolution FAB-MS(m/e,$(C_{34}H_{51}N_5O_6S+H)^+$): | |
|---|---|
| Calcd: | 658.3638 |
| Found: | 658.3658 |

$^1$H-NMR(300MHz,$CDCl_3$, δ ppm):
0.78(9H,s),0.86(3H,t,J=6.8Hz),0.92-2.05(14H,m), 1.14(6H,d,J=7.1Hz),3.33(1H,dd,J=7.9Hz,14.7Hz), 3.54(1H,dd,J=5.3Hz,14.7Hz),3.92-4.06(2H,m),3.99 (3H,s),4.09-4.16(1H,m),4.94-5.07(2H,m),5.17(1H,ddd, J=5.3Hz,6.3Hz,7.9Hz),6.99(1H,d,J=6.3Hz),7.22(1H,t, J=7.8Hz),7.31(1H,t,J=7.8Hz),7.51(1H,s),7.64(1H,d, J=7.8Hz),8.11(1H,d,J=7.8Hz),8.89(1H,d,J=7.5Hz)

Example 36

Synthesis of Compound 38

(1) Preparation of 2-hydroxy-3-(3-indolyl)propionyl-DNle-$O^tBu$

To a solution of DL-3-indolelactic acid (103 mg), H-DNle-$O^tBu$ • HCl (100 mg) and NMM (58 μl) in dichloromethane (5.0 ml) were added HOBT • $H_2O$ (81 mg) and EDCI • HCl (102 mg) under ice cooling. After being stirred at room temperature over night, the mixture was diluted with dichloromethane (20 ml), washed with sat. aq $NaHCO_3$, 10% aq. citric acid and brine successively, dried over $MgSO_4$ and evaporated in vacuo. The residue was purified by MPLC (Merck, LiChroprep Si 60) with hexane/ethyl acetate = 3/2 for elution to give the product (80 mg).

FAB-MS(m/e, $(C_{21}H_{30}N_2O_4+H)^+$):375

(2) Preparation of 2,6-dimethylpiperidinocarbonyl-γMeLeu-ϕ(COO)-DTrp-DNle-$O^tBu$

To a solution of 2-hydroxy-3-(3-indolyl)propionyl-DNle-$O^tBu$ (62 mg, prepared in (1)) and 2,6-dimethylpiperidinocarbonyl- γMeLeu-OH (72 mg) in THF (1.5 ml) were added EDCI • HCl (48 mg) and DMAP (10 mg) under ice cooling. The mixture was stirred at room temperature for 3 d, and concentrated under reduced pressure. To the residue was added water (20 ml), and the mixture was extracted with ethyl acetate (10 ml × 3). The combined organic layers were washed with 1N-hydrochloric acid (10 ml), sat. aq. $NaHCO_3$ and brine successively, dried over $MgSO_4$ and evaporated in vacuo. The residue was purified by MPLC (Merck, LiChroprep Si 60) with hexane/ethyl acetate = 2/1 for elution to give the product (91 mg).

FAB-MS(m/e, $(C_{36}H_{56}N_4O_6+H)^+$):641

(3) Preparation of 2,6-dimethylpiperidinocarbonyl-γMeLeu-φ(COO)-DTrp(COOMe)-DNle-$O^tBu$

To a solution of 2,6-dimethylpiperidinocarbonyl-γMeLeu-φ(COO)-DTrp-DNle-$O^tBu$ (76 mg, prepared in (2)) in dichloromethane were added methyl chloroformate (14 μl), pulverized NaOH (12 mg) and TBAHS (1 mg) under ice cooling. After being stirred at room temperature for 6 h, the mixture was diluted with dichloromethane (30 ml). The solution was washed with water (20 ml), dried over $MgSO_4$ and evaporated in vacuo. The residue was purified by preparative TLC (Merck, Kieselgel 60 $F_{254}$) with hexane/ethyl acetate = 2/1 for development to give the compound (41 mg).

FAB-MS(m/e, $(C_{38}H_{58}N_4O_8+H)^+$):699

(4) Preparation of Compound 38

A solution of 2,6-dimethylpiperidinocarbonyl-γMeLeu-φ(COO)-DTrp(COOMe)-DNle-$O^tBu$ (36 mg, prepared in (3)) in TFA (3 ml) was stirred at room temperature for 1 h, and concentrated under reduced pressure. The residue was triturated with water to give Compound 38 (32 mg).

m.p.: 74-79 °C
IR(KBr,$cm^{-1}$):
3406,2962,1746,1671,1629,1539,1461,1386,1260,1200, 1164,1092,765,747
FAB-MS(m/e,$(C_{34}H_{50}N_4O_8+H)^+$):643
$^1$H-NMR(300MHz,$CDCl_3$, δ ppm):
0.83(3H,t,J=7.2Hz),0.89(9H,s),1.00-1.90(14H,m), 1.17(3H,d,J=7.5Hz),1.20(3H,d,J=7.5Hz),3.31(1H,dd, J=7.5Hz,14.9Hz),3.40(1H,dd,J=3.8Hz,14.9Hz),3.70-4.10(2H,m),4.02(3H,s),4.07-4.19(1H,m),4.30-4.40 (1H,m),5.48(1H,dd,J=3.8Hz,7.5Hz),7.25(1H,t, J=7.5Hz),7.34(1H,t,J=7.5Hz),7.48(1H,s),7.59(1H,d, J=7.5Hz),7.20-8.00(2H,brs),8.16(1H,d,J=7.5Hz)

Example 37

<u>Synthesis of Compound 39</u>

Compound 39 was prepared in the same manner described in Example 30.

$^1$H-NMR(300MHz,$CDCl_3$, δ ppm):
0.82(3H,t,J=7.2Hz),0.89(9H,s),1.15(3H,d,J=7.1Hz), 1.05-1.75(14H,m),1.16(3H,d,J=7.1Hz),1.41(9H,s), 3.17(1H,dd,J=6.2Hz,15.0Hz),3.37(1H,dd,J=5.9Hz, 15.0Hz),3.93-4.10(2H,m),4.00(3H,s),4.10-4.23(1H,m), 4.24-4.36(1H,m),4.65(1H,d,J=7.1Hz),4.81(1H,ddd, J=5.9Hz,6.2Hz,8.5Hz),6.48(1H,d,J=8.5Hz),7.03(1H,d, J=7.6Hz),7.24(1H,t,J=7.6Hz),7.33(1H,t,J=7.6Hz), 7.47(1H,s),7.62(1H,d,J=7.6Hz),8.16(1H,d,J=7.8Hz)

Example 38

<u>Synthesis of Compound 40</u>

(1) Preparation of N-(2-thienylcarbamoyl)- γMeLeu-OBzl

To a solution of 2-thiophenecarboxylic acid (30 mg) and diphenylphosphoryl azide (50.5 µl) in toluene (10 ml) was added TEA (32.6 µl) under ice cooling. The mixture was stirred at the same temperature for 1 h, at room temperature for 10 h and refluxed for 6 h . The mixture was cooled to room temperature. To the mixture was added a solution of γMeLeu-OBzl • TsOH (61 mg) and TEA (21 µl) in DMF (1 ml). After being stirred at room temperature for 15 h, the reaction mixture was diluted with ethyl acetate, washed with sat. aq. $NaHCO_3$, 1N-hydrochloric acid and brine successively, dried over $MgSO_4$ and evaporated in vacuo. The residue was purified by preparative TLC (Merck, Kieselgel 60 $F_{254}$) with hexane/ethyl acetate = 4/1 for development to give the product.

FAB-MS(m/e,$(C_{19}H_{24}N_2O_3S+H)^+$):361

(2) Preparation of Compound 40

N-(2-Thienylcarbamoyl)- γMeLeu-OBzl (27.0 mg, prepared in (1)) was hydrolyzed with aqueous sodium hydroxide in methanol to afford N-(2-thienylcarbamoyl)- γMeLeu-OH (22.0 mg), which was condensed with H-DTrp(COOMe)-DNle-$O^tBu$ • HCl (35.0 mg, prepared in Example 21-(1)) in a similar manner to that of Example 33-(3) to afford Compound 66 (5.4 mg).

| High Resolution FAB-MS(m/e,($C_{31}H_{41}N_5O_7S$+H)$^+$): | |
|---|---|
| Calcd: | 628.2805 |
| Found: | 628.2822 |

Each compound 41-44 in the following Examples 39-42 was prepared using each corresponding amine and amino acid benzyl ester tosylate in the same manner described in Example 17.

Example 39

<u>Compound 41</u>

m.p.: 98-102 °C
IR(KBr,$cm^{-1}$):
3316,2962,2872,1743,1632,1521,1461,1386,1260,1092, 765,747

| High Resolution FAB-MS(m/e,($C_{31}H_{45}N_5O_7$+H)$^+$): | |
|---|---|
| Calcd: | 600.3397 |
| Found: | 600.3371 |

$^1$H-NMR(300MHz,$CDCl_3$, δ ppm):
0.50(3H,d,J=6.7Hz),0.57(3H,d,J=6.7Hz),0.86(3H,t, J=6.8Hz),1.10(6H,d,J=5.6Hz),0.92-2.10(11H,m), 2.84(1H,dd,J=11.2Hz,14.4Hz),3.21(1H,dd,3.1Hz, 14.4Hz),3.67-3.90(3H,m),3.96(3H,s),4.08-4.20(1H,m), 4.57-4.72(1H,m),5.47(1H,d,J=7.8Hz),7.25(1H,t, J=7.6Hz),7.32(1H,t,J=7.6Hz),7.52(1H,s),7.69(1H,d, J=7.6Hz),8.03(1H,d,J=7.6Hz),8.31(1H,d,J=8.1Hz), 8.35(1H,d,J=9.0Hz)

Example 40

<u>Compound 42</u>

IR(KBr,$cm^{-1}$):
2954,2859,1737,1616,1525,1442,1340,1309,1226,1199, 1126,765,748

| High Resolution FAB-MS(m/e,($C_{33}H_{47}N_5O_7$+H)$^+$): | |
|---|---|
| Calcd: | 626.3554 |
| Found: | 626.3572 |

$^1$H-NMR(300MHz,DMSO-$d_6$, δ ppm):

-0.80--0.95(2H,m),0.01-0.39(3H,m),0.86(3H,t, J=7.0Hz),1.03(3H,d,J=7.4Hz),1.05(3H,d,J=7.4Hz), 0.98-1.82(14H,m),2.85(1H,dd,J=10.9Hz,14.7Hz), 3.22(1H,dd,3.1Hz,14.7Hz),3.95(3H,s),3.90-4.20 (4H,m),4.53-4.65(1H,m),6.05(1H,d,J=6.8Hz),7.24 (1H,t,J=7.4Hz),7.31(1H,t,J=7.4Hz),7.49(1H,s),7.68(1H,d,J=7.4Hz),8.02(1H,d,J=7.4Hz),8.24(1H,d, J=6.9Hz),8.25(1H,d,J=8.8Hz)

Example 41

Compound 43

m.p.: 103-113 °C

IR(KBr,$cm^{-1}$):

2958,2937,1737,1654,1618,1527,1477,1444,1382,1340, 1309,1259,1226,1201,1145,1126,765

| High Resolution FAB-MS(m/e,$(C_{32}H_{45}N_5O_7+H)^+$): | |
|---|---|
| Calcd: | 612.3397 |
| Found: | 612.3384 |

$^1H$-NMR(300MHz,DMSO-$d_6$, δ ppm):

0.02-0.39(4H,m),0.79-0.97(1H,m),0.84(3H,t,J=7.0Hz), 1.04(3H,d,J=6.7Hz),1.06(3H,d,J=6.7Hz),1.12-1.78 (12H,m),2.85(1H,dd,J=10.9Hz,14.7Hz),3.21-3.37 (2H,m),3.96(3H,s),4.02-4.21(3H,m),4.50-4.62(1H,m),6.27(1H,d,J=5.6Hz),7.26(1H,t,J=7.4Hz),7.33(1H,t, J=7.4Hz),7.52(1H,s),7.65(1H,d,J=7.4Hz),8.05(1H,d, J=7.4Hz),8.20(1H,d,J=6.8Hz),8.21(1H,d,J=9.3Hz)

Example 42

Compound 44

IR(KBr,$cm^{-1}$):

2954,2861,1737,1650,1531,1444,1413,1342,1299,1224, 1091,765,746

| High Resolution FAB-MS(m/e,$(C_{33}H_{47}N_5O_7+H)^+$): | |
|---|---|
| Calcd: | 626.3554 |
| Found: | 626.3538 |

$^1H$-NMR(300MHz,DMSO-$d_6$, δ ppm):

0.76-0.96(3H,m),0.96-2.30(23H,m),2.73-2.90(1H,m), 3.00-3.61(5H,m),3.61-3.75(1H,m),3.96(3H,s),4.03-4.20(1H,m),4.49-4.63(1H,m),5.97(1H,d,J=4.9Hz),7.16-7.38(2H,m),7.53(1H,s),7.65(1H,d,J=7.2Hz),8.05(1H,d, J=7.2Hz),8.24(1H,d,J=6.5Hz),8.32(1H,d,J=7.7Hz)

Example 43

Synthesis of Compound 45 and 46

(1) Preparation of 2,6-dimethylpiperidinocarbonyl-γ MeLeu-DTrp(OH)-DNle-O$^t$Bu

To a solution of 2,6-dimethylpiperidinocarbonyl-γMeLeu-DTrp-DNle-O$^t$Bu (128 mg) in acetic acid (1.0 ml) was added sodium cyanoborohydride (126 mg). The mixture was stirred at room temperature for 17 h, and water (10 ml) was added to the mixture. The mixture was extracted with ethyl acetate (10 ml × 3 ). The combined organic layers were washed with sat. aq. $NaHCO_3$ (3 ml), dried over $MgSO_4$ and evaporated in vacuo. The residue was purified by MPLC (Merck, LiChroprep Si 60) with hexane/ethyl acetate = 1/1 for elution to give a colorless powder (58.2 mg). To a solution of the product (32.1 mg) and sodium tungstate dihydrate (3.3 mg) in methanol (1.0 ml) and water (0.3 ml) was added 30% hydrogen peroxide (51 μl). After being stirred at room temperature for 1 h, the mixture was evaporated in vacuo. The residue was purified by silica gel chromatography (Wakogel, C-200) with hexane/ethyl acetate = 1/1 for elution to give the product (13.7 mg). FAB-MS(m/e, $(C_{36}H_{57}N_5O_6+H)^+$):656

(2) Preparation of Compound 45

A solution of 2,6-dimethylpiperidinocarbonyl-γMeLeu-DTrp(OH)-DNle-O$^t$Bu (11 mg, prepared in (1)) in formic acid (0.4 ml) was stirred at room temperature for 13 h. The mixture was concentrated under reduced pressure, and the residue was purified by preparative TLC (3M, Empore Silica Sheet) with chloroform/methanol/acetic acid = 20/1/1 for development to give the product (3.4 mg).

m.p.: 165 °C(dec.)

| High Resolution FAB-MS(m/e,$(C_{32}H_{49}N_5O_6+H)^+$): | |
|---|---|
| Calcd: | 600.3761 |
| Found: | 600.3774 |

$^1$H-NMR(300MHz,DMSO-$d_6$, δ ppm):
0.80(9H,s),1.05(6H,d,J=6.8Hz),0.80-1.80(17H,m), 2.84(1H,dd,J=9.7Hz,14.4Hz),3.10-3.20(1H,m),4.00-4.25(4H,m),4.35-4.45(1H,m),6.10(1H,d,J=7.1Hz), 6.95(1H,t,J=7.5Hz),7.09(1H,t,J=7.5Hz),7.17(1H,s), 7.28(1H,d,J=7.5Hz),7.57(1H,d,J=7.5Hz),8.00-8.20 (2H,m)

(3) Preparation of Compound 46

A solution of 2,6-dimethylpiperidinocarbonyl-γMeLeu-DTrp(OH)-DNle-O$^t$Bu (11 mg, prepared in (1)) in methanol (3 ml) was treated with etherial diazomethane. The mixture was evaporated in vacuo, and the residue was purified by silica gel chromatography (Wakogel, C-200) with hexane/ethyl acetate = 3/2 for elution to give a pale yellow powder (8.2 mg). The product (7.9 mg) was dissolved in formic acid (0.4 ml) and the mixture was stirred at room temperature for 14 h. The reaction mixture was evaporated in vacuo and the residue was purified by preparative TLC (3M, Empore Silica Sheet) with chloroform/methanol = 5/1 for development to give the product (4.1 mg).

m.p.: 115-120 °C

| High Resolution FAB-MS(m/e,$(C_{33}H_{51}N_5O_6+H)^+$): | |
|---|---|
| Calcd: | 614.3917 |
| Found: | 614.3881 |

$^1$H-NMR(300MHz,DMSO-$d_6$, δ ppm):
0.79(9H,s),1.05(6H,d,J=6.6Hz),0.80-1.80(17H,m), 2.88(1H,dd,J=9.1Hz,14.6Hz),3.13(1H,dd,J=4.2Hz, 14.6Hz),3.85-3.95(1H,m),4.10-4.25(3H,m),4.40-4.50 (1H,m),3.98(3H,s),6.11(1H,d,J=7.9Hz),7.01(1H,t, J=7.5Hz),7.15(1H,t,J=7.5Hz),7.29(1H,s),7.35(1H,d, J=7.5Hz),7.59(1H,d,J=7.5Hz),7.80-7.90(1H,m),7.90-8.05(1H,m)

Example 44

Synthesis of Compound 47

To a solution of Compound 21 (27 mg, prepared in Example 21) in methanol (0.5 ml) was added a solution of sodium bicarbonate (3.5 mg) in water (0.1 ml). The mixture was evaporated in vacuo and the residue was dissolved in DMF (0.5 ml). Iodomethane (0.2 ml) was added to the mixture, and the mixture was stirred at room temperature for 20 h. The mixture was evaporated in vacuo and the residue was purified by preparative TLC (Merck, Kieselgel 60 $F_{254}$) with hexane/ ethyl acetate = 1/1 for development to give the product (11 mg).

m.p.: 80-88 °C
IR(KBr,$cm^{-1}$):
3412,2956,1746,1656,1623,1560,1521,1461,1386,1341, 1260,1092

| High Resolution FAB-MS(m/e,($C_{35}H_{53}N_5O_7$+H)$^+$): | |
|---|---|
| Calcd: | 656.4023 |
| Found: | 656.3996 |

$^1$H-NMR(300MHz,$CDCl_3$, δ ppm):
0.82(3H,t,J=7.2Hz),0.89(9H,s),1.05-1.85(14H,m), 1.15(3H,d,J=7.1Hz),1.16(3H,d,J=6.8Hz),3.17(1H,dd, J=5.7Hz,15.0Hz),3.39(1H,dd,J=6.2Hz,15.0Hz),3.65 (3H,s),3.90-4.06(2H,m),4.06-4.20(1H,m),4.36-4.47 (1H,m),4.64(1H,d,J=6.8Hz),4.78-4.90(1H,m),6.38 (1H,d,J=9.2Hz),7.27(1H,brs),7.25(1H,t,J=7.3Hz), 7.34(1H,t,J=7.3Hz),7.47(1H,s),7.61(1H,d,J=7.3Hz), 8.17(1H,d,J=7.3Hz)

Example 45

Synthesis of Compound 48

Compound 48 was prepared using iodoethane instead of iodomethane in the same manner described in Example 44.

m.p.: 80-85 °C
IR(KBr,$cm^{-1}$):
3412,2950,1746,1653,1626,1533,1461,1386,1257,1194, 1089,465

| High Resolution FAB-MS(m/e,($C_{36}H_{55}N_5O_7$+H)$^+$): | |
|---|---|
| Calcd: | 670.4180 |
| Found: | 670.4161 |

$^1$H-NMR(300MHz,$CDCl_3$, δ ppm):
0.82(3H,t,J=7.2Hz),0.89(9H,s),1.05-1.85(14H,m), 1.15(3H,d,J=7.1Hz),1.16(3H,d,J=7.1Hz),1.22(3H,t, J=7.3Hz),3.17(1H,dd,J=5.8Hz,14.7Hz),3.38(1H,dd, J=6.1Hz,14.7Hz),3.90-4.05(2H,m),4.00(3H,s),

4.09(2H,dq,J=1.3Hz,7.2Hz),4.05-4.20(1H,m),4.35-4.45(1H,m),4.66(1H,d,J=7.3Hz),4.75-4.88(1H,m),6.49(1H,d,J=8.6Hz),7.18(1H,d,J=7.3Hz),),7.24(1H,t,J=7.3Hz),7.33(1H,t,J=7.3Hz),7.48(1H,s),7.61(1H,d,J=7.3Hz),8.17(1H,d,J=8.0Hz)

Example 46

<u>Synthesis of Compound 49</u>

Compound 49 was prepared using 2-iodopropane instead of iodomethane in the same manner described in Example 44.

m.p.: 75-80 °C
IR(KBr,$cm^{-1}$):
3400,2950,2872,1745,1656,1623,1527,1461,1386,1341, 1311,1260,1197,1146,1107,1092,765

| High Resolution FAB-MS(m/e,($C_{37}H_{57}N_5O_7$+H)$^+$): | |
|---|---|
| Calcd: | 684.4337 |
| Found: | 684.4334 |

$^1$H-NMR(300MHz,$CDCl_3$, δ ppm):
0.82(3H,t,J=7.2Hz),0.88(9H,s),1.05-1.85(14H,m),1.15(3H,d,J=6.8Hz),1.16(3H,d,J=7.1Hz),1.19(3H,d,J=6.3Hz),1.21(3H,d,J=6.3Hz),3.17(1H,dd,J=5.9Hz,14.9Hz),3.37(1H,dd,6.4Hz,14.9Hz),3.90-4.05(2H,m),4.00(3H,s),4.05-4.20(1H,m),4.31-4.42(1H,m),4.67(1H,d,J=6.8Hz),4.75-4.86(1H,m),4.94(1H,sept,J=6.3Hz),6.49(1H,d,J=8.4Hz),7.12(1H,d,J=7.6Hz),7.24(1H,t,J=7.8Hz),7.33(1H,t,J=7.8Hz),7.48(1H,s),7.62(1H,d,J=7.8Hz),8.17(1H,d,J=7.8Hz)

Example 47

<u>Synthesis of Compounds 50 and 51</u>

To a solution of Compound 47 (94 mg, prepared in Example 44) in dry THF (1 ml) were added lithium chloride (12.3 mg), sodium borohydride (11 mg) and dry ethanol (2 ml), and the mixture was stirred at room temperature over night. The mixture was cooled with ice-water, adjusted to pH 4 by the gradual addition of 10% aq. citric acid, and concentrated in vacuo. Water (20 ml) was added to the residue,and the mixture was extracted with dichloromethane (10 ml × 3). Combined organic layers were washed with brine, dried over $MgSO_4$ and evaporated in vacuo. The residue was purified by preparative TLC (Merck, Kieselgel 60 $F_{254}$) with ethyl acetate for development to give Compound 50 (21 mg) and Compound 51 (11 mg).

<u>Compound 50</u>

IR(KBr,$cm^{-1}$):
3400,2950,2866,1743,1653,1620,1536,1461,1386,1260, 1092,765

| High Resolution FAB-MS(m/e,($C_{34}H_{53}N_5O_6$+H)$^+$): | |
|---|---|
| Calcd: | 628.4074 |
| Found: | 628.4075 |

$^1$H-NMR(300MHz,$CDCl_3$, δ ppm):
0.86(3H,t,J=7.1Hz),0.87(9H,s),1.15(3H,d,J=6.9Hz), 1.16(3H,d,J=6.9Hz),1.05-1.85(14H,m),3.22(1H,dd, J=4.9Hz,14.8Hz),3.45(1H,dd,J=6.1Hz,14.8Hz),3.45-3.65(2H,m),3.68-3.79(1H,m),3.79-3.98(2H,m),4.01 (3H,s),4.04-4.17(1H,m),4.65(1H,d,J=6.1Hz),4.75-4.86 (1H,m),5.95(1H,d,J=8.8Hz),7.16(1H,d,J=8.1Hz),7.26 (1H,dt,J=1.3Hz,7.8Hz),7.36(1H,dt,J=1.3Hz,7.8Hz), 7.48(1H,s),7.59(1H,dd,J=1.3Hz,7.8Hz),8.19(1H,dd, J=1.3Hz,7.8Hz)

Compound 51

IR(KBr,$cm^{-1}$):
3304,2950,2866,1743,1653,1611,1539,1461,1386,1254, 1089,1044,756

| High Resolution FAB-MS(m/e,($C_{35}H_{55}N_5O_6$+H)$^+$): | |
|---|---|
| Calcd: | 642.4231 |
| Found: | 642.4227 |

$^1$H-NMR(300MHz,$CDCl_3$, δ ppm):
0.85(3H,t,J=7.0Hz),0.87(9H,s),1.15(3H,d,J=7.1Hz), 1.16(3H,d,J=7.1Hz),1.05-1.83(14H,m),1.46(3H,t, J=7.0Hz),3.23(1H,dd,J=5.3Hz,14.8Hz),3.44(1H,dd, J=6.2Hz,14.8Hz),3.45-3.65(2H,m),3.68-3.78(1H,m), 3.78-3.98(2H,m),4.05-4.16(1H,m),4.40-4.53(2H,m), 4.64(1H,d,J=6.1Hz),4.73-4.85(1H,m),5.95(1H,d, J=8.8Hz),7.15(1H,d,J=8.1Hz),7.25(1H,dt,J=1.2Hz, 7.5Hz),7.35(1H,dt,J=1.2Hz,7.5Hz),7.50(1H,s), 7.59(1H,dd,J=1.2Hz,7.5Hz),8.19(1H,dd,J=1.2Hz,7.5Hz)

Example 48

Synthesis of Compounds 52 and 53

Compound 52 (ester form) and Compound 53 (deprotected form) were prepared using 2,6-dimethylpiperidine and H-Cpeg-OBzl • HCl instead of cyclopentylpropylamine and H-Leu-OBzl • TsOH in the same manner described in Example 17.

Compound 52

| High Resolution FAB-MS(m/e,($C_{38}H_{57}N_5O_7$+H)$^+$): | |
|---|---|
| Calcd: | 696.4337 |
| Found: | 696.4319 |

$^1$H-NMR(300MHz,$CDCl_3$, δ ppm):
0.82(3H,t,J=7.1Hz),0.96-1.81(20H,m),1.17(6H,d, J=7.4Hz),1.41(9H,s),2.09-2.20(1H,m),3.21(1H,dd, J=5.7Hz,14.7Hz),3.33(1H,dd,J=6.5Hz,14.7Hz),3.64-3.75(1H,m),3.90-4.05(1H,m),4.12-4.24(1H,m),4.01 (3H,s),4.25-4.35(1H,m),4.82(1H,d,J=6.9Hz),4.80-4.93 (1H,m),6.33(1H,d,J=9.1Hz),7.16(1H,d,J=7.8Hz),7.25 (1H,dt,J=1.4Hz,7.7Hz),7.33(1H,dt,J=1.4Hz,7.7Hz), 7.47(1H,s),7.63(1H,dd,J=1.4Hz,7.7Hz),8.16(1H,dd,

J=1.4Hz,7.7Hz)

Compound 53

| High Resolution FAB-MS(m/e,($C_{34}H_{49}N_5O_7$+H)$^+$): | |
|---|---|
| Calcd: | 640.3710 |
| Found: | 640.3607 |

$^1$H-NMR(300MHz,DMSO-$d_6$, δ ppm):
0.86(3H,t,J=6.9Hz),1.03(3H,d,J=6.8Hz),1.05(3H,d, J=6.8Hz),0.92-1.80(20H,m),1.80-1.91(1H,m),2.81 (1H,dd,J=11.6Hz,14.6Hz),3.30(1H,dd,J=2.6Hz,14.6Hz), 3.50-3.84(2H,m),3.96(3H,s),4.03-4.20(2H,m),4.50-4.62(1H,m),6.05(1H,d,J=6.8Hz),7.25(1H,dt,J=1.3Hz, 7.3Hz),7.32(1H,dt,J=1.3Hz,7.3Hz),7.53(1H,s),7.64 (1H,dd,J=1.3Hz,7.3Hz),8.05(1H,dd,J=1.3Hz,7.3Hz), 8.23(1H,d,J=7.6Hz),8.33(1H,d,J=8.8Hz)

Example 49

Synthesis of Compound 54

Compound 54 was prepared using H-DβAbu-O$^t$Bu instead of H-DNle-O$^t$Bu in the same manner described in Example 41.

m.p.: 122-126 °C

| High Resolution FAB-MS(m/e,($C_{30}H_{41}N_5O_7$+H)$^+$): | |
|---|---|
| Calcd: | 584.3085 |
| Found: | 584.3079 |

$^1$H-NMR(300MHz,DMSO-$d_6$, δ ppm):
0.03-0.42(4H,m),0.83-1.00(1H,m),1.07(3H,d,J=6.8Hz), 1.09(3H,d,J=7.0Hz),1.13(3H,d,J=6.6Hz),1.33-1.82 (6H,m),2.15(1H,dd,J=8.9Hz,15.0Hz),2.33-2.41(1H,m), 2.75-2.90(1H,m),3.17-3.24(4H,m),3.96(3H,s),4.05-4.23(1H,m),4.34-4.46(1H,m),6.37(1H,d,J=5.6Hz), 7.26(1H,t,J=7.1Hz),7.34(1H,t,J=7.1Hz),7.50(1H,s), 7.63(1H,d,J=7.1Hz),7.92(1H,d,J=7.1Hz),8.05(1H,d, J=8.1Hz),8.60(1H,d,J=8.6Hz)

Preparative Example 4

(1) Preparation of 2,6-dimethylpiperidinocarbonyl-γMeLeu-DTrp(COOMe)-OH

To a solution of Boc-DTrp-OBzl (12.19 g) in dichloromethane (60 ml) were added methyl chloroformate (4.8 ml), pulverized sodium hydroxide (4.1 g) and TBAHS (9.2 g), and the mixture was stirred at room temperature for 4 h. The reaction mixture was washed with water, dried over $MgSO_4$ and evaporated in vacuo. The residue was crystallized from dichloromethane and hexane to give Boc-DTrp(COOMe)-OBzl (12.57 g). Boc-DTrp(COOMe)-OBzl (4.52 g) was

dissolved in 4N-hydrogen chloride/1,4-dioxane (50 ml) and the mixture was stirred at room temperature for 45 min. Ethyl ether (50 ml) was added to the mixture to complete the precipitation. The precipitate was collected by filtration and dried in vacuo to give H-DTrp(COOMe)-OBzl • HCl (3.71 g). To a suspension of H-DTrp(COOMe)-OBzl • HCl (1.94 g) in dichloromethane (50 ml) were added NMM (0.55 ml), 2,6-dimethylpiperidinocarbonyl- γMeLeu-OH (1.42 g), HOBT • $H_2O$ (0.84 g) and EDCI • HCl (1.05 g) under ice cooling. The mixture was stirred at the same temperature for 2 h and at room temperature over night. The reaction mixture was washed with sat. aq $NaHCO_3$ (50 ml), 10% aq. citric acid (50 ml) and brine (50 ml) successively, dried over $MgSO_4$ and evaporated. The residue was purified by MPLC (Merck, LiChroprep Si 60) with hexane/ethyl acetate = 2/1 for elution to give 2,6-dimethylpiperidinocarbonyl- γMeLeu-DTrp(COOMe)-OBzl (1.17 g). A solution of 2,6-dimethylpiperidinocarbonyl- γMeLeu-DTrp(COOMe)-OBzl (1.10 g) in methanol (30 ml) was hydrogenated over 10% Pd/C (50 mg) at an atmospheric pressure of hydrogen for 1.5 h. After removal of the catalyst by filtration, the filtrate was evaporated in vacuo to give the product (0.93 g).

(2)

To a solution of 2,6-dimethylpiperidinocarbonyl-γMeLeu-DTrp(COOMe)-OH (53 mg, prepared in (1)) and HOSu (17 mg) in DMF (1.0 ml) was added EDCI • HCl (29 mg) under ice cooling, and the mixture was stirred at the same temperature for 3.5 h. To the mixture was added a solution of glycine (11 mg) and NMM (16 μl) in water (1.0 ml). The mixture was stirred at the same temperature for 2 h and at room temperature over night. To the mixture was added 1N-hydrochloric acid (20 ml), and the mixture was extracted with dichloromethane (10 ml × 3). The combined organic layers were washed with brine (10 ml), dried over $MgSO_4$ and evaporated in vacuo. The residue was purified by preparative TLC (Merck, Kieselgel 60 $F_{254}$) with chloroform/methanol/acetic acid = 20/1/1 for development to give the Compound (23 mg).

m.p.: 124-127 °C
IR(KBr,$cm^{-1}$):
2960,1738,1659,1612,1531,1458,1444,1383,1259,1090, 766,748

| High Resolution FAB-MS(m/e,($C_{30}H_{43}N_5O_7$+H)$^+$): | |
|---|---|
| Calcd: | 586.3241 |
| Found: | 586.3232 |

$^1$H-NMR(300MHz,$CDCl_3$, δ ppm):
0.74(9H,s),1.16(3H,d,J=6.8Hz),1.19(3H,d,J=6.8Hz), 1.31-1.82(8H,m),3.14(1H,dd,J=10.0Hz,14.8Hz),3.49 (1H,dd,4.4Hz,14.8Hz),3.74(1H,dd,J=6.3Hz,16.7Hz), 3.90-4.14(3H,m),3.97(3H,s),4.22(1H,dd,J=6.3Hz, 16.7Hz),4.77-4.93(2H,m),7.13-7.36(3H,m),7.51(1H,s), 7.57(1H,d,J=7.7Hz),7.87(1H,t,J=6.3Hz),8.13(1H,d, J=7.7Hz)

Compound 55 in the following Example 50 was prepared using each corresponding amino acid instead of glycine in the same manner described in Preparative Example 4.

Example 50

<u>Compound 55</u>

m.p.: 103-108 °C
IR(KBr,$cm^{-1}$):
3300,2958,2939,1738,1653,1533,1456,1444,1383,1259, 1092,766,748

| High Resolution FAB-MS(m/e,($C_{35}H_{53}N_5O_7$+H)$^+$): | |
|---|---|
| Calcd: | 656.4023 |
| Found: | 656.4044 |

$^1$H-NMR(300MHz,$CDCl_3$, δ ppm):
0.82(3H,t,J=7.0Hz),0.83(9H,s),1.18(3H,d,J=6.8Hz), 1.19(3H,d,J=6.8Hz),1.08-1.82(14H,m),2.40(1H,dd, J=5.2Hz,15.4Hz),2.53(1H,dd,J=6.7Hz,15.4Hz),3.22 (1H,dd,J=6.8Hz,14.9Hz),3.29(1H,dd,J=6.8Hz,14.9Hz), 3.86-4.24(4H,m),4.02(3H,s),4.67-4.79(1H,m), 6.48(1H,d,J=6.9Hz),7.16(1H,brs),7.21-7.40(2H,m), 7.32(1H,t,J=7.8Hz),7.48(1H,s),7.60(1H,d,J=7.8Hz), 8.16(1H,d,J=7.8Hz)

Preparative Example 5

To a solution of 2,6-dimethylpiperidinocarbonyl-γMeLeu-DTrp(COOMe)-OH (53 mg, prepared in Preparative Example 4-(1)) and HOSu (17 mg) in DMF (1.0 ml) was added EDCI • HCl (29 mg) under ice cooling, and the mixture was stirred at the same temperature for 3.5 h. To the mixture was added a solution of aminomethanesulfonic acid (17 mg) and NMM (16 μl) in water (1.0 ml). The mixture was stirred at the same temperature for 2 h and at room temperature over night. The mixture was purified by reverse phase MPLC (Merck,LiChroprep RP-18) with methanol/water(containing 0.1% TFA) = 3/2 for elution to give the product (26 mg).

m.p.: 135-140 °C
IR(KBr,$cm^{-1}$):
1782,1737,1666,1547,1458,1385,1259,1207,1169,1092, 1041,768

| High Resolution FAB-MS(m/e,($C_{29}H_{43}N_5O_8S$+H)$^+$): | |
|---|---|
| Calcd: | 622.2911 |
| Found: | 622.2953 |

$^1$H-NMR(300MHz,DMSO-$d_6$, δ ppm):
0.73(9H,s),1.02(3H,d,J=6.9Hz),1.03(3H,d,J=6.9Hz), 0.98-1.73(8H,m),2.88(1H,dd,J=10.2Hz,15.1Hz),3.09 (1H,dd,4.3Hz,15.1Hz),3.93(3H,s),3.82-4.40(5H,m), 4.59-4.69(1H,m),5.93(1H,brs),7.22(1H,t,J=7.7Hz), 7.30(1H,t,J=7.7Hz),7.52(1H,s),7.74(1H,d,J=7.7Hz), 8.03(1H,d,J=7.7Hz),8.03(1H,d,J=8.0Hz),8.44(1H,t, J=6.1Hz)

Each Compound 56 and 57 in the following Examples 51 and 52 was prepared using each corresponding aminosulfonic acid instead of aminomethanesulfonic acid in the same manner described in Preparative Example 5. J=7.9Hz)

Example 51

Compound 56

m.p.: 141-144 °C
IR(KBr,$cm^{-1}$):
3400,2958,1778,1738,1660,1539,1456,1383,1259,1153, 1092,1036,768

| High Resolution FAB-MS(m/e,($C_{34}H_{53}N_5O_8S$+H)$^+$): | |
|---|---|
| Calcd: | 692.3693 |
| Found: | 692.3713 |

$^1$H-NMR(300MHz,DMSO-$d_6$, δ ppm):
0.73(9H,s),0.83(3H,t,J=6.7Hz),1.04(3H,d,J=6.7Hz), 1.05(3H,d,J=6.7Hz),0.97-2.00(14H,m),2.42-2.53 (2H,m),2.87(1H,dd,J=10.6Hz,15.0Hz),3.21(1H,dd, J=3.2Hz,15.0Hz),3.94(3H,s),3.90-4.37(5H,m),6.10 (1H,d,J=6.6Hz),7.24(1H,t,J=7.4Hz),7.31(1H,t, J=7.4Hz),7.51(1H,s),7.66(1H,d,J=7.4Hz),7.75(1H,d, J=8.5Hz),8.04(1H,d,J=7.4Hz),8.21(1H,d,J=7.7Hz)

Example 52

Compound 57

IR(KBr,cm$^{-1}$):
2954,2866,1778,1738,1666,1539,1477,1456,1383,1257, 1169,1092,1041,768,748,627

| High Resolution FAB-MS(m/e,($C_{33}H_{51}N_5O_8S$+H)$^+$): | |
|---|---|
| Calcd: | 678.3537 |
| Found: | 678.3507 |

$^1$H-NMR(300MHz,DMSO-$d_6$, δ ppm):
0.72+0.75(9H,s),0.80-1.96(23H,m),2.80-2.93(1H,m), 3.05-3.18(1H,m),3.93(3H,s),3.99-4.71(5H,m),5.88+ 6.01(1H,brs),7.21(1H,t,J=8.0Hz),7.30(1H,t,J=8.0Hz), 7.72+7.75(1H,d×2,J=8.0Hz), 7.56(1H,s),7.83-8.12 (3H,m)

Example 53

Synthesis of Compound 58

To a solution of 2,6-dimethylpiperidinocarbonyl-γMeLeu-DTrp(COOMe)-OH (106 mg, prepared in Preparative Example 4-(1)), diethyl (R)-1-aminopentylphosphonate (67 mg) and HOBT • $H_2O$ (51 mg) in dichloromethane (10 ml) was added EDCI • HCl (73 mg) under ice cooling, and the mixture was stirred at room temperature for 16 h. The mixture was diluted with ethyl acetate, washed with 10% aq. citric acid, water, sat. aq. $NaHCO_3$ and brine successively, dried over $MgSO_4$ and evaporated in vacuo. The residue was purified by silica gel chromatography (Merck,Kieselgel 60) with hexane/ethyl acetate = 1/1 for elution to give the diethyl ester of Compound 58 (61 mg). To a solution of the diethyl ester (41 mg) in dichloromethane (0.8 ml) was added bromotrimethylsilane (0.8 ml) under ice cooling. The mixture was stirred at room temperature for 4 h, and concentrated in vacuo. The residue was dissolved in 90% acetone/water (5 ml). The mixture was stirred at 45 min and evaporated in vacuo. The residue was triturated with water to give Compound 58 (25 mg).

m.p.: 205-210 °C
IR(KBr,cm$^{-1}$):
2954,2949,1736,1649,1524,1458,1383,1259,1092,768

| High Resolution FAB-MS(m/e,($C_{33}H_{52}N_5O_8P$+H)$^+$): | |
|---|---|
| Calcd: | 678.3632 |
| Found: | 678.3674 |

$^1$H-NMR(300MHz,$CD_3OD$, δ ppm):
0.82(9H,s),0.92(3H,t,J=6.8Hz),1.13(3H,d,J=6.8Hz), 1.14(3H,d,J=6.8Hz),1.20-2.00(14H,m),3.0-3.1(1H,m), 3.3-3.4(1H,m),4.01(3H,s),4.05-4.30(4H,m),4.7-4.8 (1H,m),7.25(1H,t,J=7.3Hz),7.31(1H,t,J=7.3Hz), 7.53(1H,s),7.67(1H,d,J=7.3Hz),8.12(1H,d,J=7.3Hz)

Example 54

Synthesis of Compound 59

To a solution of Compound 21 (32 mg, prepared in Example 21), 2-aminoethanol (4 mg) and HOBT • $H_2O$ (10 mg) was added EDCI • HCl (13 mg) under ice cooling. The mixture was stirred at the same temperature for 1 h and at room temperature over night. The mixture was diluted with ethyl acetate (20 ml), washed with sat. aq. $NaHCO_3$ (20 ml), 10% aq. citric acid (20 ml) and brine successively,dried over $MgSO_4$ and evaporated in vacuo. The residue was purified by preparative TLC (Merck, Kieselgel 60 $F_{254}$) with chloroform/methanol = 30/1 for development to give the product (23.5 mg).

m.p.: 106-109 °C
IR(KBr,cm$^{-1}$):
1736,1655,1610,1524,1458,1383,1257,1088,1257,1088, 766

| High Resolution FAB-MS(m/e,($C_{36}H_{56}N_6O_7$+H)$^+$): | |
|---|---|
| Calcd: | 685.4289 |
| Found: | 685.4280 |

$^1$H-NMR(300MHz,$CDCl_3$, δ ppm):
0.78(9H,s),0.84(3H,t,J=7.1Hz),1.13(6H,d,J=7.1Hz), 1.04-1.79(13H,m),2.00-2.15(1H,m),3.22-3.43(4H,m), 3.55-3.80(3H,m),3.82-4.17(2H,m),4.04(3H,s),4.44-4.56(2H,m),4.59-4.71(1H,m),4.68(1H,d,J=4.7Hz), 6.31(1H,d,J=6.8Hz),6.75(1H,t,J=4.3Hz),7.28(1H,t, J=7.8Hz),7.38(1H,t,J=7.8Hz),7.52(1H,s),7.59(1H,d, J=7.8Hz),7.73(1H,d,J=9.0Hz),8.20(1H,d,J=7.8Hz)

Example 55

Synthesis of Compound 60

To a solution of 2,6-dimethylpiperidinocarbonyl-γMeLeu-DTrp(COOMe)-OH (20 mg) and NMM (4.2 µl) in dry THF (2.0 ml) was added isopropyl chloroformate (5.3 µl) at -25 °C over 5 min. To the mixture was added a solution of (R)-5-(1-aminopentyl)-1H-tetrazole hydrochloride (10.9 mg) and NMM (6.3 µl) in dry THF (2.0 ml) at -20 °C. The mixture was stirred at -20 °C - -15 °C for 2 h and at 10 °C for 10 h, then concentrated under reduced pressure. The residue was partitioned between ethyl acetate (10 ml) and water (10 ml). The organic layer was washed with 1N-hydrochloric acid (10 ml × 2) and water (10 ml), dried over anhydrous sodium sulfate and evaporated in vacuo. The residue was purified by preparative TLC (Merck, Kieselgel 60 $F_{254}$) with chloroform/methanol = 20/1 for development to give the product (13.5 mg).

m.p.: 111-116 °C
IR(KBr,$cm^{-1}$):
3400,3300,2942,2870,1659,1612,1529,1456,1383,1259, 1091,766,748

| High Resolution FAB-MS(m/e,($C_{34}H_{51}N_9O_5$+H)$^+$): | |
|---|---|
| Calcd: | 666.4092 |
| Found: | 666.4113 |

$^1$H-NMR(300MHz,$CDCl_3$, δ ppm):
0.83(9H,s),0.88(3H,d,J=6.7Hz),0.98(3H,t,J=6.7Hz), 0.99(3H,d,J=6.7Hz),1.08-1.90(13H,m),2.16-2.30 (1H,m),3.30-3.48(2H,m),3.54(1H,quint,J=6.7Hz), 3.61-3.82(2H,m),4.05(3H,s),4.62-4.73(1H,m),4.66 (1H,d,J=6.0Hz),5.44(1H,dt,J=3.9Hz,9.7Hz),6.32(1H,d, J=6.6Hz),7.30(1H,t,J=7.4Hz),7.40(1H,t,J=7.4Hz), 7.54(1H,s),7.61(1H,d,J=7.4Hz),7.94(1H,d,J=9.7Hz), 8.20(1H,d,J=7.4Hz)

Each Compound 61 and 62 in the following Examples 56 and 57 was prepared using 2,6-dimethylpiperidinocarbonyl-Val-DTrp(COOMe)-OH and each corresponding aminosulfonic acid as starting materials in the same manner described in Preparative Example 5.

Example 56

<u>Compound 61</u>

IR(KBr,$cm^{-1}$):
3290,2960,2935,1738,1668,1610,1522,1458,1383,1259, 1201,1092,768,748
FAB-MS(m/e,($C_{31}H_{47}N_5O_8S$+H)$^+$):650
$^1$H-NMR(300MHz,$CD_3OD$, δ ppm):
0.52(3H,d,J=6.8Hz),0.77(3H,d,J=6.8Hz),0.80-1.00 (3H,m),1.17+1.19(3H×2,d×2,J=6.6Hz),1.10-1.85 (13H,m),2.96(1H,dd,J=11.2Hz,14.6Hz),3.30-4.50 (5H,m),4.01(3H,s),4.75(1H,dd,J=3.9Hz,11.2Hz), 7.22-7.34(2H,m),7.54(1H,s),7.67(1H,d,J=7.2Hz), 8.10(1H,d,J=8.1Hz)

Example 57

<u>Compound 62</u>

m.p.: 128-131 °C
IR(KBr,$cm^{-1}$):
3273,2960,1778,1736,1662,1541,1456,1382,1259,1201, 1039,768,748

| High Resolution FAB-MS(m/e,($C_{32}H_{49}N_5O_8S$+H)$^+$): | |
|---|---|
| Calcd: | 664.3380 |
| Found: | 664.3437 |

$^1$H-NMR(300MHz,DMSO-$d_6$, δ ppm):

0.51(3H,d,J=6.4Hz),0.65(3H,d,J=6.4Hz),0.83(3H,t, J=7.0Hz),1.07(6H,d,J=6.5Hz),1.01-2.00(13H,m),2.40-2.55(2H,m),2.83(1H,dd,J=11.0Hz,14.5Hz),2.23(1H,dd, J=3.0Hz,14.5Hz),3.69-3.83(1H,m),3.95(3H,s),3.97-4.23(3H,m),4.35-4.48(1H,m),5.95(1H,brs),7.25(1H,t, J=7.5Hz),7.32(1H,t,J=7.5Hz),7.51(1H,s),7.66(1H,d, J=7.5Hz),7.81(1H,d,J=8.3Hz),8.03(1H,d,J=7.5Hz), 8.34(1H,d,J=8.1Hz)

Each Compound 63 and 64 in the following Examples 58 and 59 was prepared using 2,6-dimethylpiperidinocarbonyl-Leu-DTrp(COOMe)-OH and each corresponding aminosulfonic acid as starting materials in the same manner described in Preparative Example 5.

Example 58

Compound 63

m.p.: 139-142 °C
IR(KBr,$cm^{-1}$):
3271,2956,2871,1774,1736,1655,1543,1458,1383,1259, 1092,1039,768

| High Resolution FAB-MS(m/e,($C_{33}H_{51}N_5O_8S$+H)$^+$): | |
|---|---|
| Calcd: | 678.3537 |
| Found: | 678.3585 |

$^1$H-NMR(300MHz,DMSO-$d_6$, δ ppm):
0.67(3H,d,J=5.6Hz),0.71(3H,d,J=5.6Hz),0.83(3H,t, J=6.8Hz),1.04(3H,d,J=6.7Hz),1.05(3H,d,J=6.7Hz), 1.10-2.00(15H,m),2.45-2.65(2H,m),2.85(1H,dd, J=10.8Hz,14.5Hz),3.22(1H,dd,J=3.4Hz,14.5Hz), 3.95(3H,s),3.95-4.45(5H,m),6.07(1H,brs), 7.24(1H,t,J=7.6Hz),7.32(1H,t,J=7.6Hz),7.47(1H,s), 7.66(1H,d,J=7.6Hz),7.78(1H,d,J=8.5Hz),8.04(1H,d, J=7.6Hz),8.21(1H,d,J=7.8Hz)

Example 59

Compound 64

IR(KBr,$cm^{-1}$):
3271,2956,2872,1738,1668,1610,1539,1456,1385,1259, 1203,1092,1041,768

| High Resolution FAB-MS(m/e,($C_{32}H_{49}N_5O_8S$+Na)$^+$): | |
|---|---|
| Calcd: | 686.3187 |
| Found: | 686.3200 |

$^1$H-NMR(300MHz,DMSO-$d_6$, δ ppm):
0.57-0.89(9H,m),0.95-1.10(6H,m),1.10-1.97(15H,m), 2.78-2.93(1H,m),3.09-3.31(1H,m),3.94+3.95(3H,s×2), 4.00-4.75(5H,m),5.85+6.10(1H,brs×2),7.18+7.54 (1H,brs×2),7.20-7.36(2H,m),7.46+7.55(1H,s×2), 7.64+7.76(1H,d×2,J=7.3Hz),8.03+8.05(1H,d×2,

J=7.3Hz),7.95+8.33(1H,d×2,J=9.5Hz,8.8Hz)

Example 60

Synthesis of Compound 65

(1) Preparation of 2,6-dimethylpiperidinocarbonyl-γMeLeu-DTrp(2-Br)-DNle-O$^t$Bu

To a solution of 2,6-dimethylpiperidinocarbonyl-γMeLeu-DTrp-DNle-O$^t$Bu (320 mg) in anhydrous acetic acid (1.0 ml) was added dropwise over 10 min a solution of N-bromosuccinimide (107 mg) in anhydrous acetic acid (3.0 ml). After being stirred at room temperature for 20 h, the mixture was diluted with dichloromethane (50 ml) and the solution was washed with water (25 ml × 2), sat. aq. $NaHCO_3$ (25 ml) and sat. brine successively, dried over $MgSO_4$ and evaporated in vacuo. The residue was purified by preparative TLC (Merck, Kieselgel 60 $F_{254}$) with chloroform/methanol = 30/1 for development to give the product (15 mg).

FAB-MS (m/e, $(C_{36}H_{56}BrN_5O_5+H)^+$) : 718 and 720

(2) Preparation of Compound 65

A solution of 2,6-dimethylpiperidinocarbonyl-γMeLeu-DTrp(2-Br)-DNle-O$^t$Bu (14 mg, prepared in (1)) in TFA (1.0 ml) was stirred under ice cooling for 3 h. The mixture was concentrated under reduced pressure and the residue was purified by preparative TLC (Merck, Kieselgel 60 $F_{254}$) with chloroform/methanol/acetic acid = 30/1/1 for development to give Compound 65 (8.4 mg).

m.p.: 145-148 °C
IR(KBr,$cm^{-1}$):
2954,2868,1724,1659,1620,1514,1247,1136,743

| High Resolution FAB-MS(m/e, $(C_2H_{48}BrN_5O_5+H)^+$): | |
|---|---|
| Calcd: | 662.2917 |
| Found: | 662.2944 |

$^1$H-NMR(300MHz,$CDCl_3$, δ ppm):
0.78(3H,t,J=7.1Hz),0.91(9H,s),1.13(3H,d,J=7.1Hz), 1.16(3H,d,J=7.1Hz),0.98-1.86(14H,m),3.19(1H,dd, J=6.2Hz,14.6Hz),3.39(1H,dd,5.7Hz,14.6Hz),3.93-4.20 (3H,m),4.20-4.35(1H,m),4.50-4.66(1H,m),4.90-5.07 (1H,m),6.54-6.66(1H,m),7.09(1H,t,J=7.6Hz),7.15 (1H,t,J=7.6Hz),7.25(1H,d,J=7.6Hz),7.62(1H,d, J=7.6Hz),7.30-7.44(1H,m),8.74(1H,brs)

Example 61

Synthesis of Compound 66

(1) Preparation of α-N,1-bis-t-butoxycarbonyl-α-N-trifluoroacetyl-2-chloro-D-tryptophan methyl ester

To a solution of α-N-trifluoroacetyl-2-chloro-D-tryptophan methyl ester (283 mg) in acetonitrile (5 ml) were added di-tert-butyl dicarbonate and DMAP (20 mg) and the mixture was stirred at room temperature for 23 h. The mixture was evaporated in vacuo and the residue was purified by MPLC (Merck, LicChroprep Si 60) with hexane/ethyl acetate = 5/1 for elution to give the product (265 mg).

FAB-MS (m/e, $(C_{24}H_{28}F_3ClN_2O_7)^+$) : 548 and 550

(2) Preparation of α-N,1-bis-t-butoxycarbonyl-2-chloro-D-tryptophan

To a solution of α-N,1-bis-t-butoxycarbonyl-α-N-trifluoroacetyl-2-chloro-D-tryptophan methyl ester (255 mg, prepared in (1)) in methanol (5 ml) was added 1N NaOH (1.03 ml) and the mixture was stirred at room temperature for 7 h. The mixture was diluted with water (50 ml), acidified with 10% citric acid aq. solution and the mixture was extracted with ethyl acetate (30 ml × 3). Combined organic extracts were washed with sat. brine (30 ml), dried over $MgSO_4$ and evaporated in vacuo to give the product (206 mg).

FAB-MS (m/e, $(C_{21}H_{27}ClN_2O_6)^+$) : 438 and 440

(3) Preparation of Boc-DTrp(1-Boc,2-Cl)-DNle-$O^tBu$

To a solution of α-N,1-bis-t-butoxycarbonyl-2-chloro-D-tryptophan (206 mg, prepared in (2)) and H-DNle-$O^tBu$ • HCl (125 mg) in dichloromethane (10 ml) were added NMM (62 μl), HOBT • $H_2O$ (86 mg) and EDCI • HCl (107 mg) under ice cooling. The mixture was stirred at the same temperature for 1 h and at room temperature for 3 h. The mixture was diluted with dichloromethane (20 ml), washed with sat. aq. $NaHCO_3$ (10 ml), 10% citric acid aq. solution (10 ml) and sat. brine (10 ml) successively, dried over $MgSO_4$ and evaporated in vacuo. The residue was purified by silica gel chromatography (Merck, Kieselgel 60) with hexane/ethyl acetate = 5/1 for elution to give the product (235 mg).

FAB-MS (m/e, $(C_{31}H_{46}ClN_3O_7+H)^+$) : 608 and 610

(4) Preparation of H-DTrp(1-Boc,2-Cl)-DNle-$O^tBu$

A solution of Boc-DTrp(1-Boc,2-Cl)-DNle-$O^tBu$ (252 mg, prepared in (3)) in formic acid (10 ml) was stirred at room temperature for 1 h. The mixture was concentrated under reduced pressure and the residue was dissolved in ethyl acetate (50 ml). The solution was washed with sat. aq. $NaHCO_3$ (50 ml × 2) and sat. brine (50 ml) successively, dried over $MgSO_4$ and evaporated in vacuo. The residue was purified by preparative TLC (Merck, Kieselgel 60 $F_{254}$) with chloroform/methanol = 30/1 for development to give the product (98 mg).

FAB-MS (m/e, $(C_{26}H_{38}ClN_3O_5+H)^+$) : 508 and 510

(5) Preparation of 2,6-dimethylpiperidinocarbonyl-γMeLeu-DTrp(1-Boc,2-Cl)-DNle-$O^tBu$

To a solution of H-DTrp(1-Boc,2-Cl)-DNle-$O^tBu$ (39 mg, prepared in (4)) and 2,6-dimethylpiperidinocarbonyl-γMeLeu-OH (34 mg) in dichloromethane (3 ml) were added HOBT • $H_2O$ (18 mg) and EDCI • HCl (23 mg) under ice cooling. The mixture was stirred at the same temperature for 1 h and at room temperature for 3 h. The mixture was diluted with dichloromethane (30 ml), washed with sat. aq. $NaHCO_3$ (20 ml), 10% citric acid aq. solution (20 ml) and sat. brine successively, dried over $MgSO_4$ and evaporated in vacuo. The residue was purified by preparative TLC (Merck, Kieselgel 60 $F_{254}$) with hexane/ethyl acetate = 2/1 for development to give the product (51 mg).

FAB-MS (m/e, $(C_{41}H_{64}ClN_5O_7+H)^+$) : 774 and 776

(6) Preparation of Compound 66

A mixture of 2,6-dimethylpiperidinocarbonyl-γMeLeu-DTrp(1-Boc,2-Cl)-DNle-$O^tBu$ (12.3 mg, prepared in (5)) in TFA (1.0 ml) was stirred at room temperature for 1 h and concentrated in vacuo. The residue was purified by preparative TLC (Merck, Kieselgel 60 $F_{254}$) with chloroform/methanol/acetic acid = 30/1/1 for development to give Compound 66 (7.4 mg).

m.p.: 145-149 °C
IR(KBr,$cm^{-1}$):
3290,2956,2360,1646,1618,1533,1247,1128,743

| High Resolution FAB-MS(m/e,($C_{32}H_{48}ClN_5O_5$+H)$^+$): | |
|---|---|
| Calcd: | 618.3422 |
| Found: | 618.3431 |

$^1$H-NMR(300MHz,$CDCl_3$, δ ppm):
0.78(3H,t,J=7.2Hz),0.91(9H,s),1.13(3H,d,J=6.9Hz), 1.16(3H,d,J=6.9Hz),0.96-1.88(14H,m),3.20(1H,dd, J=5.7Hz,15.0Hz),3.39(1H,dd,5.7Hz,15.0Hz),3.90-4.08 (2H,m),4.08-4.20(1H,m),4.20-4.33(1H,m),4.48-4.64 (1H,m),4.90-5.05(1H,m),6.49-6.63(1H,m),7.10(1H,t, J=8.0Hz),7.17(1H,t,J=8.0Hz),7.25(1H,d,J=8.0Hz), 7.59(1H,d,J=8.0Hz),7.33-7.48(1H,m),8.61(1H,brs)

Each Compound 67 and 68 in the following Examples 62 and 63 was prepared using 2,6-dimethylpiperidinocarbonyl-Leu-OH or hexahydroazepinylcarbonyl-Leu-OH respectively instead of 2,6-dimethylpiperidinocarbonyl-γMeLeu-OH in Example 61-(5) in the same manner described in Example 61-(5) and (6).

Example 62

<u>Compound 67</u>

m.p.: 142-147 °C
IR(KBr,$cm^{-1}$):
3402,2956,2870,2360,1653,1622,1522,1387,1340,1238, 1128,743

| High Resolution FAB-MS(m/e,($C_{31}H_{46}ClN_5O_5$+H)$^+$): | |
|---|---|
| Calcd: | 604.3266 |
| Found: | 604.3260 |

$^1$H-NMR(300MHz,$CDCl_3$, δ ppm):
0.79(3H,t,J=7.2Hz),0.86(3H,d,J=4.9Hz),0.88(3H,d, J=4.9Hz),1.15(3H,d,J=6.9Hz),1.16(3H,d,J=6.9Hz), 0.93-1.87(15H,m),3.21(1H,dd,J=6.3Hz,14.4Hz), 3.38(1H,dd,6.3Hz,14.4Hz),3.91-4.07(2H,m),4.07-4.20 (1H,m),4.20-4.34(1H,m),4.52-4.73(1H,m),4.82-5.02 (1H,m),6.52-6.70(1H,m),7.11(1H,t,J=7.1Hz),7.17 (1H,t,J=7.1Hz),7.26(1H,d,J=7.1Hz),7.59(1H,d, J=7.1Hz),7.31-7.49(1H,m),8.63(1H,brs)

Example 63

<u>Compound 68</u>

m.p.: 127-130 °C
IR(KBr,$cm^{-1}$):
3300,2929,2864,1647,1529,1452,1342,1217,743

| High Resolution FAB-MS(m/e,($C_{30}H_{44}ClN_5O_5$+H)$^+$): | |
|---|---|
| Calcd: | 590.3109 |
| Found: | 590.3067 |

$^1$H-NMR(300MHz,$CDCl_3$, δ ppm):
0.78(3H,t,J=7.2Hz),0.86(3H,d,J=4.4Hz),0.88(3H,d,J= 4.4Hz),0.92-1.87(17H,m),3.05-3.47(6H,m),3.92-4.06 (1H,m),4.23-4.38(1H,m),4.51-4.70(1H,m),4.92-5.08 (1H,m),6.50-6.70(1H,m),7.11(1H,t,J=7.6Hz),7.17 (1H,t,J=7.6Hz),7.26(1H,d,J=7.6Hz),7.59(1H,d,J= 7.6Hz),7.37-7.52(1H,m),8.60(1H,brs)

Example 64

Synthesis of Compound 69

To a solution of 2,6-dimethylpiperidinocarbonyl-γMeLeu-DTrp-DNle-O$^t$Bu (199 mg) in acetic acid (2 ml) was added methanesulfenyl chloride and the mixture was stirred at room temperature for 2 days. The mixture was concentrated under reduced pressure and the residue was purified by preparative TLC (Merck, Kieselgel 60 $F_{254}$) with chloroform/methanol = 10/1 for development to give Compound 69 (65 mg).

m.p.: 158 - 161 °C
IR(KBr,$cm^{-1}$):
3305,2954,2869,1652,1618,1521,1448,1386,1247,744

| High Resolution FAB-MS(m/e,($C_{33}H_{51}N_5O_5S$+H)$^+$): | |
|---|---|
| Calcd: | 630.3689 |
| Found: | 630.3663 |

$^1$H-NMR(300MHz,aceton-$d_6$, δ ppm):
0.80-1.90(23H,m),0.83(9H,s),2.47(3H,s),3.27(1H,dd, J=8.9Hz,14.2Hz),3.51(1H,dd,4.4Hz,14.2Hz),4.00-4.15 (1H,m),4.15-4.35(3H,m),4.69-4.77(1H,m),5.65(1H,d, J=8.4Hz),7.02(1H,t,J=7.6Hz),7.10(1H,t,J=7.6Hz), 7.31(1H,d,J=7.6Hz),7.40-7.43(1H,m),7.69(1H,d,J=7.6 Hz),7.75(1H,d,J=6.6Hz),10.37(1H,s)

Example 65

Synthesis of Compound 70

To a solution of Compound 69 (50 mg, prepared in Example 64) in acetic acid (2 ml) was added 30% hydrogen peroxide (13.6 µl) and the mixture was stirred at room temperature for 2 h. The mixture was concentrated under reduced pressure and the residue was purified by preparative TLC (Merck, Kieselgel 60 $F_{254}$) with chloroform/methanol/acetic acid = 10/1/1 for development to give Compound 70 (18.9 mg).

m.p.: 161-168 °C
IR(KBr,$cm^{-1}$):
3390,2956,1652,1603,1538,1405,1128,746

| High Resolution FAB-MS(m/e,($C_{33}H_{52}N_5O_6S$+H)$^+$): | |
|---|---|
| Calcd: | 646.3638 |
| Found: | 646.3663 |

$^1$H-NMR(300MHz,acetone-$d_6$-$CD_3OD$,dppm):
0.80-1.90(23H,m),0.82+0.87(9H,s×2),3.06(3H,s), 3.15-3.65(2H,m),4.05-4.40(4H,m),4.62-4.78(1H,m), 7.12(1H,t,J=7.5Hz),7.26(1H,t,J=7.5Hz),7.46(1H,d, J=7.5Hz),7.81-7.89(1H,m)

Example 66

Synthesis of Compound 71

To a solution of Compound (23 mg, prepared in Example 101) in acetic acid (1 ml) was added 30% hydrogen peroxide (41 μl) and the mixture was stirred at room temperature for 4 h. The mixture was concentrated under reduced pressure and the residue was purified by preparative TLC (Merck, Kieselgel 60 $F_{254}$) with chloroform/methanol = 10/1 for development to give Compound 71 (4 mg).

FAB-MS (m/e, ($C_{33}H_{51}N_5O_7S$-H)$^-$) : 660
$^1$H-NMR(300MHz,acetone-$d_6$, δ ppm):
0.80(9H,s)0.84-1.86(23H,m),3.34(3H,s)3.50-3.65 (1H,m),3.78(1H,dd,J=4.3Hz,14.3Hz),3.90-4.04(1H,m), 4.15-4.32(3H,m),4.65-4.75(1H,m),5.64(1H,d,J= 6.5Hz),7.19(1H,t,J=7.4Hz),7.36(1H,t,J=7.4Hz), 7.55(1H,d,J=7.4Hz),7.71(1H,brs),7.87(1H,d,J= 7.4Hz),7.97(1H,d,J=8.0Hz),10.95(1H,brs)

Referential Example 1

Synthesis of (R)-2-Aminohexanesulfonic acid

(1) Preparation of (R)-2-t-butoxycarbonylamino-1-hexanol

To a solution of Boc-DNle-OMe (4.23 g) in dry THF (30 ml) were added lithium chloride (0.85 g), sodium borohydride (0.76 g) and dry ethanol (60 ml), and the mixture was stirred at the same temperature for 2 h and at room temperature over night. The mixture was cooled with ice-water, adjusted to pH 3 by the gradual addition of 10% aq. citric acid, and concentrated in vacuo. Water (200 ml) was added to the residue,and the mixture was extracted with dichloromethane (50 ml × 3). The combined organic layers were washed with brine, dried over $MgSO_4$ and evaporated in vacuo. The residue was purified by silica gel chromatography (Merck, Kieselgel 60) with hexane/ethyl acetate = 1/1 for elution to give the product (2.95 mg).

(2) Preparation of (R)-2-t-butoxycarbonylamino-1-hexyl methanesulfonate

To a solution of (R)-2-t-butoxycarbonylamino-1-hexanol (2.17 g, prepared in (1)) in dichloromethane (20 ml) were added triethylamine (1.67 ml) and methanesulfonyl chloride (0.93 ml) under ice cooling. After being stirred at the same temperature for 1 h, the mixture was washed with water, 10% aq. citric acid, sat. aq $NaHCO_3$ and brine successively, dried over $MgSO_4$ and evaporated in vacuo. The residue was purified by silica gel chromatography (Merck, Kieselgel 60) with hexane/ethyl acetate = 2/1 for elution to give the product (1.32 g).

(3) Preparation of (R)-2-aminohexanesulfonic acid

A mixture of (R)-2-t-butoxycarbonylamino-1-hexyl methanesulfonate (428 mg, prepared in (1)) in 4N-hydrogen chloride/1,4-dioxane (10 ml) was stirred at room temperature for 1 h, and evaporated in vacuo. The residue was dissolved in water (3.0 ml) and sodium sulfite (378 mg) was added. After being stirred at room temperature for 4 d, the reaction mixture was passed through a Dowex 50W-X8 column ($H^+$ form) and washed with excess water. The combined

water solution was concentrated in vacuo to give the product (114 mg).

FAB-MS(m/e, $(C_6H_{15}NO_3S+H)^+$):182

Referential Example 2

Synthesis of (R)-3-Aminoheptanoic acid

(1) Preparation of (R)-2-t-butoxycarbonylaminoheptanenitrile

A mixture of (R)-2-t-butoxycarbonylamino-1-hexyl methanesulfonate (561 mg, prepared in Referential Example 1-(2)) and sodium cyanide (117 mg) in DMF (3 ml) was stirred at 60 °C for 5 h. After cooling, water (50 ml) was added to the mixture. The mixture was extracted with dichloromethane (25 ml × 3). Combined organic layers were washed with brine, dried over $MgSO_4$ and evaporated in vacuo. The residue was purified by silica gel chromatography (Merck,Kieselgel 60) with hexane/ethyl acetate = 3/1 for elution to give the product (327 mg).

(2) Preparation of (R)-3-aminoheptanoic acid

A mixture of (R)-2-t-butoxycarbonylaminoheptanenitrile (165 mg, prepared in (1)) in 6N-hydrochloric acid (10 ml) was refluxed for 3 h. After cooling, the mixture was evaporated in vacuo. The residue was dissolved in methanol (2 ml) and propylene oxide (1 ml) was added to the mixture. After being stirred at room temperature over night, the mixture was evaporated in vacuo. The residue was crystallized from methanol and diethyl ether to give the product (52 mg).

FAB-MS(m/e, $(C_7H_{15}NO_2+H)^+$):146

## Claims

1. A peptide derivative of the formula:

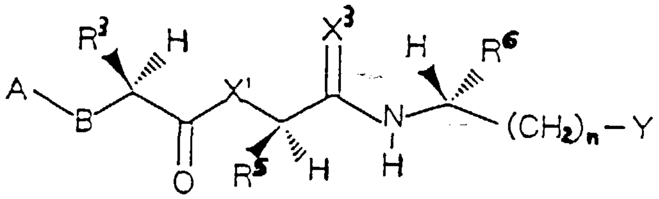

(I)

wherein A is a group of the formula $R^{12}R^{13}N-C(=O)-$ (wherein $R^{12}$ is a $C_{1-6}$ alkyl group, a cyclopropyl group, a cyclobutyl group, a cyclopentyl group, a cyclohexyl group, a cycloheptyl group, a cyclooctyl group, a cyclononyl group, a 1-adamantyl group, a phenyl group wherein one or two optional hydrogen atoms on the benzene ring may independently be replaced by a halogen atom, a trifluoromethyl group, a nitro group, an amino group or a formylamino group, or a thienyl group, $R^{13}$ is a hydrogen atom, a $C_{1-6}$ alkyl group or a cyclopropyl group, a cyclobutyl group, a cyclopentyl group, a cyclohexyl group, a cycloheptyl group, a cyclooctyl group, a cyclononyl group, or $R^{12}$ and $R^{13}$ form, together with the adjacent nitrogen atom, a 5- to 9- membered nitrogen-containing saturated heterocyclic ring having 4 to 8 carbon atoms, wherein among methylene groups forming the ring, one optional methylene group not adjacent to the above nitrogen atom may be replaced by a thio group, and one to four optional hydrogen atoms on the carbon atoms of the heterocyclic ring may independently be replaced by a $C_{1-6}$ alkyl group, and further two adjacent carbon atoms in the heterocyclic ring may form a benzo-fused ring); B is an oxygen atom or a group of the formula $-NR^2-$ (wherein $R^2$ is a hydrogen atom or a $C_{1-6}$ alkyl group); $R^3$ is a $C_{1-6}$ alkyl group, a cyclopropyl group, a cyclobutyl group, a cyclopentyl group, a cyclohexyl group, a cycloheptyl group, a cyclooctyl group, a cyclononyl group, a phenyl group, a 1-naphthyl group, a 2-naphthyl group, a thienyl group, a furyl group, a thiazolyl group, an imidazolyl group, a pyridyl group, an indolyl group, a benzothienyl group, a $C_{1-6}$ alkyl group having a substituent selected from the group consisting of a cyclopropyl group, a cyclobutyl group, a cyclopentyl group, a cyclohexyl group, a cycloheptyl group, a cyclooctyl group, a cyclononyl group, a phenyl group, a 1-naphthyl group, a 2-naphthyl group, a thienyl group, a furyl group, a thiazolyl group, an imidazolyl group, a pyridyl group, an indolyl group and a benzothienyl group; $X^1$ is an oxygen atom or a group of the formula $-NR^4-$

(wherein $R^4$ is a hydrogen atom or a $C_{1-6}$ alkyl group); $R^5$ is a 3-indolylmethyl group wherein the indole ring is substituted at the 1-position and/or 2-position by one or two substituents selected from the group consisting of a $C_{1-6}$ alkoxy group, a $C_{1-6}$ alkylthio group, a $C_{2-6}$ alkanoyloxy group, a $C_{1-6}$ alkoxy-carbonyl group and a halogen atom; $X^3$ is an oxygen atom or a sulfur atom; $R^6$ is a $C_{1-6}$ alkyl or $C_{2-6}$ alkenyl group which may have a substituent selected from the group consisting of a $C_{1-6}$ alkoxy group and a $C_{1-6}$ alkylthio group;
n is 0 or 1; Y is a hydroxymethyl group, a group of the formula $CO_2R^{71}$ (wherein $R^{71}$ is a hydrogen atom or a $C_{1-6}$ alkyl group), a group of the formula $CONHR^{72}$ (wherein $R^{72}$ is a hydrogen atom or a $C_{1-6}$ alkyl group which may have a substituent selected from the group consisting of a hydroxyl group, a carboxyl group and a sulfo group), a 1H-tetrazol-5-yl group, a sulfo group and a phosphono group; or a pharmaceutically acceptable salt thereof.

**2.** A compound of the formula:

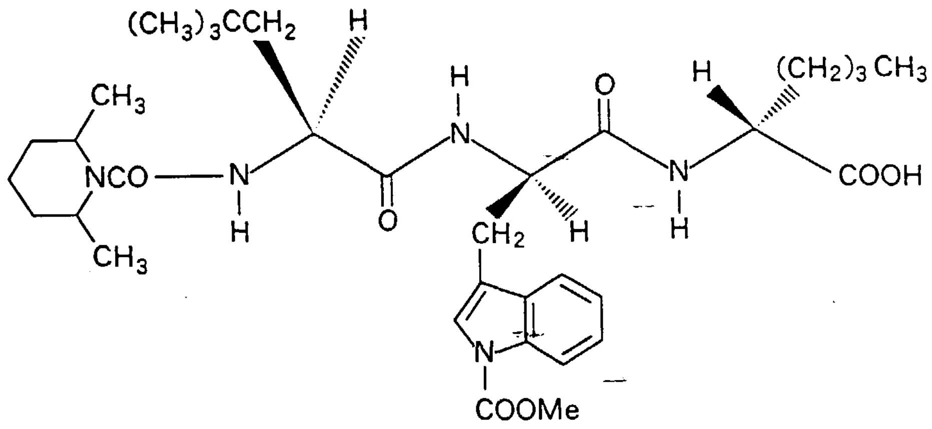

**3.** An endothelin antagonistic agent comprising a compound as defined in Claim 1 or a pharmaceutically acceptable salt thereof.

**4.** Use of a peptide derivative of the formula (I) as defined in Claim 1 or a pharmaceutically acceptable salt thereof for preparing a medicine having $ET_B$ receptor antagonistic activity.

**Patentansprüche**

**1.** Peptidderivat der Formel:

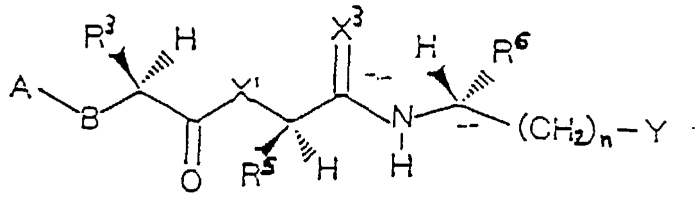

(I)

wobei A für eine Gruppe der Formel $R^{12}R^{13}N-C(=O)-$ steht (wobei $R^{12}$ für eine $C_{1-6}$Alkylgruppe, eine Cyclopropylgruppe, eine Cyclobutylgruppe, eine Cyclopentylgruppe, eine Cyclohexylgruppe, eine Cycloheptylgruppe, eine Cyclooctylgruppe, eine Cyclononylgruppe, eine 1-Adamantylgruppe, eine Phenylgruppe, wobei gegebenenfalls 1 oder 2 Wasserstoffatome des Benzolrings unabhängig voneinander durch ein Halogenatom, eine Trifluormethylgruppe, eine Nitrogruppe, eine Aminogruppe oder eine Formylaminogruppe ersetzt sein können, oder für eine Thienylgruppe steht, $R^{13}$ für ein Wasserstoffatom, eine $C_{1-6}$ Alkylgruppe oder eine Cyclopropylgruppe, eine Cyclobutylgruppe, eine Cyclopentylgruppe, eine Cyclohexylgruppe, eine Cycloheptylgruppe, eine Cyclooctylgruppe,

eine Cyclononylgruppe steht oder $R^{12}$ und $R^{13}$ zusammen mit dem benachbarten Stickstoffatom einen 5- bis 9-gliedrigen Stickstoff enthaltenden gesättigten heterocyclischen Ring bilden, der 4 bis 8 Kohlenstoffatome aufweist, wobei von den Methylengruppen, die den Ring bilden, gegebenenfalls eine Methylengruppe, die nicht in Nachbarstellung zu dem obigen Stickstoffatom steht, ersetzt sein kann durch eine Thiogruppe, und 1 bis 4 Wasserstoffatome an den Kohlenstoffatomen des heterocyclischen Rings können gegebenenfalls unabhängig durch eine $C_{1-6}$ Alkylgruppe ersetzt sein und weitere zwei benachbarte Kohlenstoffatome in dem heterocyclischen Ring können einen benzoannelierten Ring aufweisen); B für ein Sauerstoffatom oder eine Gruppe der Formel $-NR^2-$ steht (wobei $R^2$ für ein Wasserstoffatom oder eine $C_{1-6}$ Alkylgruppe steht); $R^3$ für eine $C_{1-6}$ Alkylgruppe, eine Cyclopropylgruppe, eine Cyclobutylgruppe, eine Cyclopentylgruppe, eine Cyclohexylgruppe, eine Cycloheptylgruppe, eine Cyclooctylgruppe, eine Cyclononylgruppe, eine Phenylgruppe, eine 1-Naphthylgruppe, eine 2-Naphthylgruppe, eine Thienylgruppe, eine Furylgruppe, eine Thiazolylgruppe, eine Imidazolylgruppe, eine Pyridylgruppe, eine Indolylgruppe, eine Benzothienylgruppe, eine $C_{1-6}$ Alkylgruppe steht, die einen Substituenten aufweist, ausgewählt aus der Gruppe bestehend aus einer Cyclopropyolgruppe, einer Cyclobutylgruppe, einer Cyclopentylgruppe, einer Cyclohexylgruppe, einer Cycloheptylgruppe, einer Cyclooctylgruppe, einer Cyclononylgruppe, einer Phenylgruppe, einer 1-Naphthylgruppe, einer 2-Naphthylgruppe, einer Thienylgruppe, einer Furylgruppe, einer Thiazolylgruppe, einer Imidazolylgruppe, einer Pyridylgruppe, einer Indolylgruppe und einer Benzothienylgruppe; $X^1$ für ein Sauerstoffatom oder eine Gruppe der Formel $-NR^4-$ steht (wobei $R^4$ ein Wasserstoffatom oder eine $C_{1-6}$ Alkylgruppe ist); $R^5$ für eine 3-Indolylmethylgruppe steht, wobei der Indolring an der 1-Position und/oder 2-Position durch ein oder zwei Substituenten substituiert ist, ausgewählt aus der Gruppe, bestehend aus einer $C_{1-6}$ Alkoxygruppe, einer $C_{1-6}$ Alkylthiogruppe, einer $C_{2-6}$ Alkanoyloxygruppe, einer $C_{1-6}$Alkoxycarbonylgruppe und einem Halogenatom; $X^3$ für ein Sauerstoffatom oder ein Schwefelatom steht; $R^6$ für eine $C_{1-6}$ Alkyl- oder $C_{2-6}$ Alkenylgruppe steht, die einen Substituenten, ausgewählt aus der Gruppe bestehend aus einer $C_{1-6}$ Alkoxygruppe und einer $C_{1-6}$ Alkylthiogruppe, aufweisen kann;

n für 0 oder 1 steht; Y für eine Hydroxymethylgruppe, eine Gruppe der Formel $CO_2R^{71}$ (wobei $R^{71}$ ein Wasserstoffatom oder eine $C_{1-6}$ Alkylgruppe ist), eine Gruppe der Formel $CONHR^{72}$ (wobei $R^{72}$ für ein Wasserstoffatom oder eine $C_{1-6}$ Alkylgruppe steht, die einen Substituenten aufweisen kann, ausgewählt aus der Gruppe, bestehend aus einer Hydroxylgruppe, einer Carboxylgruppe und einer Sulfogruppe), eine 1H-Tetrazol-5-yl-gruppe, eine Sulfogruppe und eine Phosphonogruppe steht; oder ein pharmazeutisch verträgliches Salz desselben.

**2.** Verbindung der Formel:

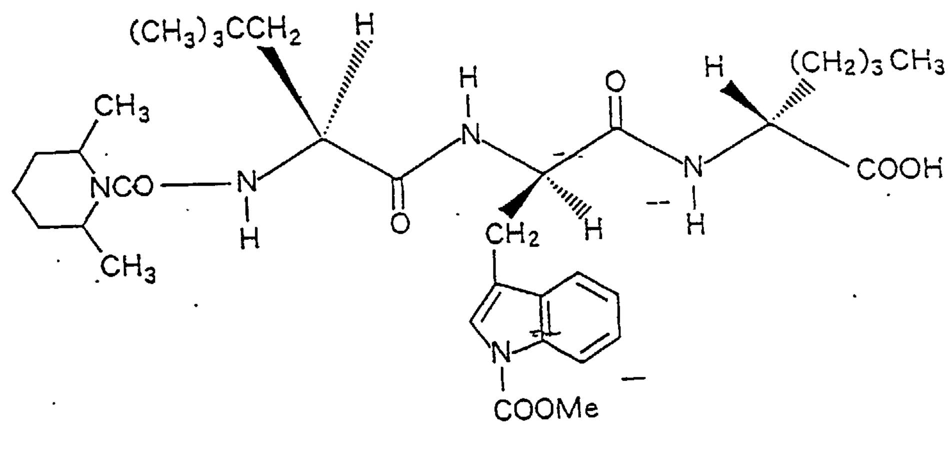

**3.** Endothelin antagonistisches Mittel, umfassend eine Verbindung, wie in Anspruch 1 definiert, oder ein pharmazeutisch verträgliches Salz derselben.

**4.** Verwendung eines Peptidderivats der Formel (I), wie es in Anspruch 1 definiert wird, oder eines pharmazeutisch verträglichen Salzes desselben zur Herstellung eines Arzneimittels, das $ET_B$ Rezeptorantagonismusaktivität aufweist.

**Revendications**

1. Dérivé peptidique de formule :

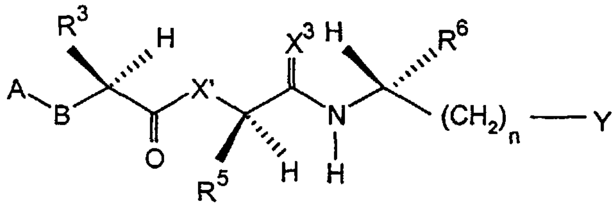

(I)

dans laquelle A est un groupe de formule $R^{12}R^{13}N-C(=O)-$(où $R^{12}$ est un groupe alkyle en $C_1$ à $C_6$, un groupe cyclopropyle, un groupe cyclobutyle, un groupe cyclopentyle, un groupe cyclohexyle, un groupe cycloheptyle, un groupe cyclooctyle, un groupe cyclononyle, un groupe 1-adamantyle, un groupe phényle dans lequel un ou deux atomes d'hydrogène au choix sur le cycle benzène peuvent indépendamment être remplacés par un atome d'halogène, un groupe trifluorométhyle, un groupe nitro, un groupe amino ou un groupe formylamino, ou un groupe thiényle, $R^{13}$ est un atome d'hydrogène, un groupe alkyle en $C_1$ à $C_6$ ou un groupe cyclopropyle, un groupe cyclobutyle, un groupe cyclopentyle, un groupe cyclohexyle, un groupe cycloheptyle, un groupe cyclooctyle, un groupe cyclononyle ou bien $R^{12}$ et $R^{13}$ forment, conjointement avec l'atome d'azote adjacent, un hétérocycle saturé contenant de l'azote à 5 à 9 chaînons et ayant de 4 à 8 atomes de carbone, dans lequel parmi les groupes méthylène formant le cycle, un groupe méthylène quelconque qui n'est pas adjacent à l'atome d'azote ci-dessus peut être remplacé par un groupe thio, et un à quatre atomes d'hydrogène au choix sur les atomes de carbone de l'hétérocycle peuvent indépendamment être remplacés par un groupe alkyle en $C_1$ à $C_6$, et en outre deux atomes de carbone adjacents dans l'hétérocycle peuvent former un cycle benzo-condensé) ; B est un atome d'oxygène ou un groupe de formule $-NR^2-$ (dans lequel $R^2$ est un atome d'hydrogène ou un groupe alkyle en $C_1$ à $C_6$); $R^3$ est un groupe alkyle en $C_1$ à $C_6$, un groupe cyclopropyle, un groupe cyclobutyle, un groupe cyclopentyle, un groupe cyclohexyle, un groupe cycloheptyle, un groupe cyclooctyle, un groupe cyclononyle, un groupe phényle, un groupe 1-naphtyle, un groupe 2-naphtyle, un groupe thiényle, un groupe furyle, un groupe thiazolyle, un groupe imidazolyle, un groupe pyridyle, un groupe indolyle, un groupe benzothiényle, un groupe alkyle en $C_1$ à $C_6$ ayant un substituant choisi dans le groupe se composant d'un groupe cyclopropyle, d'un groupe cyclobutyle, d'un groupe cyclopentyle, d'un groupe cyclohexyle, d'un groupe cycloheptyle, d'un groupe cyclooctyle, d'un groupe cyclononyle, d'un groupe phényle, d'un groupe 1-naphtyle, d'un groupe 2-naphtyle, d'un groupe thiényle, d'un groupe furyle, d'un groupe thiazolyle, d'un groupe imidazolyle, d'un groupe pyridyle, d'un groupe indolyle et d'un groupe benzothiényle ; $X^1$ est un atome d'oxygène ou un groupe de formule $-NR^4-$(dans laquelle $R^4$ est un atome d'hydrogène ou un groupe alkyle en $C_1$ à $C_6$) ; $R^5$ est un groupe 3-indolylméthyle dans lequel le cycle indole est substitué en position 1 et/ou en position 2 par un ou deux substituants choisis dans l'ensemble constitué par un groupe alcoxy en $C_1$ à $C_6$, un groupe alkylthio en $C_1$ à $C_6$, un groupe alcanoyloxy en $C_2$ à $C_6$, un groupe (alcoxy en $C_1$-$C_6$)carbonyle et un atome d'halogène ; $X^3$ est un atome d'oxygène ou un atome de soufre ; $R^6$ est un groupe alkyle en $C_1$ à $C_6$ ou alcényle en $C_2$ à $C_6$, pouvant avoir un substituant choisi dans le groupe se composant d'un groupe alcoxy en $C_1$ à $C_6$ et d'un groupe alkylthio en $C_1$ à $C_6$ ;
n vaut 0 ou 1 ; Y est un groupe hydroxyméthyle, un groupe de formule $CO_2R^{71}$ (dans laquelle $R^{71}$ est un atome d'hydrogène ou un groupe alkyle en $C_1$ à $C_6$), un groupe de formule $CONHR^{72}$ ($R^{72}$ est un atome d'hydrogène ou un groupe alkyle en $C_1$ à $C_6$ pouvant avoir un substituant choisi dans le groupe se composant d'un groupe hydroxyle, d'un groupe carboxyle et d'un groupe sulfo), un groupe 1H-tétrazol-5-yle, un groupe sulfo ou un groupe phosphono ; ou un de ses sels acceptables en pharmacie.

2. Composé de formule :

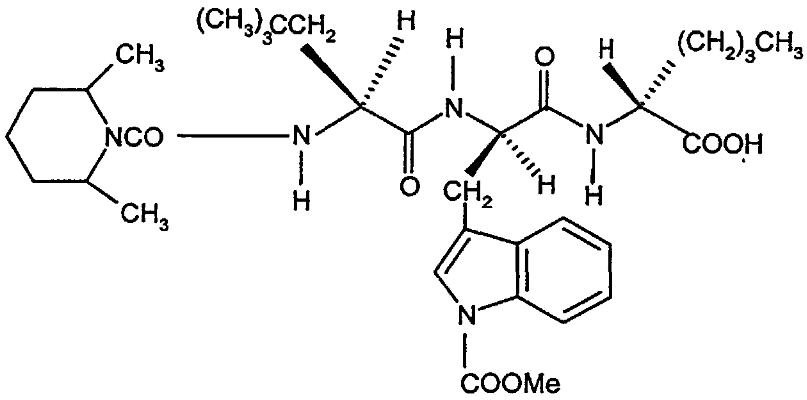

3. Agent antagoniste de l'endothéline comprenant un composé selon la revendication 1, ou un de ses sels acceptables en pharmacie.

4. Utilisation d'un dérivé peptidique de formule (I) selon la revendication 1 ou d'un de ses sels acceptables en pharmacie pour préparer un médicament ayant une activité antagoniste du récepteur $ET_B$.

FIGURE 1

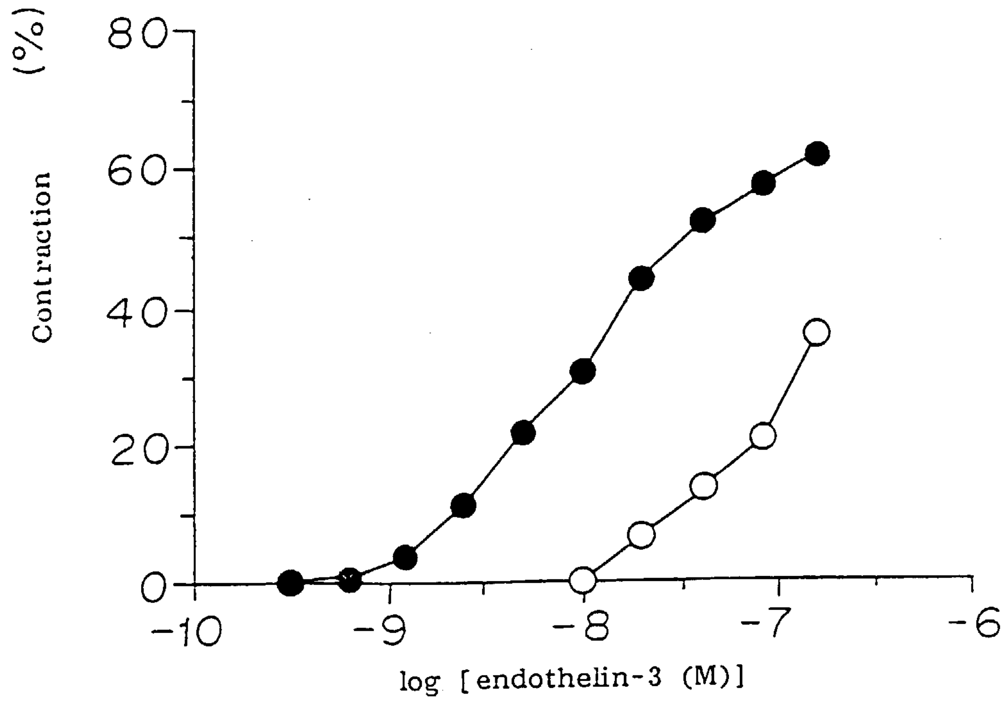


FIGURE 2

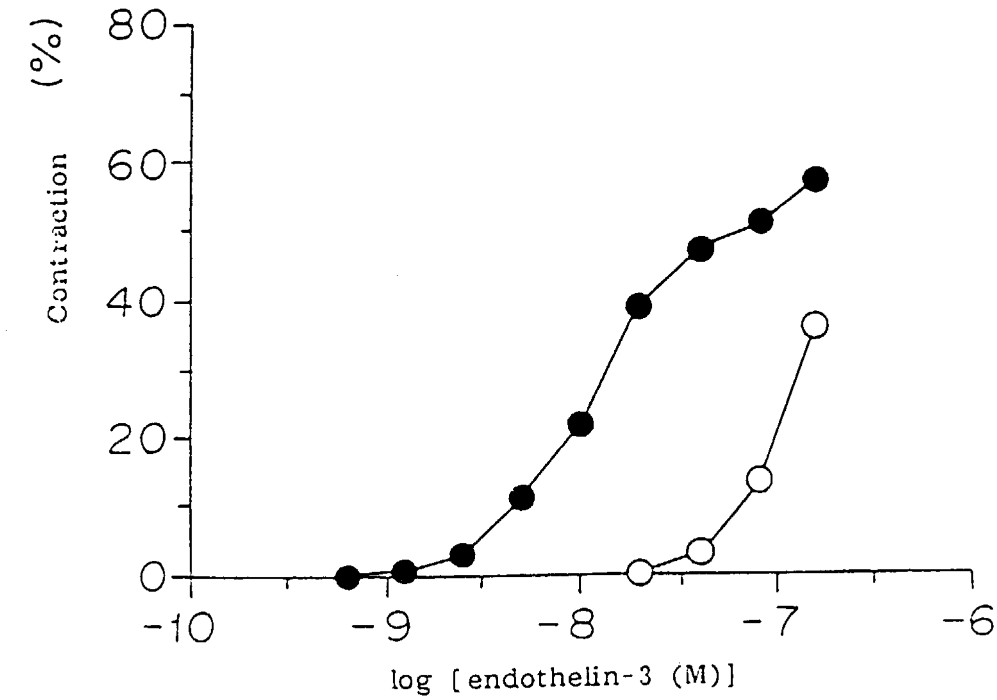

FIGURE 3